# EUROPEAN PATENT APPLICATION

(11) **EP 4 459 377 A2**
(43) Date of publication of application: **06.11.2024**
(21) Application number: 24168908.2
(22) Date of filing: 08.04.2024
(51) Int. Cl.: G03F 7/09, C07C 1/00, C07C 43/215, C07C 215/74, C07C 217/78, C07D 251/24, C07C 43/243

(54) **MATERIAL FOR FORMING ORGANIC FILM, SUBSTRATE FOR MANUFACTURING SEMICONDUCTOR DEVICE, METHOD FOR FORMING ORGANIC FILM, PATTERNING PROCESS, AND COMPOUND**

(30) Priority: 10.04.2023 JP 2023063361
(71) Applicant: SHIN-ETSU CHEMICAL CO., LTD., Tokyo 1000005 (JP)
(72) Inventor: Kori, Daisuke, Niigata 950-0000 (JP); Sawamura, Takashi, Niigata 950-0000 (JP)
(74) Representative: Sonnenhauser, Thomas Martin

(57) **Abstract**

The present invention is a material for forming an organic film, including: a compound for forming an organic film (A) represented by the following general formula (1A); and an organic solvent (B), 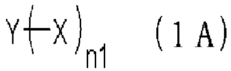 wherein Y represents an n1-valent organic group, a trivalent nitrogen atom, or a tetravalent carbon atom; n1 represents an integer of 3 to 8; and X is represented by the following general formula (1B), 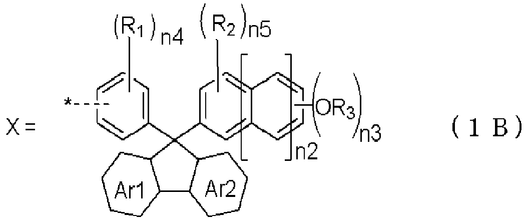 This provides: a compound that can form a cured organic film under inert gas conditions, having excellent heat resistance, filling and planarization characteristics of a pattern formed on a substrate, and having good adhesiveness to the substrate; and a material for forming an organic film containing the compound.

## Description

### TECHNICAL FIELD

The present invention relates to a material for forming an organic film, a substrate for manufacturing a semiconductor device using the material, a method for forming an organic film, a patterning process with a multilayer resist method, and a compound suitably usable for the material.

### BACKGROUND ART

Conventional increase in integration and increase in speed of semiconductor devices have been achieved by miniaturizing a pattern size with shortened wavelength of a light source in lithographic technology using light exposure (photolithography) as a common technology. To form such a fine circuit pattern in a substrate for manufacturing a semiconductor device (substrate to be processed), commonly used is a method of processing the substrate to be processed by dry etching while using a pattern-formed photoresist film as an etching mask. However, no dry-etching method can have practically perfect etching selectivity between the photoresist film and the substrate to be processed. Thus, substrate processing by a multilayer resist method have become common in recent years. This method includes: interposing an intermediate film having etching selectivity different from the photoresist film (hereinafter, also referred to as "the resist upper layer film") between the resist upper layer film and the substrate to be processed; obtaining a pattern in the resist upper layer film; then transferring the pattern to the intermediate film by dry etching while using the resist upper layer film pattern as a dry-etching mask; and further transferring the pattern to the substrate to be processed by dry etching while using the intermediate layer as a dry-etching mask.

One of the multilayer resist methods is a three-layer resist method, which can use a resist composition commonly used in a single-layer resist method. This method includes: applying and baking an organic underlayer film material (hereinafter, also referred to as the material for forming an organic film) composed of an organic-resin-containing composition on a substrate to be processed to form an organic underlayer film (hereinafter, also referred to as the organic film); applying and baking a resist intermediate film material composed of a silicon-containing resin-containing composition (hereinafter, also referred to as the silicon-containing resist intermediate film material) thereon to form a silicon-containing film (hereinafter, also referred to as the silicon-containing resist intermediate film); and forming a common organic photoresist film (resist upper layer film) thereon. After a pattern is formed in this resist upper layer film, dry etching with a fluorine-based gas plasma can achieve a good etching selectivity rate of the organic resist upper layer film against the silicon-containing resist intermediate film. Thus, in the resist upper layer film pattern can be transferred to the silicon-containing resist intermediate film. According to this method, the pattern can be easily transferred to the silicon-containing resist intermediate film even by using a resist upper layer film having no sufficient thickness for directly processing the substrate to be processed or by using a resist upper layer film having no sufficient dry-etching resistance for processing the substrate to be processed because the silicon-containing resist intermediate film typically has a thickness equal to or less than that of the resist upper layer film. Subsequently, the pattern-transferred silicon-containing resist intermediate film is used as a dry-etching mask to transfer the pattern to the organic film by dry etching with oxygen-based or hydrogen-based gas plasma, which can transfer the pattern to the organic film having sufficient dry-etching resistance for processing the substrate. This transferred pattern in the organic film can be transferred to the substrate by dry etching using a fluorine-based gas or a chlorine-based gas.

Meanwhile, the miniaturization in the semiconductor device manufacturing process has been reaching a fundamental limit derived from a wavelength of a light source for the photolithography. Thus, investigated in recent years are methods for high integration of the semiconductor devices without depending on the miniaturization. One of such methods is approached by a semiconductor device having a complex structure such as a multi-gate structure, and some devices have already been practically used. When such a structure is formed by the multilayer resist method, a material for forming an organic film that can achieve planarization by embedding fine patterns, such as holes, trenches, and fins, formed on the substrate to be processed in the film without a gap; or by embedding a step- or pattern-dense portion and a pattern-free region in the film can be applied. Forming the planarized surface of the organic film on the stepped substrate by using such a material for forming an organic film can reduce change in thickness of the silicon-containing resist intermediate film and the resist upper layer film, formed thereon, and can reduce decrease in focus latitude of the photolithography and decrease in margin in the step of processing the substrate to be processed thereafter. This enables to manufacture semiconductor devices with a good yield. Meanwhile, in the single layer resist method, the upper layer resist film becomes thick in order to fill the substrate to be processed having steps and patterns. This thickness decreases pattern-forming latitude during the exposure, such as pattern collapse after exposure and development and deterioration of a pattern shape due to reflection from the substrate during the exposure. Thus, it is difficult to manufacture the semiconductor devices with a good yield.

As a method for increasing speed of next-generation semiconductor devices, application of a novel material using strained silicon or gallium-arsine, for example, with high electron mobility, and a precision material such as ultrathin polysilicon regulated in angstrom scale are beginning to be investigated. However, in a substrate to be processed to which such a novel precision material is applied under conditions of planarized film formation of the above material for forming an organic film, for example a film-forming condition in air at 300°C or higher, the material may be corroded due to oxygen in air to fail to exhibit material-designed performance of increase in speed of the semiconductor device, leading to failure to achieve a viable yield for industrial production. Hence, to prevent the decrease in the yield caused by the substrate corrosion due to air under such a high temperature condition, a material for forming an organic film that can be formed in an inert gas is promising.

As the material for forming an organic film for the multilayer resist method, conventionally known is condensed resins produced by using carbonyl compounds, such as ketones and aldehydes, or aromatic alcohols as a condensing agent to react with phenol-based or naphthol-based compounds. Examples thereof include a fluorene-bisphenol novolac resin described in Patent Document 1, a bisphenol compound and a novolac resin thereof described in Patent Document 2, a novolac resin of an adamantane phenol compound described in Patent Document 3, and a bisnaphthol compound and a novolac resin thereof described in Patent Document 4. Such materials form a film having solvent resistance against a coating material used in the next process by a curing action. This action is exhibited by crosslinking using a methylol compound as a crosslinker or by oxidation at an α-position of the aromatic ring due to an action of oxygen in air and a subsequent crosslinking reaction resulting from condensation. However, oxidation which is a driving force to exhibit this solvent resistance cannot satisfy performance such as sufficient heat resistance and planarization characteristics in an inert gas.

### CITATION LIST

### PATENT LITERATURE

Patent Document 1: JP 2005-128509 A
Patent Document 2: JP 2006-293298 A
Patent Document 3: JP 2006-285095 A
Patent Document 4: JP 2010-122656 A

### SUMMARY OF INVENTION

### TECHNICAL PROBLEM

The present invention has been made in view of the above circumstances. An object of the present invention is to provide: a compound that is cured under film-forming conditions not only in air but also in an inert gas, that can form an organic film having excellent heat resistance, filling and planarization characteristics of a pattern formed on a substrate, and good adhesiveness to the substrate; and a material for forming an organic film containing the compound. In addition, an object of the present invention is to provide a substrate for manufacturing a semiconductor device, method for forming an organic film, and patterning process using the above material.

### SOLUTION TO PROBLEM

To solve the above problem, the present invention provides a material for forming an organic film comprising:
a compound for forming an organic film (A) represented by the following general formula (1A); and
an organic solvent (B), 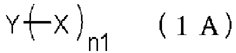
wherein Y represents an n1-valent organic group, a trivalent nitrogen atom, or a tetravalent carbon atom; n1 represents an integer of 3 to 8; and X represents a partial structure represented by the following general formula (1B), 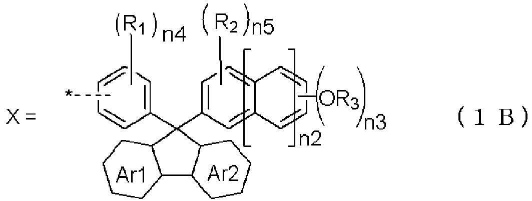
wherein a broken line represents an attachment point; R₁ and R₂ represent a halogen atom, an alkyl group having 1 to 4 carbon atoms, an alkyloxy group having 1 to 4 carbon atoms, an alkynyl group having 2 to 4 carbon atoms, or an alkenyl group having 2 to 4 carbon atoms, a hydrogen atom on a carbon atom to constitute the alkyl group, the alkyloxy group, the alkynyl group, and the alkenyl group may be substituted with a fluorine atom, and aromatic rings optionally substituted with R₁ may form a cross-linked structure with a single bond or via a divalent group obtained by removing one hydrogen atom from the R₁; n4 and n5 represent an integer of 0 to 2; Ar1 and Ar2 each represent a benzene ring or a naphthalene ring; n2 represents 0 or 1; n3 represents 1 or 2; and R₃ represents a hydrogen atom, an alkyl group having 1 to 4 carbon atoms, an alkynyl group having 2 to 4 carbon atoms, or an alkenyl group having 2 to 4 carbon atoms.

Such a material for forming an organic film can be cured under film-forming conditions not only in air but also in an inert gas, and can form an organic film having high heat resistance, good adhesiveness to a substrate, and high filling/planarization characteristics.

In this case, R₃ in the general formula (1B) preferably represents any one of groups represented by the following formula (1C), 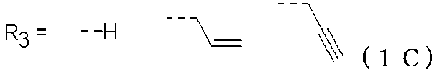 wherein a broken line represents an attachment point.

It is preferable to introduce the above terminal structure into the compound for forming an organic film being the component (A) from the viewpoint of imparting excellent curability, planarization characteristics, filling characteristics, and adhesiveness to a substrate.

In the general formula (1A), n1 preferably represents 3 or 4.

It is preferable to regulate the number of substituents in the compound for forming an organic film being the component (A) within the appropriate range from the viewpoint of prevention of impairing thermal flowability due to drastic curing.

In the general formula (1B), n2 preferably represents 1.

Introducing the above structure into the compound for forming an organic film being the component (A) can increase a carbon density to impart etching resistance. In addition, shrinkage of the film due to heat can be inhibited to form a film having good heat resistance and planarization characteristics.

Y in the general formula (1A) preferably represents any one of partial structures represented by the following formula (1D), 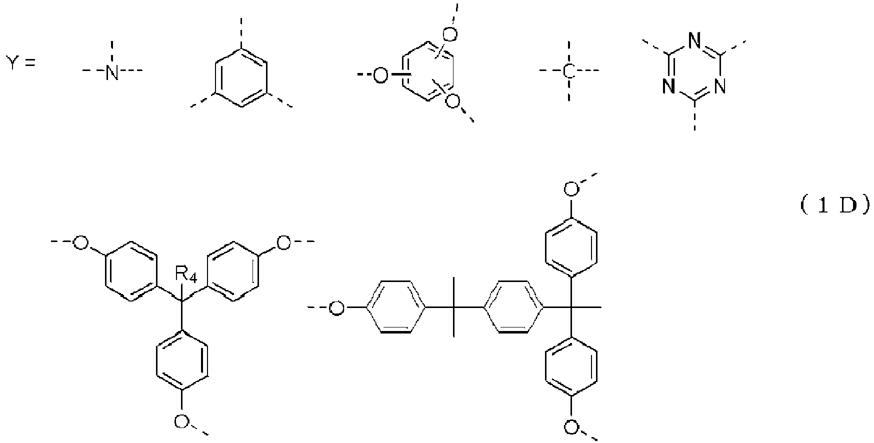 wherein a broken line represents an attachment point; and R₄ represents a hydrogen atom or a methyl group.

The compound for forming an organic film being the component (A) preferably has the above structure from the viewpoint of improvement of properties such as heat resistance, solvent solubility, and planarization characteristics.

A ratio Mw/Mn of a weight-average molecular weight Mw to a number-average molecular weight Mn of the component (A) in terms of polystyrene by a gel permeation chromatography method is preferably 1.00 ≤ Mw/Mn ≤ 1.10.

Regulating Mw/Mn of the compound for forming an organic film being the component (A) within the above range can form the organic film having excellent filling characteristics and planarization characteristics.

The organic solvent (B) is preferably a mixture of one or more kinds of organic solvents having a boiling point of lower than 180°C and one or more kinds of organic solvents having a boiling point of 180°C or higher.

The material for forming an organic film in which the organic solvent is the mixture as above can provide an organic film having further advanced filling/planarization characteristics with thermal flowability imparted by adding the high-boiling-point solvent. In the present invention, the boiling point is a value at 1 atm (1013 hPa).

The material for forming an organic film of the present invention may further comprise one or more of an acid generator (C), a surfactant (D), a crosslinker (E), and a plasticizer (F).

The material for forming an organic film of the present invention may comprise one or more of the above components (C) to (F) according to its purpose.

In addition, the present invention provides a substrate for manufacturing a semiconductor device comprising a cured organic film of the material for forming an organic film of the present invention on a substrate.

Such a substrate contains the organic film having high heat resistance, good adhesiveness to the substrate, and high filling/planarization characteristics, and thereby an yield of semiconductor devices becomes good when the substrate is used in the semiconductor device manufacturing process.

Further, the present invention provides a method for forming an organic film applied in a semiconductor device manufacturing process, the method comprising steps of: applying the material for forming an organic film of the present invention on a substrate to be processed by spin-coating; and heat-treating the substrate to be processed coated with the material for forming an organic film under an inert gas atmosphere at a temperature of 50°C or higher and 600°C or lower within a range of 5 seconds to 7200 seconds to obtain a cured film.

The organic film formed by the method of the present invention and applied in the semiconductor device manufacturing process is cured even with film formation in an inert gas, and the organic film has high heat resistance and high filling/planarization characteristics. Thus, an yield of semiconductor devices becomes good when the organic film is used in the semiconductor device manufacturing process.

In addition, the present invention provides a method for forming an organic film applied in a semiconductor device manufacturing process, the method comprising steps of: applying the material for forming an organic film of the present invention on a substrate to be processed by spin-coating; heat-treating the substrate to be processed coated with the material for forming an organic film in air at a temperature of 50°C or higher and 300°C or lower within a range of 5 seconds to 600 seconds to form an applied film; and subsequently heat-treating the applied film under an inert gas atmosphere at a temperature of 200°C or higher and 600°C or lower within a range of 10 seconds to 7200 seconds to obtain a cured film.

The organic film formed by the method of the present invention and applied in the semiconductor device manufacturing process is cured even with film formation partially in an inert gas, and the organic film has high heat resistance and high filling/planarization characteristics. Thus, an yield of semiconductor devices becomes good when the organic film is used in the semiconductor device manufacturing process.

In the method for forming an organic film of the present invention, an oxygen concentration in the inert gas is preferably 1 vol% or less.

The material for forming an organic film of the present invention is sufficiently cured without generation of sublimates even when heated in such an inert gas atmosphere, and can form the organic film having excellent adhesiveness to the substrate. In addition, the heating in such an inert gas atmosphere can prevent corrosion of the substrate to be processed. In the present invention, the oxygen concentration is described on a volume basis.

In the present invention, a substrate to be processed having a structure or step with 30 nm or more in height may be used as the substrate to be processed.

The method for forming an organic film of the present invention is particularly useful for forming a planarized organic film on such a substrate to be processed.

In addition, the present invention provides a patterning process comprising steps of: forming an organic film on a body to be processed by using the material for forming an organic film of the present invention; forming a silicon-containing resist intermediate film on the organic film by using a silicon-containing resist intermediate film material; forming a resist upper layer film on the silicon-containing resist intermediate film by using a photoresist composition; forming a circuit pattern in the resist upper layer film; transferring the pattern to the silicon-containing resist intermediate film by etching while using the patterned resist upper layer film as a mask; transferring the pattern to the organic film by etching while using the patterned silicon-containing resist intermediate film as a mask; and transferring the pattern to the body to be processed by etching while using the patterned organic film as a mask.

In addition, the present invention provides a patterning process comprising steps of: forming an organic film on a body to be processed by using the composition for forming an organic film of the present invention; forming a silicon-containing resist intermediate film on the organic film by using a silicon-containing resist intermediate film material; forming an organic anti-reflection film on the silicon-containing resist intermediate film; forming a resist upper layer film on the organic anti-reflection film by using a photoresist composition to form a four-layer film structure; forming a circuit pattern in the resist upper layer film; transferring the pattern to the organic anti-reflection film and the silicon-containing resist intermediate film by etching while using the patterned resist upper layer film as a mask; transferring the pattern to the organic film by etching while using the patterned silicon-containing resist intermediate film as a mask; and transferring the pattern to the body to be processed by etching while using the patterned organic film as a mask.

In addition, the present invention provides a patterning process comprising steps of: forming an organic film on a body to be processed by using the composition for forming an organic film of the present invention; forming an inorganic hard mask selected from a silicon oxide film, a silicon nitride film, a silicon oxynitride film, a titanium oxide film, and a titanium nitride film on the organic film; forming a resist upper layer film on the inorganic hard mask by using a photoresist composition; forming a circuit pattern in the resist upper layer film; transferring the pattern to the inorganic hard mask by etching while using the patterned resist upper layer film as a mask; transferring the pattern to the organic film by etching while using the patterned inorganic hard mask as a mask; and transferring the pattern to the body to be processed by etching while using the patterned organic film as a mask.

In addition, the present invention provides a patterning process comprising steps of: forming an organic film on a body to be processed by using the composition for forming an organic film of the present invention; forming an inorganic hard mask selected from a silicon oxide film, a silicon nitride film, a silicon oxynitride film, a titanium oxide film, and a titanium nitride film on the organic film; forming an organic anti-reflection film on the inorganic hard mask; forming a resist upper layer film on the organic anti-reflection film by using a photoresist composition to form a four-layer film structure; forming a circuit pattern in the resist upper layer film; transferring the pattern to the organic anti-reflection film and the inorganic hard mask by etching while using the patterned resist upper layer film as a mask; transferring the pattern to the organic film by etching while using the patterned inorganic hard mask as a mask; and transferring the pattern to the body to be processed by etching while using the patterned organic film as a mask.

The material for forming an organic film of the present invention can be suitably used for various patterning processes such as: the three-layer resist process using the silicon-containing resist intermediate film or the inorganic hard mask; and the four-layer resist process using the organic anti-reflection film in addition thereto. In the semiconductor device manufacturing process, semiconductor devices can be manufactured with a good yield by forming the circuit pattern with the patterning process of the present invention as above.

When the inorganic hard mask is used, this hard mask is preferably formed by a CVD method or an ALD method.

In the patterning process of the present invention, the inorganic mask may be formed by such methods, for example.

In the present invention, the circuit pattern is preferably formed by lithography using light having a wavelength of 10 nm or longer and 300 nm or shorter, direct writing with electron beam, nanoimprinting, or a combination thereof.

The circuit pattern is preferably developed with alkaline development or an organic solvent.

In the patterning process of the present invention, such a circuit pattern forming means and a developing means can be suitably used.

A semiconductor device substrate, or a substrate in which any of a metal film, a metal carbide film, a metal oxide film, a metal nitride film, a metal oxycarbide film, and a metal oxynitride film is formed on the semiconductor device substrate is preferably used as the body to be processed.

A material containing silicon, titanium, tungsten, hafnium, zirconium, chromium, germanium, copper, silver, gold, aluminum, indium, gallium, arsenic, palladium, iron, tantalum, iridium, cobalt, manganese, molybdenum, or an alloy thereof is preferably used as the body to be processed.

The patterning process of the present invention can process the body to be processed as above to form a pattern.

The present invention provides a compound represented by the following general formula (1A), 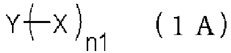 wherein Y represents an n1-valent organic group, a trivalent nitrogen atom, or a tetravalent carbon atom; n1 represents an integer of 3 to 8; and X represents a partial structure represented by the following general formula (1B), 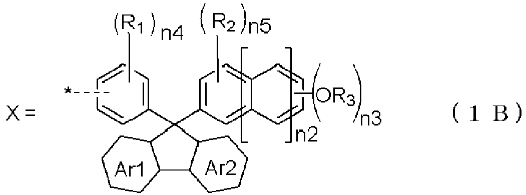 wherein a broken line represents an attachment point; R₁ and R₂ represent a halogen atom, an alkyl group having 1 to 4 carbon atoms, an alkyloxy group having 1 to 4 carbon atoms, an alkynyl group having 2 to 4 carbon atoms, or an alkenyl group having 2 to 4 carbon atoms, a hydrogen atom on a carbon atom to constitute the alkyl group, the alkyloxy group, the alkynyl group, and the alkenyl group may be substituted with a fluorine atom, and aromatic rings optionally substituted with R₁ may form a cross-linked structure with a single bond or via a divalent group obtained by removing one hydrogen atom from the R₁; n4 and n5 represent an integer of 0 to 2; Ar1 and Ar2 each represent a benzene ring or a naphthalene ring; n2 represents 0 or 1; n3 represents 1 or 2; and R₃ represents a hydrogen atom, an alkyl group having 1 to 4 carbon atoms, an alkynyl group having 2 to 4 carbon atoms, or an alkenyl group having 2 to 4 carbon atoms.

Such a compound having three or more cardo structures in one molecule can be cured even under film-forming conditions in not only air but also an inert gas due to the substituent introduced at the terminal structure, and can form the organic film having high heat resistance, good adhesiveness to the substrate, and high filling/planarization characteristics. Introducing the plurality of the cardo structures into the molecule can reduce crystallinity of the compound to improve solubility in a solvent without impairing the heat resistance, and can achieve the contrary characteristics of the thermal flowability and heat resistance.

In this case, R₃ in the general formula (1B) preferably represents any one of groups represented by the following formula (1C), 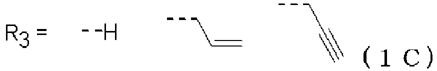 wherein a broken line represents an attachment point.

Introducing such a terminal structure can exhibit crosslinking characteristics, good adhesiveness to the substrate, high filling/planarization characteristics, etc.

In the compound of the present invention, n1 in the general formula (1A) preferably represents 3 or 4.

It is preferable to regulate the number of substituents in the compound within the appropriate range from the viewpoint of prevention of impairing thermal flowability due to drastic curing.

In the general formula (1B), n2 preferably represents 1.

Introducing the above structure into the compound can increase a carbon density to impart etching resistance. In addition, shrinkage of the film due to heat can be inhibited to form a film having good heat resistance and planarization characteristics.

Y in the general formula (1A) preferably represents any one of partial structures represented by the following formula (1D), 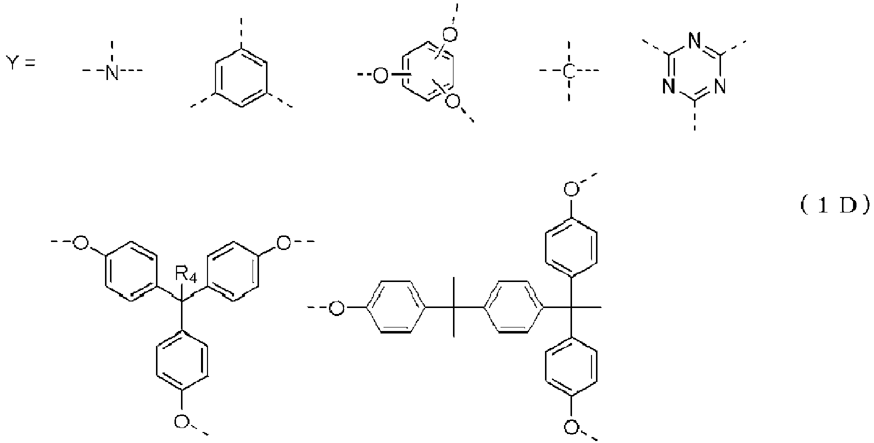 wherein a broken line represents an attachment point; and R₄ represents a hydrogen atom or a methyl group.

The compound of the present invention preferably has the above structure from the viewpoint of improvement of properties such as heat resistance, solvent solubility, and planarization characteristics.

### ADVANTAGEOUS EFFECTS OF INVENTION

As described above, the compound of the present invention is useful for forming the organic film cured without generation of byproducts even in film formation in an inert gas, preventing corrosion of the substrate, and having high heat resistance, good adhesiveness to the substrate, and advanced filling and planarization characteristics. The material for forming an organic film containing this compound (compound for forming an organic film) is a material for forming the organic film having excellent filling/planarization characteristics and having properties such as heat resistance and etching resistance. Therefore, the organic film obtained with the material for forming an organic film of the present invention has advanced filling/planarization characteristics to become an organic film having no micro-pores due to filling failure and no roughness on the organic film surface due to insufficient planarization. Thus, the material for forming an organic film of the present invention is extremely useful as, for example: the material for forming an organic film in the multilayer resist method such as the two-layer resist method, the three-layer resist method using the silicon-containing intermediate film, and four-layer resist method using the silicon-containing intermediate film and the organic anti-reflection film; or the planarization material for semiconductor device manufacturing. Since the organic film formed from the material for forming an organic film of the present invention has excellent heat resistance, there is no change in film thickness due to pyrolysis even when a CVD hard mask is formed on this organic film, and the organic film is suitable for patterning. Further, the semiconductor device substrate planarized with the organic film of the present invention has wide process latitude in patterning, and the semiconductor devices can be manufactured with a good yield. In the semiconductor device manufacturing process, the semiconductor devices can be manufactured with a good yield by forming the circuit pattern with the patterning process of the present invention.

### BRIEF DESCRIPTION OF DRAWINGS

FIG. 1 is an explanatory diagram of planarization characteristics in the present invention;
FIG. 2 is an explanatory diagram of an example of a patterning process of the present invention with a three-layer resist method;
FIG. 3 is an explanatory diagram of a method for evaluating filling characteristics in Examples;
FIG. 4 is an explanatory diagram of a method for evaluating planarization characteristics in Examples; and
FIG. 5 is an explanatory diagram of a method for measuring adhesiveness in Examples.

### DESCRIPTION OF EMBODIMENT

As noted above, there has been a demand for development of a compound for forming an organic film that generates no byproducts even under film-forming conditions in an inert gas in order to prevent corrosion of the substrate, such as, for example, 300°C or higher, and that can form an organic film having not only excellent filling and planarization characteristics for a pattern formed on the substrate but also good dry-etching resistance in substrate processing. Further, there has been a demand for development of a material for forming an organic film without change in film thickness of the organic film due to pyrolysis even when a CVD hard mask is formed on this organic film, and a compound for forming an organic film useful for a patterning process using this material.

For forming a typical organic film, a compound for forming an organic film is dissolved in an organic solvent to prepare a composition, this composition is applied on a substrate on which a structure, wiring, etc. of a semiconductor device are formed, and the applied film is baked to form the organic film. The applied film is formed along a shape of a step structure on the substrate immediately after applying the composition, but baking the applied film evaporates almost all of the organic solvent until curing to form the organic film with the compound for forming an organic film remained on the substrate. The present inventors have found that, when the compound for forming an organic film remained on the substrate in this time has sufficient thermal flowability, the thermal flow can planarize the step shape immediately after the applying to form a planarized film.

The present inventors have further made earnest study, and found that a compound for forming an organic film having a fluorene-type substituent represented by the following general formula (1A) provides a material for forming an organic film: that has thermal curability comparable to conventional materials for forming an organic film not only in air but also in an inert gas due to an action of the substituent introduced at the terminal; that has good thermal flowability to exhibit high filling/planarization characteristics; that uses a curing mechanism independent on oxidation, and thereby not deteriorates the etching resistance due to oxidation of the compound for forming an organic film and has good dry-etching resistance; and that has heat resistance with no change in film thickness of the applied film due to pyrolysis even when a CVD hard mask is formed because of a plurality of the rigid fluorene structures. This finding has led to completion of the present invention.

Specifically, the present invention is a material for forming an organic film comprising: a compound for forming an organic film (A) represented by the following general formula (1A); and an organic solvent (B), 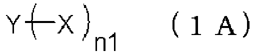 wherein Y represents an n1-valent organic group, a trivalent nitrogen atom, or a tetravalent carbon atom; n1 represents an integer of 3 to 8; and X represents a partial structure represented by the following general formula (1B), 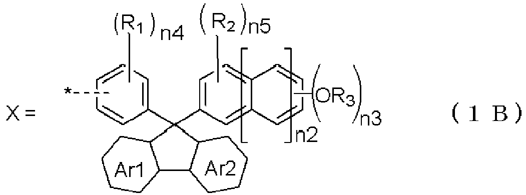 wherein a broken line represents an attachment point; R₁ and R₂ represent a halogen atom, an alkyl group having 1 to 4 carbon atoms, an alkyloxy group having 1 to 4 carbon atoms, an alkynyl group having 2 to 4 carbon atoms, or an alkenyl group having 2 to 4 carbon atoms, a hydrogen atom on a carbon atom to constitute the alkyl group, the alkyloxy group, the alkynyl group, and the alkenyl group may be substituted with a fluorine atom, and aromatic rings optionally substituted with R₁ may form a cross-linked structure with a single bond or via a divalent group obtained by removing one hydrogen atom from the R₁; n4 and n5 represent an integer of 0 to 2; Ar1 and Ar2 each represent a benzene ring or a naphthalene ring; n2 represents 0 or 1; n3 represents 1 or 2; and R₃ represents a hydrogen atom, an alkyl group having 1 to 4 carbon atoms, an alkynyl group having 2 to 4 carbon atoms, or an alkenyl group having 2 to 4 carbon atoms.

In addition, the present invention is a compound usable as a material for forming an organic film and represented by the general formula (1A).

Hereinafter, the present invention will be described in detail, but the present invention is not limited thereto.

The material for forming an organic film of the present invention comprises: a compound for forming an organic film represented by the general formula (1A) (component A); and an organic solvent (component B). This material for forming an organic film comprises the compound represented by the general formula (1A) (compound for forming an organic film) and the organic solvent, and may contain other components as necessary. Hereinafter, each component will be described.

### <Compound for Forming Organic Film>

The compound for forming an organic film of the present invention is a compound for forming an organic film represented by the following general formula (1A), 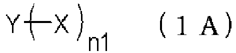 wherein Y represents an n1-valent organic group, a trivalent nitrogen atom, or a tetravalent carbon atom; n1 represents an integer of 3 to 8; and X represents a partial structure represented by the following general formula (1B), 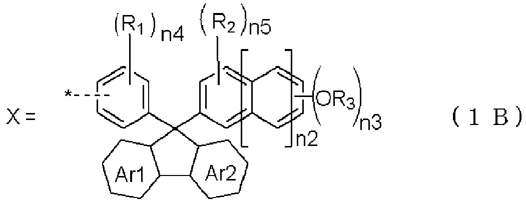 wherein a broken line represents an attachment point; R₁ and R₂ represent a halogen atom, an alkyl group having 1 to 4 carbon atoms, an alkyloxy group having 1 to 4 carbon atoms, an alkynyl group having 2 to 4 carbon atoms, or an alkenyl group having 2 to 4 carbon atoms, a hydrogen atom on a carbon atom to constitute the alkyl group, the alkyloxy group, the alkynyl group, and the alkenyl group may be substituted with a fluorine atom, and aromatic rings optionally substituted with R₁ may form a cross-linked structure with a single bond or via a divalent group obtained by removing one hydrogen atom from the R₁; n4 and n5 represent an integer of 0 to 2; Ar1 and Ar2 each represent a benzene ring or a naphthalene ring; n2 represents 0 or 1; n3 represents 1 or 2; and R₃ represents a hydrogen atom, an alkyl group having 1 to 4 carbon atoms, an alkynyl group having 2 to 4 carbon atoms, or an alkenyl group having 2 to 4 carbon atoms.

As in the general formula (1B), introducing the three or more cardo structures in the molecule can reduce crystallinity of the compound to improve solubility in the solvent without impairing the heat resistance, and can achieve both of trade-off performance of the thermal flowability and the heat resistance. OR₃ with a selected substituent can impart curability in air and under an inert gas to appropriately regulate the chain length from the viewpoints of planarization imparting, curability, etc. In addition, mixing with a compound partly having an ether group or a hydroxy group can impart high adhesiveness to the substrate. Further, appropriately selecting the structure to form the main skeleton, which is represented by Y, can regulate properties such as etching resistance and optical characteristics. These compounds can prevent film peeling in forming an inorganic hard mask directly on the organic film by using a CVD method or an ALD method, and the organic film having excellent process latitude can be formed.

Among Y in the general formula (1A), examples of an n1-valent organic group include the following groups. When these aromatic rings are contained, the aromatic ring may have a substituent, and examples of the substituent include an alkyl group having 1 to 10 carbon atoms, an alkynyl group and alkenyl group having 3 to 10 carbon atoms, an aryl group having 6 to 10 carbon atoms, a nitro group, a halogen atom, a nitrile group, an alkoxycarbonyl group having 1 to 10 carbon atoms, and an alkanoyloxy group having 1 to 10 carbon atoms.

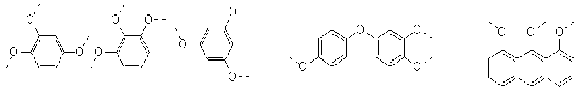

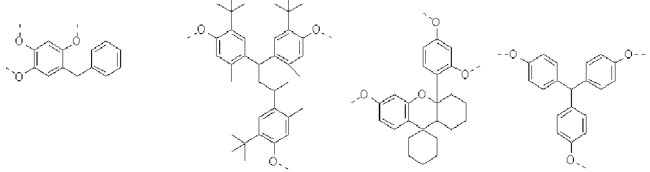

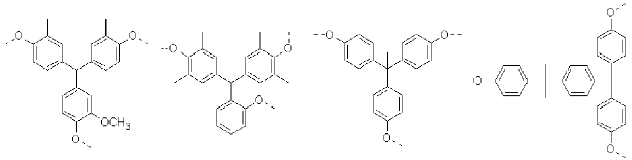

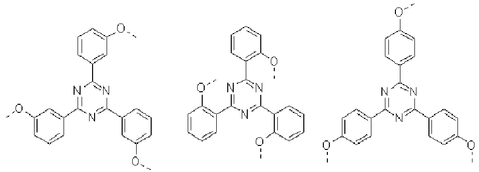

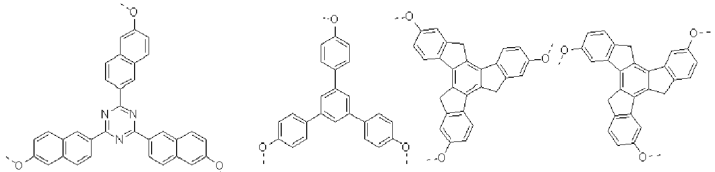

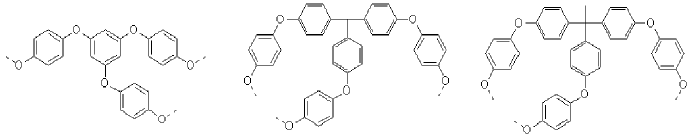

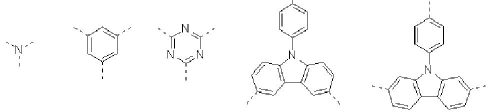

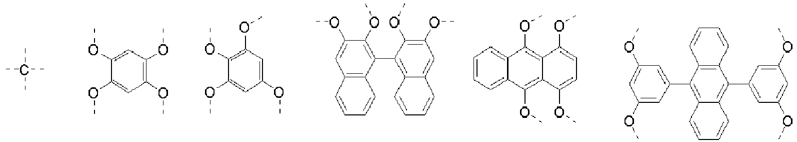

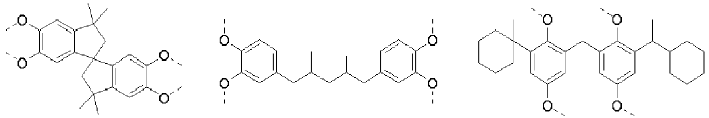

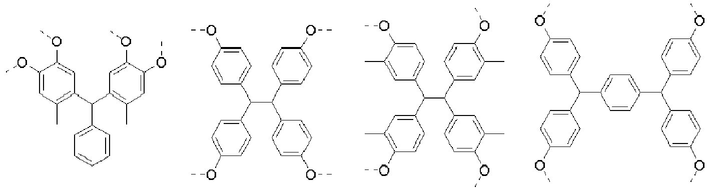

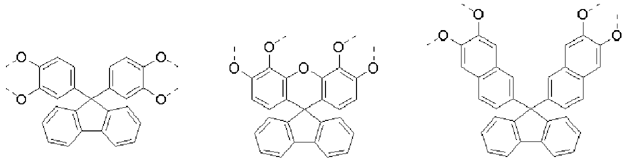

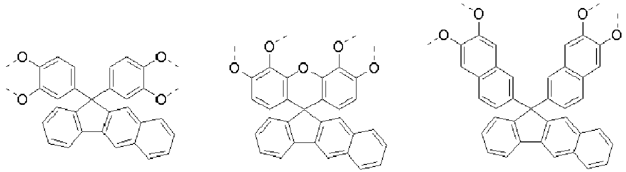

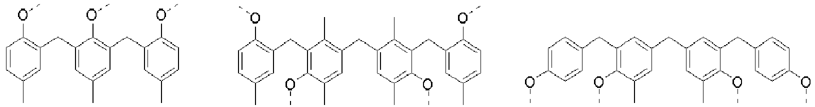

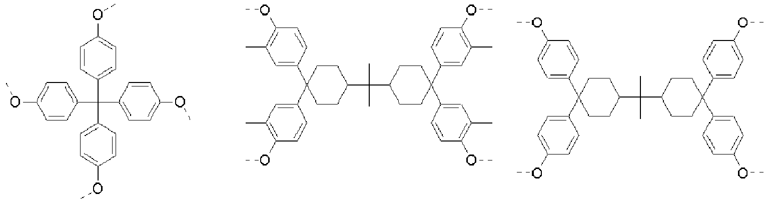

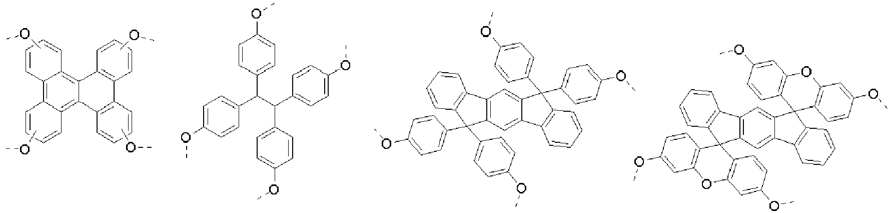

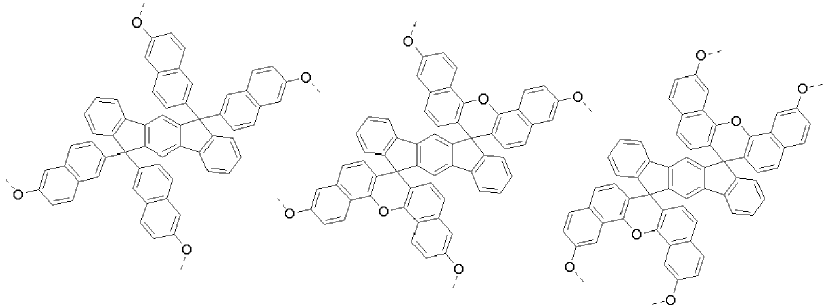

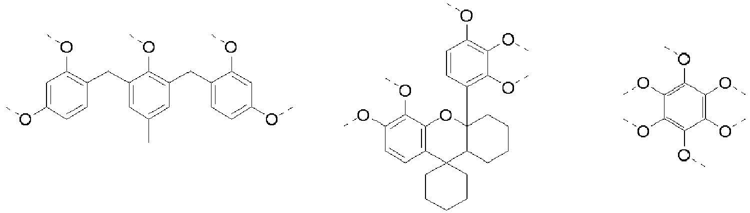

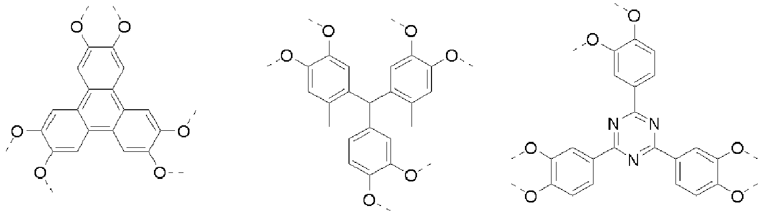

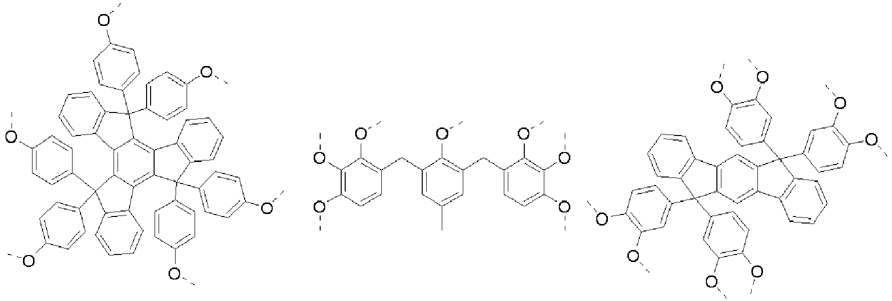

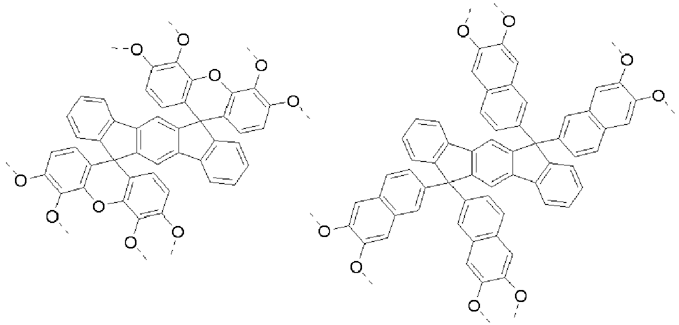

Among the compounds represented by the general formula (1A), examples of a case where the substituent R₁ on the aromatic ring in the general formula (1B) forms a cross-linked structure by replacing a hydrogen atom in the other aromatic ring (R₁ in the other aromatic rings are bonded to each other to form a cross-linked structure) include the following structure. Ar1, Ar2, n1, n2, n3, n5, R₁, R₂, and R₃ represent the same as above.

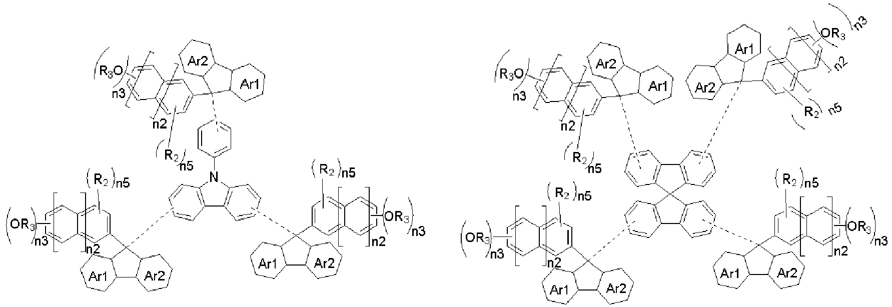

Among the above, n1=3 or 4 is preferably satisfied, that is, Y preferably represents a trivalent or tetravalent organic group from the viewpoint of regulating the number of substituents in the compound within an appropriate range to prevent impairing the thermal flowability due to drastic curing, and from the viewpoint of impairing the thermal flowability because increase in the substituent represented by X increases a molecular weight of the compound. In terms of easy availability of a raw material, n1=3 is more preferable.

Further, from the viewpoints of easy availability of a raw material, the heat resistance, and the thermal flowability, Y more preferably represents any one of partial structures represented by the following formula (1D) .

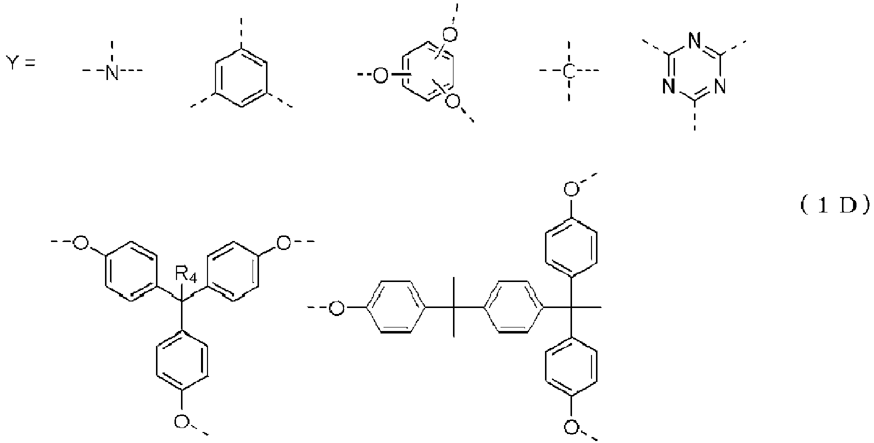

In the formulae, a broken line represents an attachment point; and R₄ represents a hydrogen atom or a methyl group.

X in the general formula (1A) is the following general formula (1B), 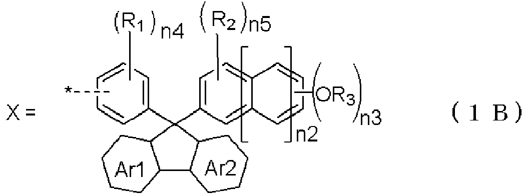 wherein a broken line represents an attachment point; R₁ and R₂ represent a halogen atom, an alkyl group having 1 to 4 carbon atoms, an alkyloxy group having 1 to 4 carbon atoms, an alkynyl group having 2 to 4 carbon atoms, or an alkenyl group having 2 to 4 carbon atoms, a hydrogen atom on a carbon atom to constitute the alkyl group, the alkyloxy group, the alkynyl group, and the alkenyl group may be substituted with a fluorine atom, and aromatic rings optionally substituted with R₁ may form a cross-linked structure with a single bond or via a divalent group obtained by removing one hydrogen atom from the R₁; n4 and n5 represent an integer of 0 to 2; Ar1 and Ar2 each represent a benzene ring or a naphthalene ring; n2 represents 0 or 1; n3 represents 1 or 2; and R₃ represents a hydrogen atom, an alkyl group having 1 to 4 carbon atoms, an alkynyl group having 2 to 4 carbon atoms, or an alkenyl group having 2 to 4 carbon atoms.

Examples of the partial structures represented by the general formula (1B) include the following structures. R₁, R₂, R₃, n3, n4, and n5 represent the same as above.

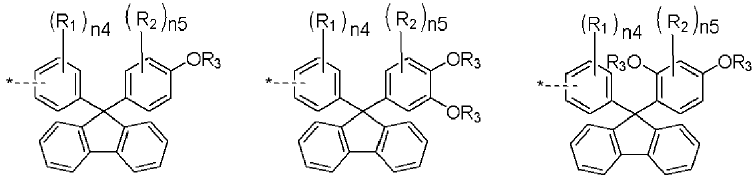

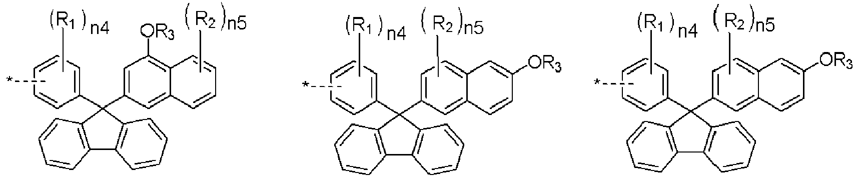

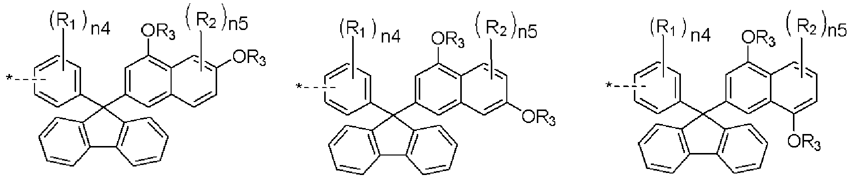

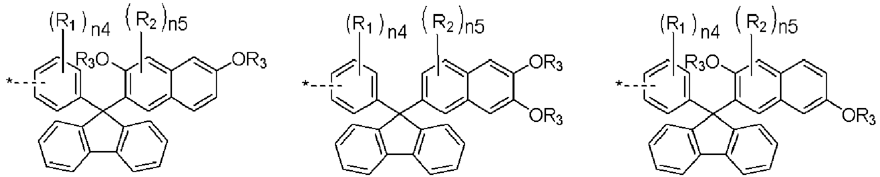

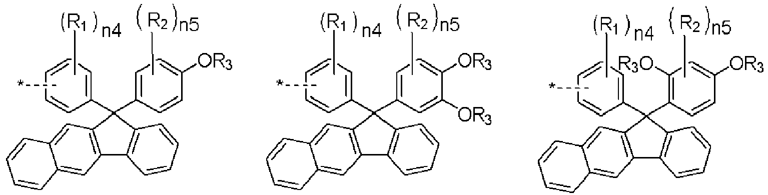

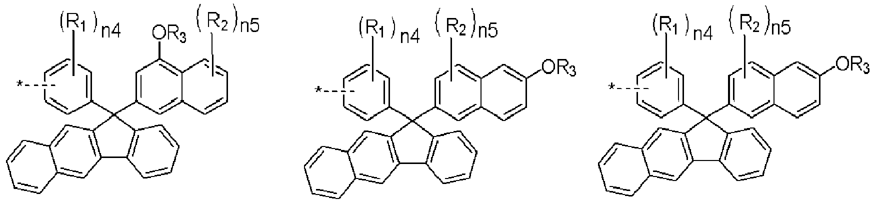

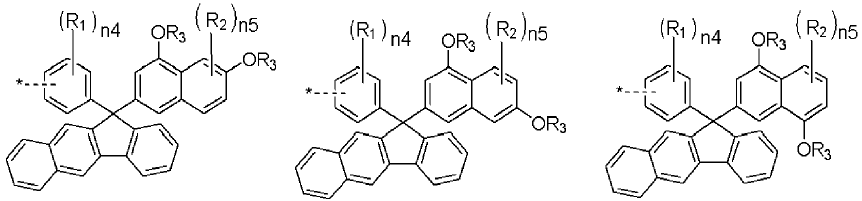

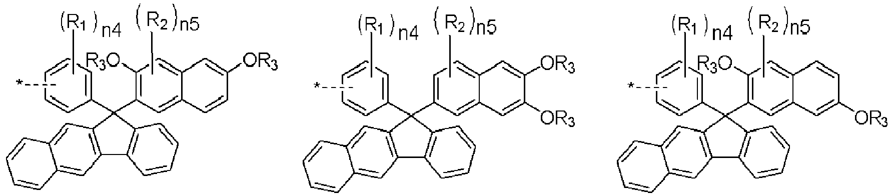

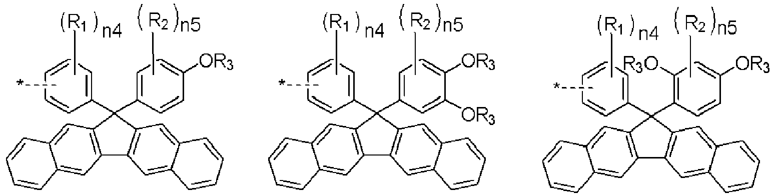

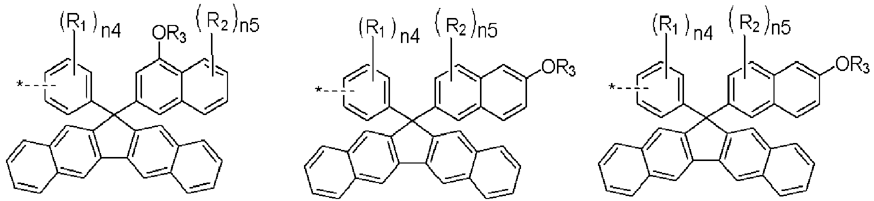

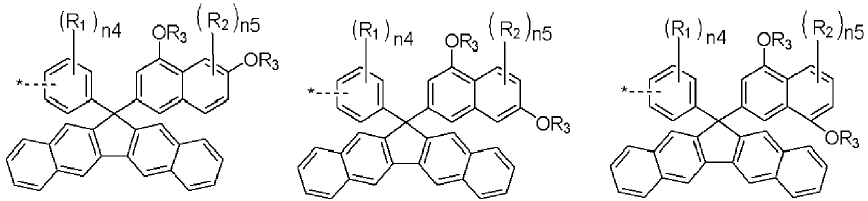

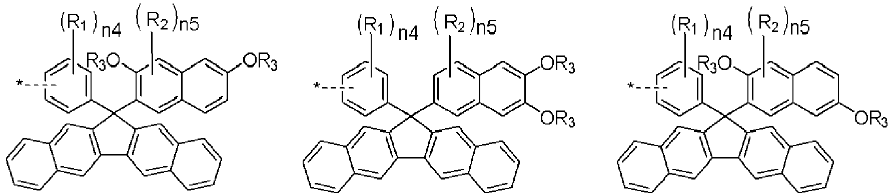

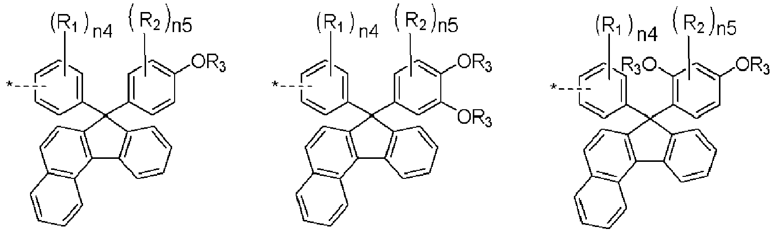

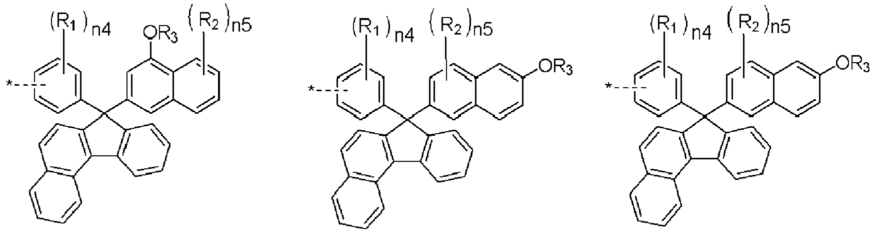

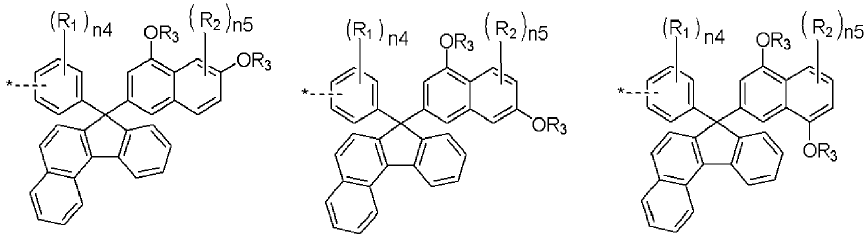

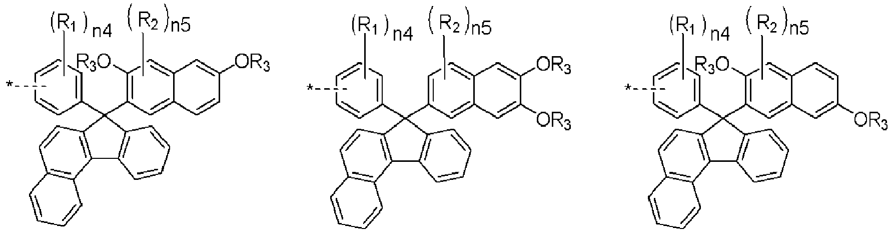

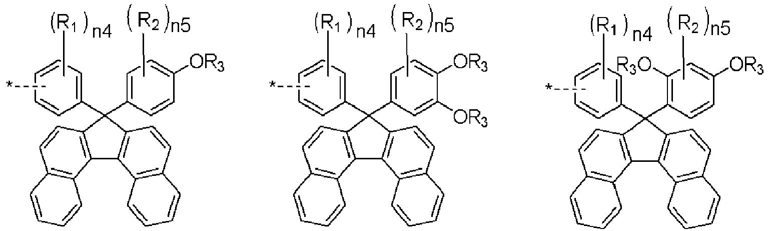

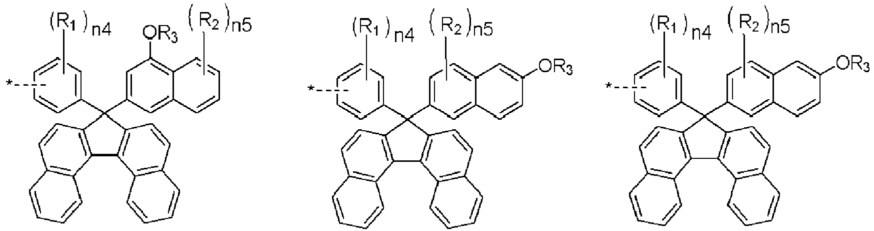

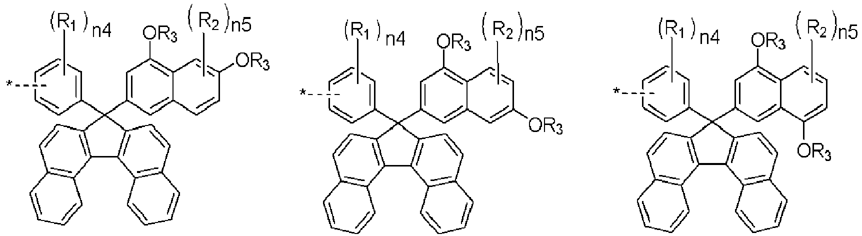

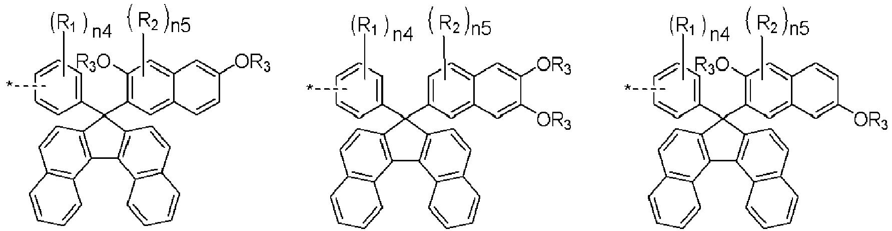

The partial structures represented by Ar1 and Ar2 in the general formula (1B) are preferably a combination of benzene rings as for Ar1 and Ar2 or a combination of a benzene ring and a naphthalene ring as for Ar1 and Ar2. When Ar1 and Ar2 each represent benzene rings, improvement of the heat resistance is expected from the viewpoint of symmetry, and when Ar1 and Ar2 are a combination of a benzene ring and a naphthalene ring, the asymmetrical structure further improves solubility of the compound in the solvent. Among these, Ar1 and Ar2 more preferably represent benzene rings in terms of easy availability of the raw material.

Examples of the substituents R₁ and R₂ on the aromatic groups in the general formula (1B) each include: halogen atoms, such as fluorine, chlorine, bromine, and iodine; alkyl groups having 1 to 4 carbon atoms, such as a methyl group, an ethyl group, a propyl group, an isopropyl group, a butyl group, a s-butyl group, and a t-butyl group; alkoxy groups having 1 to 4 carbon atoms, such as a methoxy group, an ethoxy group, a propoxy group, an n-butoxy group, a s-butoxy group, and a t-butoxy group; alkenyl groups having 2 to 4 carbon atoms, such as a vinyl group, an allyl group, and a butenyl group; and alkynyl groups having 2 to 4 carbon atoms, such as an ethynyl group, a propargyl group, and a butynyl group. A part or all of hydrogen atoms on the carbon atoms in the alkyloxy group, the alkyl group, the alkenyl group, and the alkynyl group may be substituted with a fluorine substituent such as a fluorine atom. For example, a methyl group may be a fluorinated group such as a trifluoromethyl group, a difluoromethyl group, or a monofluoromethyl group. Among these, n4, n5 = 0 is preferable from the viewpoints of heat resistance and etching resistance, and R₁ preferably represents a trifluoromethyl group from the viewpoints of planarization characteristics and solubility.

R₃ in the general formula (1B) represents a hydrogen atom, an alkyl group having 1 to 4 carbon atoms, or an alkynyl group or alkenyl group having 2 to 4 carbon atoms. Examples of the alkyl group having 1 to 4 carbon atoms include a methyl group, an ethyl group, a propyl group, an isopropyl group, a butyl group, a s-butyl group, and a t-butyl group. Examples of the alkenyl group having 2 to 4 carbon atoms include a vinyl group, an allyl group, and a butenyl group. Examples of the alkynyl group having 2 to 4 carbon atoms include an ethynyl group, a propargyl group, and a butynyl group. Among these, a structure represented by the following (1C) is preferable, and a propargyl group is more preferable from the viewpoint of thermal curability.

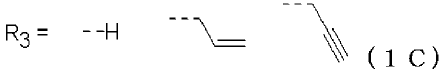

n2 in the general formula (1B) represents an integer of 0 or 1. From the viewpoints of heat resistance and etching resistance, n2=1, namely a naphthalene ring, is preferable.

n3 in the general formula (1B) represents an integer of 1 or 2. From the viewpoint of curability, n3=2 is preferable, and a catechol-like structure as the following partial structure in which substituents represented by -OR₃ are adjacent to each other is particularly preferable from the viewpoints of adhesiveness and heat resistance.

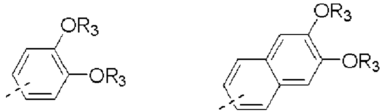

In the general formula (1B), a case as follows where n2=1 and n3 represents an integer of 1 or 2 is preferable. From the viewpoint of curability, n3=2 is more preferable, and a catechol-like structure as the following partial structure in which substituents represented by -OR₃ are adjacent to each other is particularly preferable from the viewpoints of adhesiveness and heat resistance.

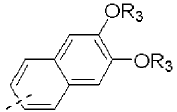

When the partial structure having the substituents represented by OR₃ has a propargyloxy group at a β-position on a naphthalene ring represented by the following formula, it is considered that the propargyloxy group forms a cyclized product with baking, and then polymerized to exhibit thermal curability. In this case, the propargyloxy group imparts flowability before the cyclization, and after the cyclization, the propargyloxy group not only acts as a curable group but also contributes to improvement of heat resistance with the ring structure formation. The curing reaction in this case can reduce a sublimate component and inhibit film shrinkage during baking because a decomposable additive, such as a thermal acid generator, and a byproduct of a condensation reaction, etc., which may result in an outgas component are not generated. These actions are further preferable because both of heat resistance and filling/planarization characteristics, which are contradictive performance, can be achieved.

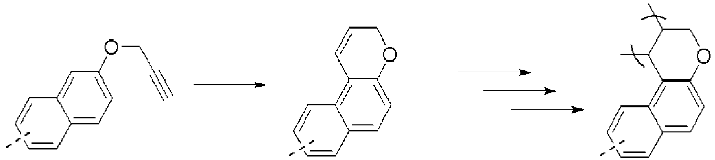

In the structure to constitute R₃, when a proportion of the hydrogen atoms is represented by "a", and a proportion of the alkyl groups having 1 to 4 carbon atoms and the alkynyl groups and alkynyl groups having 2 to 4 carbon atoms is represented by "b", occupancy of the hydrogen atoms, which have an expected effect of imparting adhesiveness to the substrate and film-formability, and occupancy of the alkyl groups having 1 to 4 carbon atoms and the alkynyl groups and alkenyl groups having 2 to 4 carbon atoms, which are expected to impart heat resistance and thermal flowability, can be regulated at any ratio to regulate the proportions according to desired performance. For regulating the ratio at any ratio, the substituent-conversion rate may be regulated with the reaction. Alternatively, a compound in which R₃ represents a hydrogen atom and a compound substituted with the alkyl groups having 1 to 4 carbon atoms, or the alkynyl groups or alkenyl groups having 2 to 4 carbon atoms may be separately prepared and mixed to regulate the ratio to be the given ratio.

In addition, the compound represented by the general formula (1A) preferably satisfies 1.00 ≤ Mw/Mn ≤ 1.10, where Mw/Mn is a ratio of a weight-average molecular weight Mw to a number-average molecular weight Mn, which are in terms of polystyrene by gel permeation chromatography (GPC). Although a monomolecular compound is supposed to have Mw/Mn of 1.00 by the definition, a measurement value may exceed 1.00 due to separability with the gel permeation chromatography. It is extremely difficult for a typical polymer having a repeating unit to have Mw/Mn closer to 1.00 unless using a special polymerization method, and a typical polymer has distribution of Mw to have a value of Mw/Mn exceeding 1. In the present invention, 1.00 ≤ Mw/Mn ≤ 1.10 may have been defined as an index indicating monomerism in order to distinguish between a monomolecular compound and a polymer. Note that the average molecular weight and the dispersion degree come from a weight-average molecular weight (Mw), a number-average molecular weight (Mn), and a dispersion degree (Mw/Mn) in terms of polystyrene measured by the GPC method with tetrahydrofuran as an eluent.

Regulating Mw/Mn of the compound for a composition for forming an organic film within the above range can form an organic film having excellent filling characteristics and planarization characteristics.

### Method for Manufacturing Compound for Forming Organic Film

An example of a method for manufacturing the compound of the present invention represented by the general formula (1A) is a method including: a step of performing an addition reaction of ketones having Ar1 and Ar2 with an organometal reagent having a metal M to obtain an intermediate fluorenols (STEP 1); and a subsequent step of performing a dehydrative condensation reaction of benzenes or naphthalenes having the OR₃ substituent as a raw material using an acid catalyst (STEP 2) to obtain the target product. For the reactions used for STEP 1 and STEP 2, a single raw material or two or more of raw materials may be used, and these may be appropriately combined according to the required performance. 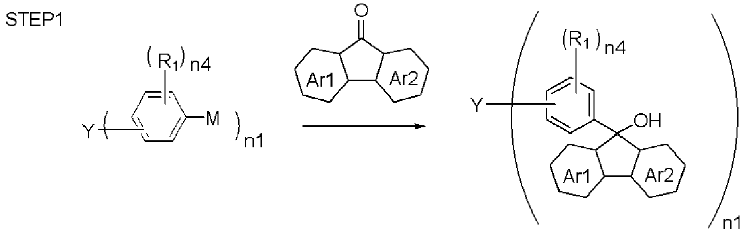 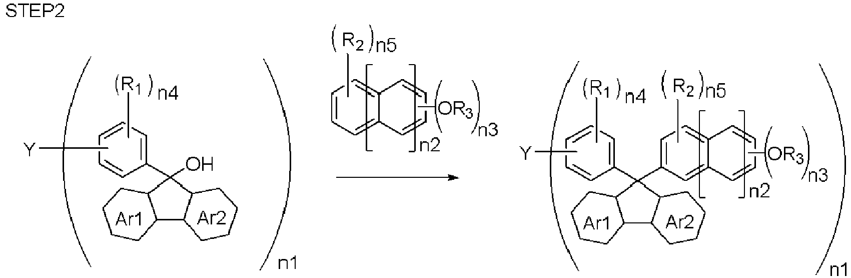 In the formulae, Y, Ar1, Ar2, n1, n2, n3, n4, n5, R₁, R₂, and R₃ represent the same as above; M represents a metal such as Li and MgX₁; and X₁ represents a halogen atom.

Examples of the organometal reagent used in STEP 1 include a Grignard reagent, an organolithium reagent, an organozinc reagent, and an organotitanium reagent, and a Grignard reagent and an organolithium reagent are particularly preferable. The Grignard reagent and the organolithium reagent may be prepared by direct metalation between a corresponding halide and metal magnesium or metal lithium, or may be prepared by a metal-halogen exchange reaction with an isopropylmagnesium halide or an aliphatic organometal compound such as methyllithium, and butyllithium.

The organozinc reagent and the organotitanium reagent may be prepared by a reaction from a corresponding Grignard reagent or organolithium reagent with a zinc halide, a titanium (IV) halide, an alkoxytitanium (IV), etc. In preparing the organometal reagent or in the reaction between the organometal reagent and a sumanene oxide, a metal salt compound may be present. In this case, a transition metal catalyst such as palladium and nickel promotes the reaction.

Examples of the metal salt compound include a cyanide, a halide, and a perhalogen acid salt, and examples of preferable metal salt compounds include: lithium salts, such as lithium chloride, lithium bromide, lithium iodide, and lithium perchlorate; and copper salts, such as copper (I) cyanide, cupper (II) cyanide, copper (I) chloride, copper (II) chloride, and dilithium tetrachlorocuprate.

The metal salt compound added at 0.01 to 5.0 equivalents, and preferably 0.2 to 2.0 equivalents relative to the organometal reagent can increase solubility of the organometal reagent to facilitate the preparation, and can regulate nucleophilicity and Lewis acidity of the reagent.

As a solvent used for preparing the organometal reagent and for the reaction with the sumanene oxide, ethers such as diethyl ether, dibutyl ether, tetrahydrofuran, 1,4-dioxane, and cyclopentyl methyl ether; hydrocarbons such as benzene, toluene, xylene, mesitylene, hexane, heptane, octane, and isooctane; and aprotic polar solvents such as N,N,N',N'-tetramethylethylenediamine, hexamethylphosphoric triamide, and N,N-dimethylformamide may be used singly or mixed for use.

Although depending on types of the ketones having Ar1 and Ar2 and the organometal reagents and the reaction conditions, the reaction temperature is preferably -70 to 150°C, and may be variously selected depending on the reaction. For example, when the organometal reagent is the organozinc reagent or the Grignard reagent, the reaction temperature may be from room temperature to reflux at a boiling point of the solvent. The reaction time is preferably typically 30 minutes to 48 hours.

Examples of the reaction method include: a method of collectively feeding the organometal reagent prepared in advance and the ketone having Ar1 and Ar2 into a solvent; and a method in which any one of the organometal reagent and the ketone having Ar1 and Ar2 is dispersed or dissolved in a solvent, and then the other is dispersed or dissolved in a solvent and the resultant is fed dropwise thereinto. The obtained intermediate fluorenol may be subsequently subjected to the dehydrative condensation reaction of STEP 2 as it is after the reaction is quenched with water, etc., but may be diluted with an organic solvent and then washed with liquid-separation or crystallized with a poor solvent to be recovered as a powder in order to remove an unreacted raw material present in the system as the reaction intermediate, the catalyst, etc.

As the acid catalyst used in the dehydrative condensation reaction represented in STEP 2, inorganic acids, such as hydrochloric acid, hydrobromic acid, sulfuric acid, nitric acid, phosphoric acid, and a heteropoly acid; organic acids, such as succinic acid, trifluoroacetic acid, methanesulfonic acid, benzenesulfonic acid, p-toluenesulfonic acid, and trifluoromethanesulfonic acid; and Lewis acids, such as aluminum trichloride, aluminum ethoxide, aluminum isopropoxide, boron trifluoride, boron trichloride, boron tribromide, tin tetrachloride, tin tetrabromide, dibutyltin dichloride, dibutyltin dimethoxide, dibutyltin oxide, titanium tetrachloride, titanium tetrabromide, titanium (IV) methoxide, titanium (IV) ethoxide, titanium (IV) isopropoxide, and titanium (IV) oxide may be used. The use amount of these catalysts may be within a range of 0.1 mol to 20 mol, and preferably 0.2 mol to 10 mol relative to a number of moles of the intermediate fluorenol.

The used solvent is not particularly limited, and examples thereof include: alcohols, such as methanol, ethanol, isopropyl alcohol, butanol, ethylene glycol, propylene glycol, diethylene glycol, glycerol, ethylene glycol monomethyl ether, and propylene glycol monomethyl ether; ethers, such as diethyl ether, dibutyl ether, diethylene glycol diethyl ether, diethylene glycol dimethyl ether, tetrahydrofuran, and 1,4-dioxane; chlorine-based solvents, such as methylene chloride, chloroform, dichloroethane, and trichloroethylene; hydrocarbons, such as hexane, heptane, benzene, toluene, xylene, and cumene; nitriles, such as acetonitrile; ketones, such as acetone, ethyl methyl ketone, and isobutyl methyl ketone; esters, such as ethyl acetate, n-butyl acetate, and propylene glycol methyl ether acetate; and aprotic polar solvents, such as dimethyl sulfoxide, N,N-dimethylformamide, and hexamethylphosphoric triamide. These solvents may be used singly, or two or more thereof may be mixed for use. These solvents may be used within a range of 0 to 2000 parts by mass relative to 100 parts by mass of the reaction raw material. The reaction temperature is preferably from -50°C to appropriately a boiling point of the solvent, and further preferably from room temperature to 150°C. The reaction time is appropriately selected from 0.1 to 100 hours.

Examples of the reaction method include: a method of collectively feeding the fluorenol, the benzene or naphthalene, and the acid catalyst being the catalyst; a method in which the fluorenol and the benzene or naphthalene are dispersed or dissolved, and then the catalyst is added collectively or in portions, or the catalyst is diluted with a solvent to be added dropwise; and a method in which the catalyst is dispersed or dissolved, and then the fluorenol and the benzene or naphthalene are each added collectively or in portions, or the fluorenol and the benzene or naphthalene are diluted with a solvent to be added dropwise. In this case, although depending on the reactivity of the benzene or naphthalene, 2 mol or more of the benzene or naphthalene is preferably used relative to 1 mol of the fluorenol. After the reaction is finished, the product is diluted with an organic solvent, and then washing with liquid-separation can be performed in order to remove the catalyst used in the reaction to recover the target product.

The organic solvent used in this case is not particularly limited as long as the organic solvent can dissolve the target product and separate the mixture into two layers even with mixing with water. Examples thereof include: hydrocarbons, such as hexane, heptane, benzene, toluene, and xylene; esters, such as ethyl acetate, n-butyl acetate, and propylene glycol methyl ether acetate; ketones, such as methyl ethyl ketone, methyl amyl ketone, cyclohexanone, and methyl isobutyl ketone; ethers, such as diethyl ether, diisopropyl ether, methyl t-butyl ether, and ethylcyclopentyl methyl ether; chlorine-based solvents, such as methylene chloride, chloroform, dichloroethane, and trichloroethylene; and a mixture thereof. As the washing water used in this time, water called as deionized water or ultrapure water is typically used. A number of times of the washing may be one or more, and preferably appropriately one to five because an expected effect is not always obtained even when the washing is performed 10 times or more.

To remove an acidic component in the system during the phase-separation washing, the washing may be performed with a basic aqueous solution. Specific examples of the base include a hydroxide of an alkali metal, a carbonate salt of an alkali metal, a hydroxide of an alkali earth metal, a carbonate salt of an alkali earth metal, ammonia, and an organic ammonium.

To remove a metal impurity or a base component in the system during the phase-separation washing, the washing may be performed with an acidic aqueous solution. Specific examples of the acid include: inorganic acids, such as hydrochloric acid, hydrobromic acid, sulfuric acid, nitric acid, phosphoric acid, and heteropoly acid; and organic acids, such as oxalic acid, fumaric acid, maleic acid, trifluoroacetic acid, methanesulfonic acid, benzenesulfonic acid, p-toluenesulfonic acid, and trifluoromethanesulfonic acid.

The phase-separation washing may be performed with only any one of the basic aqueous solution and the acidic aqueous solution, or may be performed in combination thereof. The phase-separation washing is preferably performed with the basic aqueous solution and the acidic aqueous solution in this order from the viewpoint of removal of a metal impurity.

After the phase-separation washing with the basic aqueous solution and the acidic aqueous solution, washing with neutral water may be subsequently performed. A number of times of the washing may be one or more, and preferably appropriately one to five. As the neutral water, deionized water, ultrapure water, etc., as described above, are used. A number of times of the washing may be one or more, but a few times may fail to remove the basic component and the acidic component. The number of times of the washing is preferably approximately one to five because an expected effect is not always obtained even when the washing is performed 10 times or more.

The reaction product after the phase-separation procedure can be recovered as a powder by concentrating the solvent and solidifying the product under a reduced pressure or a normal pressure, or by performing a crystallization procedure. Alternatively, the reaction product can be in a solution state at an appropriate concentration in order to improve handling for preparing the material for forming an organic film. The concentration in this case is preferably 0.1 mass% to 50 mass%, and more preferably 0.5 mass% to 30 mass%. Such a concentration, which hardly increases the viscosity, can prevent impairing the handling, and is economical because the solvent amount is not excessively large.

The solvent in this case is not particularly limited as long as the solvent can dissolve the compound. Specific examples thereof include: ketones, such as cyclohexanone and methyl 2-amyl ketone; alcohols, such as 3-methoxybutanol, 3-methyl-3-methoxybutanol, 1-methoxy-2-propanol, and 1-ethoxy-2-propanol; ethers, such as propylene glycol monomethyl ether, ethylene glycol monomethyl ether, propylene glycol monoethyl ether, ethylene glycol monoethyl ether, propylene glycol dimethyl ether, and diethylene glycol dimethyl ether; and esters, such as propylene glycol monomethyl ether acetate (PGMEA), propylene glycol monoethyl ether acetate, ethyl lactate, ethyl pyruvate, butyl acetate, methyl 3-methoxypropionate, ethyl 3-ethoxypropionate, tert-butyl acetate, tert-butyl propionate, and propylene glycol mono-tert-butyl ether acetate. These solvents may be used singly, or may be used with mixing two or more kinds thereof.

### Another Method for Manufacturing Compound

When R₃ of the compound represented by the general formula (1A) used in the material for forming an organic film of the present invention is a group other than a hydrogen atom, examples of another manufacturing method include a method via a step of a dehydrative condensation reaction using a fluorenol represented as follows and a benzene or naphthalene having a hydroxy group, namely a phenol or naphthol, as raw materials with an acid catalyst to obtain an intermediate (STEP 1-1). The monomolecular compound can be obtained by a method of performing a substitution reaction (STEP 2-1) by using a raw material represented by R₃-X₂, which has a leaving group X₂ to convert the hydroxy group to OR₃, and using a base catalyst. In this case, R₃-X₂ may be used singly, or in combination of two or more thereof, and regulating the reaction rate can regulate a ratio between the hydroxy group and OR₃. Partially introducing a polar structure such as the hydroxy group can regulate film-formability and adhesion force of the film to the substrate. 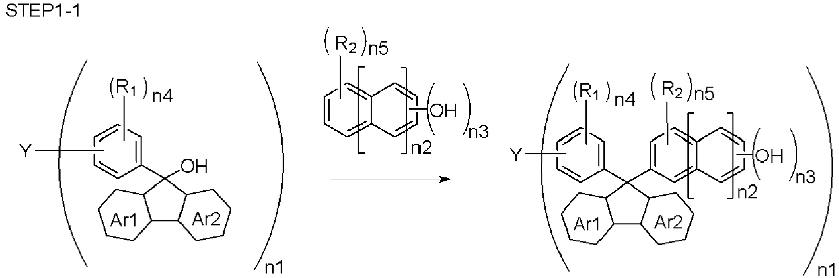 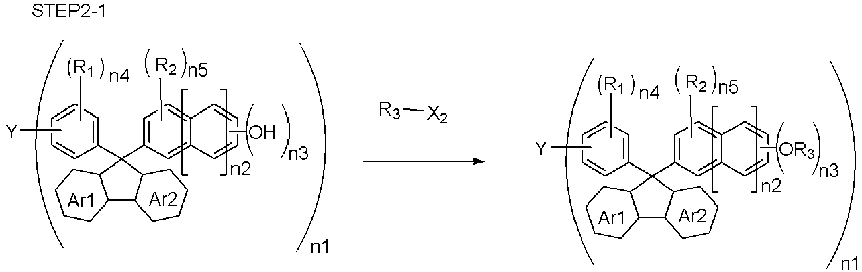 In the formulae, Y, Ar1, Ar2, n1, n2, n3, n4, n5, R₁, R₂, and R₃ represent the same as above; and X₂ represents a halogen atom, tosylate, or mesylate.

The dehydrative condensation reaction in (STEP 1-1) can be performed by a method described in the method for manufacturing the compound of the general formula (1A). The reaction method and the method for recovering the compound can be performed by the methods described in the method for manufacturing the compound of the general formula (1A).

Examples of the base catalyst used in the substitution reaction of (STEP 2-1) include: inorganic base compounds, such as sodium hydrogen carbonate, sodium carbonate, potassium carbonate, calcium carbonate, cesium carbonate, sodium hydroxide, potassium hydroxide, sodium hydride, and calcium phosphate; and organic amine compounds, such as triethylamine, pyridine, and N-methylmorpholine. These may be used singly, or in combination of two or more thereof.

The solvent used in this case is not particularly limited as long as the solvent is inert to the above reaction, and examples of thereof include: ether solvents, such as diethyl ether, tetrahydrofuran, and dioxane; aromatic solvents, such as benzene, toluene, and xylene; acetonitrile, dimethyl sulfoxide, N,N-dimethylformamide, N-methylpyrrolidone, and water. These may be used singly, or may be mixed for use.

The reaction method and the method for recovering the compound may be performed by the methods described in the method for manufacturing the compound represented by the general formula (1A).

For preparing the compound obtained by this method and used in the material for forming an organic film, various halides, tosylates, and mesylates may be used singly or in combination according to the required performance. For example, a compound having a side chain structure to contribute to improvement of the planarization characteristics, and a compound having a rigid aromatic ring structure to contribute to etching resistance and heat resistance may be combined at a given ratio. Therefore, the material for forming an organic film using these compounds can achieve both the filling/planarization characteristics and the etching resistance at a high level.

As above, the compound for forming an organic film of the present invention provides a material for forming an organic film that is cured without generation of byproducts even in film formation in an inert gas, which prevents corrosion of the substrate, that can form an organic film having good adhesiveness to the substrate, and having heat resistance of, for example, 400°C or higher, and advanced filling/planarization characteristics.

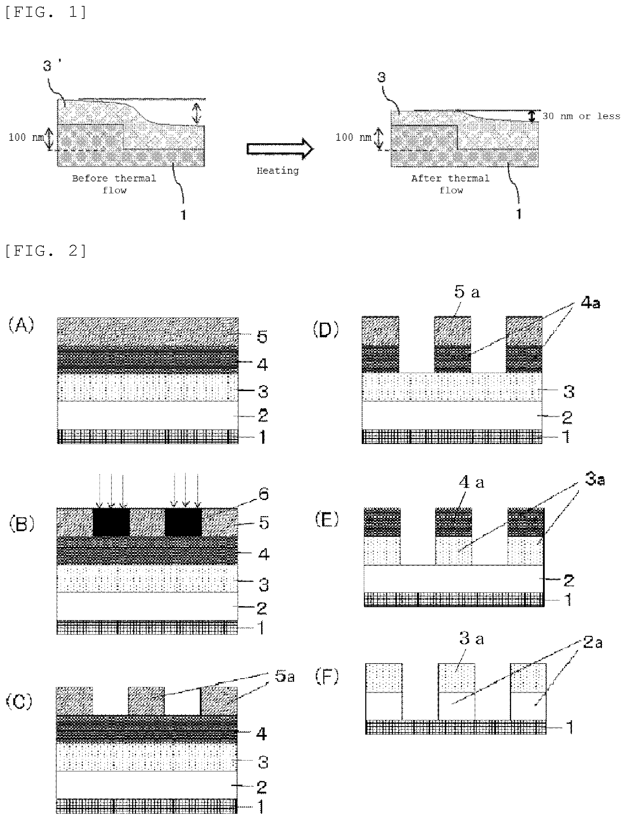

In the present invention, the planarization characteristics refer to performance to planarize a substrate surface. According to the material (composition) containing the compound for forming an organic film of the present invention, as illustrated in FIG. 1, applying a material 3' for forming an organic film on a substrate 1 and heating the coating film to form an organic film 3 can reduce a step with 100 nm on the substrate 1 to a step with 30 nm or less, for example. The step geometry illustrated in FIG. 1 describes a typical example of the step geometry on the substrate for manufacturing a semiconductor device. The step geometry on the substrate that can be planarized with the material containing the compound for forming an organic film of the present invention is of course not limited thereto.

### <Material for Forming Organic Film>

In addition, the present invention provides a material for forming an organic film comprising: the aforementioned compound for forming an organic film (A) of the present invention; and an organic solvent (B). In the material for forming an organic film of the present invention, the aforementioned compound for forming an organic film of the present invention may be used singly, or in combination of two or more thereof. The material for forming an organic film of the present invention may also be referred to as "composition for forming an organic film".

In the present invention, as described above, only two or more substituents represented by R₃ may be used in any combination, and the substitution conversion rate, the proportion of the converted substituent R₃ to a hydroxy group, may be intentionally regulated so as to exhibit the desired performance. Specific examples thereof include, when a proportion of hydrogen atoms is specified as "a" and a proportion of alkyl groups having 1 to 4 carbon atoms and alkynyl groups and alkenyl groups having 2 to 4 carbon atoms is specified as "b" in the structure to be R₃ in the compound, a method of regulating a mole fraction of the hydrogen atoms, which have an expected effect of imparting adhesiveness to the substrate and film-formability, and the substituents of the alkyl groups having 1 to 4 carbon atoms and the alkynyl groups and alkenyl groups having 2 to 4 carbon atoms, which are expected to impart heat resistance and thermal flowability, at any ratio, and the substituent conversion rate is regulated according to the desired performance so that the proportion "b" satisfies a+b = 100 [%]. For regulating the substituent conversion rate, the compound having one or two or more leaving groups X₂ can be used to perform a reaction to obtain the desired compound by the method described in the aforementioned "Another Method for Manufacturing Compound".

### <Organic Solvent>

The organic solvent usable in the material for forming an organic film of the present invention is not particularly limited as long as the solvent dissolves the compound for forming an organic film of the present invention, and an acid generator, a crosslinker, and other additives, which are optional components. Specifically, solvents having a boiling point of lower than 180°C, such as solvents described in paragraphs [0091] to [0092] in JP 2007-199653 A, may be used. Among these, propylene glycol monomethyl ether acetate, propylene glycol monomethyl ether, 2-heptanone, cyclopentanone, cyclohexanone, and a mixture of two or more thereof are preferably used.

Such a material can be applied by spin-coating. In addition, since the material contains the aforementioned compound for forming an organic film of the present invention, the material is a material for forming an organic film having heat resistance of 400°C or higher and high filling/planarization characteristics.

As the organic solvent for the material for forming an organic film of the present invention, a high-boiling-point solvent having a boiling point of 180°C or higher may be blended with the solvent having a boiling point of lower than 180°C (mixture of the solvent having a boiling point of lower than 180°C and a solvent having a boiling point of 180°C or higher). The high-boiling-point solvent is not particularly limited to hydrocarbons, alcohols, ketones, esters, ethers, and chlorine-based solvents as long as the solvent can dissolve the compound for forming an organic film. Specific examples thereof include 1-octanol, 2-ethylhexanol, 1-nonanol, 1-decanol, 1-undecanol, ethylene glycol, 1,2-propylene glycol, 1,3-butylene glycol, 2,4-pentanediol, 2-methyl-2,4-pentanediol, 2,5-hexanediol, 2,4-heptanediol, 2-ethyl-1,3-hexanediol, diethylene glycol, dipropylene glycol, triethylene glycol, tripropylene glycol, glycerin, n-nonyl acetate, ethylene glycol monohexyl ether, ethylene glycol mono-2-ethylhexyl ether, ethylene glycol monophenyl ether, ethylene glycol monobenzyl ether, diethylene glycol monoethyl ether, diethylene glycol monoisopropyl ether, diethylene glycol mono-n-butyl ether, diethylene glycol monoisobutyl ether, diethylene glycol monohexyl ether, diethylene glycol monophenyl ether, diethylene glycol monobenzyl ether, diethylene glycol diethyl ether, diethylene glycol dibutyl ether, diethylene glycol butyl methyl ether, triethylene glycol dimethyl ether, triethylene glycol monomethyl ether, triethylene glycol n-butyl ether, triethylene glycol butyl methyl ether, triethylene glycol diacetate, tetraethylene glycol dimethyl ether, dipropylene glycol monomethyl ether, dipropylene glycol mono-n-propyl ether, dipropylene glycol mono-n-butyl ether, tripropylene glycol dimethyl ether, tripropylene glycol monomethyl ether, tripropylene glycol mono-n-propyl ether, tripropylene glycol mono-n-butyl ether, ethylene glycol monoethyl ether acetate, ethylene glycol monobutyl ether acetate, diethylene glycol monomethyl ether acetate, diethylene glycol monoethyl ether acetate, diethylene glycol monobutyl ether acetate, triacetin, propylene glycol diacetate, dipropylene glycol monomethyl ether acetate, dipropylene glycol methyl n-propyl ether, dipropylene glycol methyl ether acetate, 1,4-butanediol diacetate, 1,3-butylene glycol diacetate, 1,6-hexanediol diacetate, triethylene glycol diacetate, γ-butyrolactone, dihexyl malonate, diethyl succinate, dipropyl succinate, dibutyl succinate, dihexyl succinate, dimethyl adipate, diethyl adipate, and dibutyl adipate. These solvents may be used singly, or may be mixed for use.

The boiling point of the high-boiling-point solvent is appropriately selected depending on a temperature at which the material for forming an organic film is thermally treated. The boiling point of the added high-boiling-point solvent is preferably 180°C to 300°C, and further preferably 200°C to 300°C. Such a boiling point has no risk of excessively fast evaporation during baking (thermal treatment) due to an excessively low boiling point, and yields the sufficient thermal flowability. In addition, such a boiling point eliminates failure to evaporate and remain the solvent in the film even after the baking due to an excessively high boiling point, and thereby has no risk of adverse effect on the film properties such as the etching resistance.

When the above high-boiling-point solvent is used, a blending amount of the high-boiling-point solvent is preferably 1 to 30 parts by mass relative to 100 parts by mass of the solvent having a boiling point of lower than 180°C. Such a blending amount has no risk of an excessively small blending amount to fail to impart sufficient thermal flowability during baking, and no risk of an excessively large blending amount to remain the solvent in the film, leading to deterioration of the film properties such as etching resistance.

The blending amount of the organic solvent is preferably 200 to 10,000 parts (referred to "parts by mass", the same applies hereinafter unless otherwise mentioned), and more preferably 300 to 5,000 parts relative to 100 parts of the compound (A).

Such a material for forming an organic film imparts the thermal flowability with adding the high-boiling-point solvent to the above compound for forming an organic film, resulting in the material for forming an organic film also having advanced
filling/planarization characteristics.

### <Other Components>

The material for forming an organic film of the present invention may further comprise one or more of a photoacid generator (C), a surfactant (D), a crosslinker (E), and a plasticizer (F) according to the purpose.

### (C) Acid Generator

In the material for forming an organic film of the present invention, an acid generator may be added as a component (C) to further accelerate the curing reaction. The acid generator includes a material to generate an acid by pyrolysis and a material to generate an acid by light irradiation, and any of them may be added. Specifically, materials described in paragraphs [0061] to [0085] of JP 2007-199653 A may be added, but the acid generator is not limited thereto.

The acid generator may be used singly, or in combination of two or more kinds thereof. When the acid generator is added, the addition amount is preferably 0.05 to 50 parts, and more preferably 0.1 to 10 parts relative to 100 parts of the compound.

### (D) Surfactant

In the material for forming an organic film of the present invention, a surfactant may be added as a component (D) to improve the coatability by spin-coating. For the surfactant, materials described in [0142] to [0147] of JP 2009-269953 A may be used, for example.

The surfactant may be used singly, or in combination of two or more thereof. When the surfactant is added, the addition amount is preferably 0.01 to 10 parts, and more preferably 0.05 to 5 parts relative to 100 parts of the compound.

### (E) Crosslinker

In the material for forming an organic film of the present invention, a crosslinker may be added as a component (E) to improve the curability and to further inhibit intermixing with the upper layer film. The crosslinker is not particularly limited, and known various types of crosslinkers may be widely used. An example of the crosslinker includes a melamine-based crosslinker, a glycoluril-based crosslinker, a benzoguanamine-based crosslinker, a urea-based crosslinker, a β-hydroxyalkylamide-based crosslinker, an isocyanurate-based crosslinker, an aziridine-based crosslinker, an oxazoline-based crosslinker, and an epoxy-based crosslinker.

Specific examples of the melamine-based crosslinker include hexamethoxymethylmelamine, hexabutoxymethylmelamine, alkoxy- and/or hydroxy-substituted derivatives thereof, and partially self-condensed products thereof. Specific examples of the glycoluril-based crosslinker include tetramethoxymethylglycoluril, tetrabutoxymethylglycoluril, alkoxy- and/or hydroxy-substituted derivatives thereof, and partially self-condensed products thereof. Specific examples of the benzoguanamine-based crosslinker include tetramethoxymethylbenzoguanamine, tetrabutoxymethylbenzoguanamine, alkoxy- and/or hydroxy-substituted derivatives thereof, and partially self-condensed products thereof. Specific examples of the urea-based crosslinker include dimethoxymethyldimethoxyethylene urea, alkoxy- and/or hydroxy-substituted derivatives thereof, and partially self-condensed products thereof. Specific examples of the β-hydroxyalkylamide-based crosslinker include N,N,N',N'-tetra(2-hydroxyethyl)adipamide. Specific examples of the isocyanurate-based crosslinker include triglycidylisocyanurate and triallylisocyanurate. Specific examples of the aziridine-based crosslinker include 4,4'-bis(ethyleneiminocarbonylamino)diphenylmethane and 2,2-bishydroxymethylbutanol-tris[3-(1-aziridinyl)propionate]. Specific examples of the oxazoline-based crosslinker include 2,2'-isopropylidenebis(4-benzyl-2-oxazoline), 2,2'-isopropylidenebis(4-phenyl-2-oxazoline), 2,2'-methylenebis-4,5-diphenyl-2-oxazoline, 2,2'-methylenebis-4-phenyl-2-oxazoline, 2,2'-methylenebis-4-tert-butyl-2-oxazoline, 2,2'-bis(2-oxazoline), 1,3-phenylenebis(2-oxazoline), 1,4-phenylenebis(2-oxazoline), and a 2-isopropenyloxazoline copolymer. Specific examples of the epoxy-based crosslinker include diglycidyl ether, ethylene glycol diglycidyl ether, 1,4-butanediol diglycidyl ether, 1,4-cyclohexanedimethanol diglycidyl ether, poly(glycidyl methacrylate), trimethylolethane triglycidyl ether, trimethylolpropane triglycidyl ether, and pentaerythritol tetraglycidyl ether.

Specific examples of the polynuclear-phenol-based crosslinker include compounds represented by the following general formula (1E), 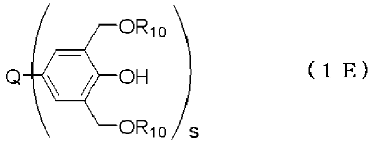 wherein Q represents a single bond or an s-valent hydrocarbon group having 1 to 20 carbon atoms; R₁₀ represents a hydrogen atom or an alkyl group having 1 to 20 carbon atoms; and "s" represents an integer of 1 to 5.

Q represents a single bond or an s-valent hydrocarbon group having 1 to 20 carbon atoms. "s" represents an integer of 1 to 5, and more preferably 2 or 3. Specific examples of Q include groups obtained by removing "s" hydrogen atoms from methane, ethane, propane, butane, isobutane, pentane, cyclopentane, hexane, cyclohexane, methylpentane, methylcyclohexane, dimethylcyclohexane, trimethylcyclohexane, benzene, toluene, xylene, ethylbenzene, ethylisopropylbenzene, diisopropylbenzene, methylnaphthalene, ethylnaphthalene, or icosane. R₁₀ represents a hydrogen atom or an alkyl group having 1 to 20 carbon atoms. Specific examples of the alkyl group having 1 to 20 carbon atoms include a methyl group, an ethyl group, a propyl group, an isopropyl group, a butyl group, an isobutyl group, a pentyl group, an isopentyl group, a hexyl group, an octyl group, an ethylhexyl group, a decyl group, and an icosanyl group. R₁₀ is preferably a hydrogen atom or a methyl group.

Specific examples of the compound represented by the general formula (1E) include the following compounds. Among these, hexamethoxymethylated derivatives of triphenolmethane, triphenolethane, 1,1,1-tris(4-hydroxyphenyl)ethane, and tris(4-hydroxyphenyl)-1-ethyl-4-isopropylbenzene are preferable from the viewpoint of improvement of the curability and uniformity of the film thickness of the organic film. R₁₀ represents the same as above.

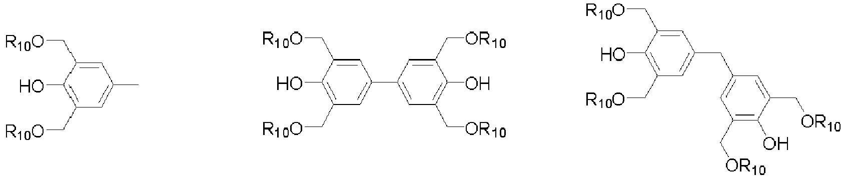

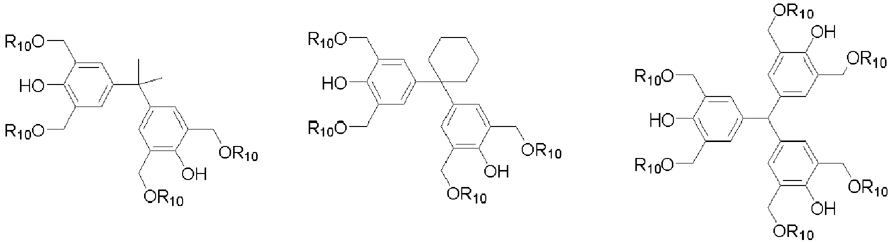

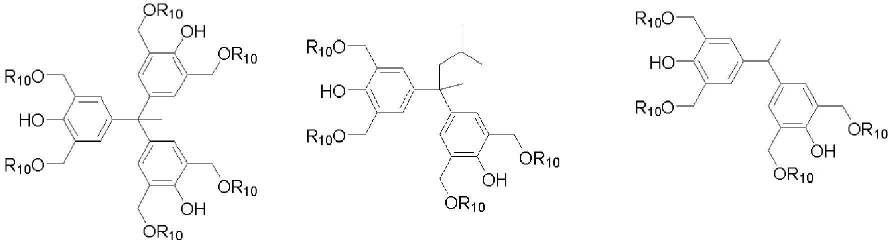

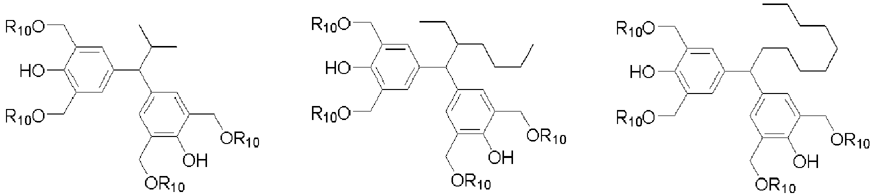

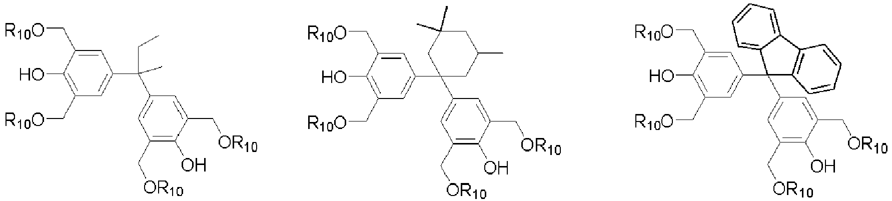

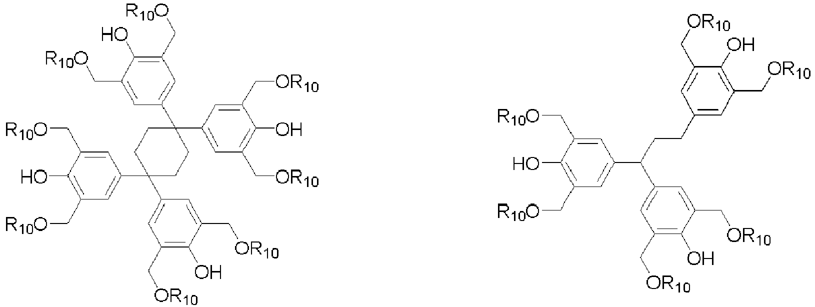

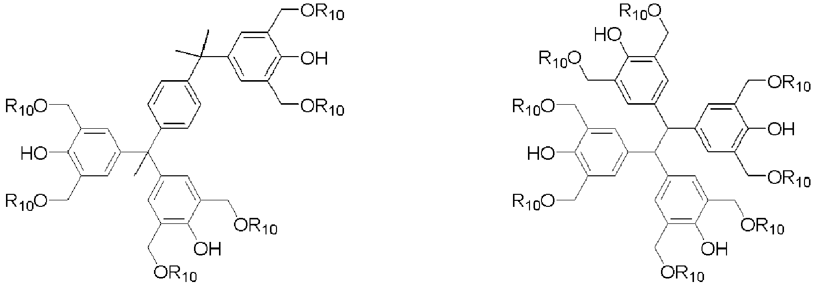

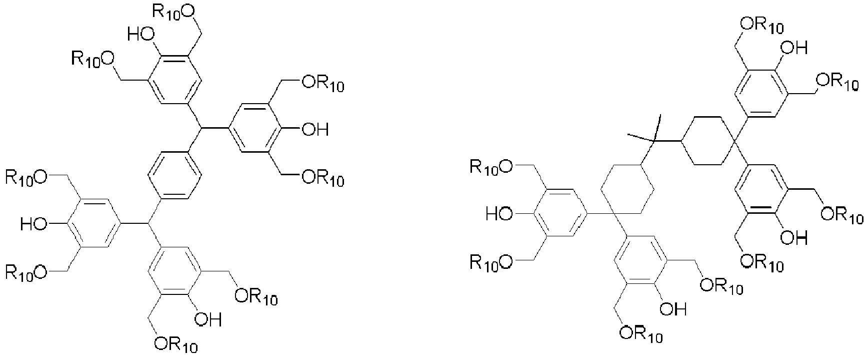

The crosslinker may be used singly, or in combination of two or more thereof. The addition amount of the crosslinker is preferably 1 to 100 parts, and more preferably 5 to 50 parts relative to 100 parts of the compound.

### (F) Plasticizer

In the material for forming an organic film of the present invention, a plasticizer may be added as a component (F) to further improve the planarizing/filling characteristics. The plasticizer is not particularly limited, and known various types of plasticizers may be widely used. An example of the plasticizer includes: low molecular-weight compounds such as phthalate esters, adipate esters, phosphate esters, trimellitate esters, and citrate esters; and polymers such as polyether polymers, polyester polymers, and polyacetal polymers described in JP 2013-253227 A. When the plasticizer is used, the addition amount is preferably 1 to 100 parts, and more preferably 5 to 30 parts relative to 100 parts of the above compound.

In the material for forming an organic film of the present invention, preferably used as additives for imparting the filling/planarizing characteristics similar to the plasticizer are: liquid additives having a polyethylene glycol structure or a polypropylene glycol structure; or pyrolysable polymers having a weight reduction rate of 40 mass% or more between 30°C to 250°C, and having a weight-average molecular weight of 300 to 200,000, for example. This pyrolysable polymer preferably has a repeating unit having an acetal structure represented by the following general formula (DP1) or (DP1a). When these pyrolysable polymers are added, the addition amount is preferably 1 to 100 parts, and more preferably 5 to 30 parts relative to 100 parts of the above compound. 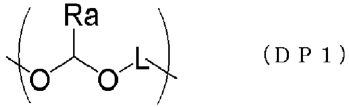 In the formula, Ra represents a hydrogen atom, or a saturated or unsaturated monovalent organic group having 1 to 30 carbon atoms and optionally having a substituent; and L represents a saturated or unsaturated divalent organic group having 2 to 30 carbon atoms. 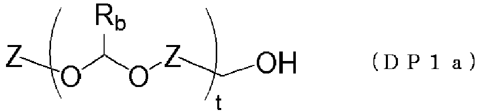 In the formula, Rb represents an alkyl group having 1 to 4 carbon atoms; Z represents a saturated or unsaturated divalent hydrocarbon group having 4 to 10 carbon atoms and optionally having an ether bond; and "t" represents an average number of the repeating units, and is 3 to 500.

As an additive for improving the filling/planarization characteristics similar to the above, the following flowability accelerator may be used. As the flowability accelerator, one or more compounds selected from the following general formulae (i) to (iii) may be used. When these flowability accelerators are added, the addition amount is preferably 1 to 100 parts, and more preferably 5 to 30 parts relative to 100 parts of the above compound.

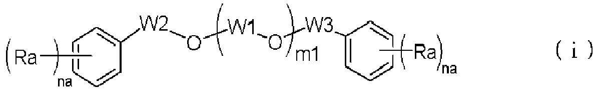

In the formula, Ra each independently represents a hydrogen atom, hydroxy group, or an optionally substituted organic group having 1 to 10 carbon atoms; W1 represents a phenylene group or a divalent group represented by the following general formula (i-1); W2 and W3 represent a single bond or any one divalent group represented by the following general formula (i-2); m1 represents an integer of 1 to 10; and "na" represents an integer of 0 to 5.

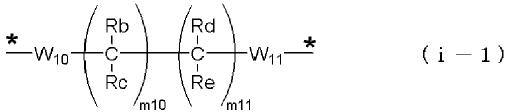

In the formula, "*" represents a bonding position; Rb, Rc, Rd, and Re represent a hydrogen atom, a hydroxy group, or an organic group having 1 to 10 carbon atoms; W10 and W11 each independently represent a single bond or a carbonyl group; m10 and m11 represent an integer of 0 to 10; and m10+n11 ≥1.

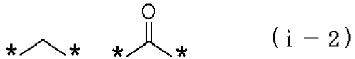

In the formulae, "*" represents a bonding position. 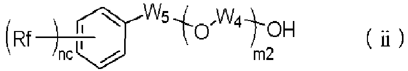 In the formula, Rf each independently represents a hydrogen atom or an optionally substituted organic group having 1 to 10 carbon atoms; W4 represents a divalent group represented by the following general formula (ii-1); W5 represents a single bond or any one divalent group represented by the following general formula (ii-2); m2 represents an integer of 2 to 10; and "nc" represents an integer of 0 to 5.

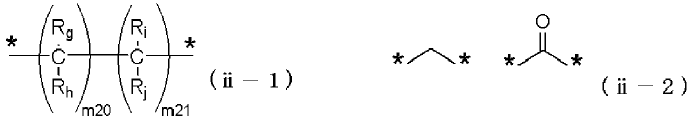

In the formula, "*" represents a bonding position; Rg, Rh, Ri, and Rj represent a hydrogen atom, a hydroxy group, or an organic group having 1 to 10 carbon atoms; m20 and m21 represent an integer of 0 to 10; and m20+m21 > 1. 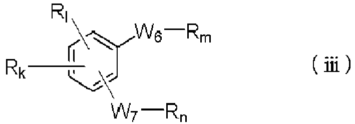 In the formula, Rk and Rl represent a hydrogen atom, a hydroxy group, or an optionally substituted organic group having 1 to 10 carbon atoms, and optionally bonded to form a cyclic structure; Rm and Rn represent an organic group having 1 to 10 carbon atoms, and Rm represents a group having any one of an aromatic ring or a divalent group represented by the following general formula (iii-1); and W₆ and W₇ represent a single bond or any one divalent group represented by the following general formula (iii-2), and at least one of W₆ and W₇ represents the divalent group represented by the following general formula (iii-2).

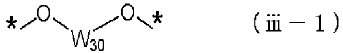

In the formula, "*" represents a bonding position; and W30 represents an organic group having 1 to 4 carbon atoms.

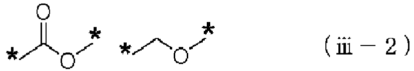

In the formulae, "*" represents a bonding position.

As above, the material for forming an organic film of the present invention is the material for forming an organic film that is cured without generation of byproducts even in film formation in an inert gas, which prevents corrosion of the substrate, and that has good adhesiveness to the substrate, high heat resistance of, for example, 400°C or higher, and advanced filling/planarization characteristics. Therefore, the material for forming an organic film of the present invention is extremely useful as the material for forming an organic film in the multilayer resist method such as: the two-layer resist method; the three-layer resist method using the silicon-containing resist intermediate film or the inorganic hard mask (for example, the silicon-containing inorganic hard mask); and the four-layer resist method using the silicon-containing resist intermediate film or the inorganic hard mask, and the organic anti-reflection film. Since the material for forming an organic film of the present invention generates no byproduct even in film formation in an inert gas, and has excellent filling/planarization characteristics, the material can be suitably used as a planarization material in the semiconductor device manufacturing process other than the multilayer resist method. That is, the material for forming an organic film of the present invention can be suitably used in the semiconductor device manufacturing process.

### <Substrate for Manufacturing Semiconductor Device>

The present invention provides a substrate for manufacturing a semiconductor device comprising a cured organic film of the material for forming an organic film of the present invention on a substrate.

It can also be mentioned that the substrate for manufacturing a semiconductor device of the present invention comprises: a substrate; and an organic film formed on the substrate and being a cured product of the material for forming an organic film of the present invention.

Such a substrate for manufacturing a semiconductor device has the organic film having high heat resistance, good adhesiveness to the substrate, and advanced filling/planarization characteristics, and thereby an yield of the semiconductor devices becomes good when the substrate is used in the semiconductor device manufacturing process.

### <Method for Forming Organic Film>

Further, the present invention provides a method for forming an organic film using the material for forming an organic film of the present invention and applied in the semiconductor device manufacturing process. Specific examples thereof include the following method.

### (One-Step Baking Method (in Inert Gas))

A method for forming an organic film applied in a semiconductor device manufacturing process, the method comprising steps of: applying the material for forming an organic film of the present invention on a substrate to be processed by spin-coating; and heat-treating the substrate to be processed coated with the material for forming an organic film under an inert gas atmosphere at a temperature of 50°C or higher and 600°C or lower within a range of 5 seconds to 7200 seconds to obtain a cured film.

### (Two-Step Baking Method (in Air - in Inert Gas))

A method for forming an organic film applied in a semiconductor device manufacturing process, the method comprising steps of: applying the material for forming an organic film of the present invention on a substrate to be processed by spin-coating; heat-treating the substrate to be processed coated with the material for forming an organic film in air at a temperature of 50°C or higher and 300°C or lower within a range of 5 seconds to 600 seconds to form an applied film; and subsequently heat-treating the applied film under an inert gas atmosphere at a temperature of 200°C or higher and 600°C or lower within a range of 10 seconds to 7200 seconds to obtain a cured film.

In the method for forming an organic film of the present invention, first, the aforementioned material for forming an organic film of the present invention is rotationally applied on the substrate to be processed (spin-coating).

In the method for forming an organic film of the present invention, the substrate to be processed coated with the material for forming an organic film is subsequently subjected to a thermal treatment (heat-film-forming step) to obtain the cured film.

For the heat-film-forming step in order to form the cured film, the one-step baking, the two-step baking, or multistep baking of three or more steps may be applied, but the one-step baking or the two-step baking is economically preferable.

The film formation with the one-step baking is performed at a temperature of 50°C or higher and 600°C or lower within a range of 5 seconds to 7200 seconds, preferably at a temperature of 150°C or higher and 500°C or lower within a range of 10 to 7200 seconds (and more preferably within a range of 10 to 3600 seconds). The thermal treatment under such conditions can promote planarization with thermal flow and the crosslinking reaction. In the multilayer resist method, the applying-type silicon-containing resist intermediate film or the CVD hard mask may be formed on this obtained film. When the applying-type silicon-containing resist intermediate film is applied, the film formation with the one-step baking is preferably performed at a temperature higher than a temperature of formation of the silicon-containing resist intermediate film. The silicon-containing resist intermediate film is typically formed at 100°C or higher and 400°C or lower, and preferably 150°C or higher and 350°C or lower. Formation of the organic film at a temperature higher than this temperature can prevent dissolution of the organic film due to the silicon-containing resist intermediate film material to form the organic film without mixing with this material.

When the CVD hard mask is applied, the organic film is preferably formed at a temperature higher than a temperature for forming the CVD hard mask. Examples of the temperature for forming the CVD hard mask include a temperature of 150°C or higher and 500°C or lower.

Examples of an atmosphere during the baking include an inert gas such as nitrogen, argon, and helium. The material of the present invention can form the sufficiently cured organic film without generation of sublimates even when baked under such an inert gas atmosphere. In addition, the material of the present invention can be cured without generation of byproducts even when formed in the inert gas, which prevents corrosion of the substrate.

Specifically, one aspect of the method for forming an organic film of the present invention is a method for forming an organic film applied in a semiconductor device manufacturing process, the method comprising steps of: applying the material for forming an organic film of the present invention on a substrate to be processed by spin-coating; and heat-treating the substrate to be processed coated with the material for forming an organic film under an inert gas atmosphere at a temperature of 50°C or higher and 600°C or lower within a range of 5 seconds to 7200 seconds to obtain a cured film.

Meanwhile, in the film formation with the two-step baking, the substrate to be processed coated with the material for forming an organic film of the present invention can be subjected to the first step baking of a thermal treatment in air and the subsequent second step baking under an inert gas atmosphere, for example.

With considering an effect of corrosion of the substrate due to oxygen in air, an upper limit of the treating temperature in air of the first step baking is 300°C or lower, and preferably 250°C or lower, and performed within a range of 5 to 600 seconds. A lower limit of the treating temperature in air may be 50°C, for example.

Examples of an atmosphere during the second step baking include an inert gas such as nitrogen, argon, and helium. The material of the present invention can form the sufficiently cured organic film without generation of sublimates even when baked under such an inert gas atmosphere. In addition, the material of the present invention can be cured without generation of byproducts even when formed in the inert gas, which prevents corrosion of the substrate.

The film formation with the second step baking is preferably performed at a temperature higher than the baking temperature of the first step, for example, 200°C or higher, and 600°C or lower, and preferably 500°C or lower within a range of 10 to 7200 seconds. In the multilayer resist method, the applying-type silicon-containing resist intermediate film or the CVD hard mask may be formed on this obtained film. When the applying-type silicon-containing resist intermediate film is applied, the film formation is preferably performed at a temperature higher than a temperature for forming the silicon-containing resist intermediate film. The silicon-containing resist intermediate film is typically formed at 100°C or higher and 400°C or lower, and preferably 150°C or higher and 350°C or lower. Formation of the organic film at a temperature higher than this temperature can prevent dissolution of the organic film due to the silicon-containing resist intermediate film material to form the organic film without mixing with this material.

When the CVD hard mask is applied with the two-step baking, the organic film is preferably formed at a temperature higher than a temperature for forming the CVD hard mask. Examples of the temperature for forming the CVD hard mask include a temperature of 150°C or higher and 500°C or lower.

Specifically, one aspect of the method for forming an organic film of the present invention is a method for forming an organic film applied in a semiconductor device manufacturing process, the method comprising steps of: applying the material for forming an organic film of the present invention on a substrate to be processed by spin-coating; heat-treating the substrate to be processed coated with the material for forming an organic film in air at a temperature of 50°C or higher and 300°C or lower within a range of 5 seconds to 600 seconds to form an applied film; and subsequently heat-treating the applied film under an inert gas atmosphere at a temperature of 200°C or higher and 600°C or lower within a range of 10 seconds to 7200 seconds to obtain a cured film.

An oxygen concentration in the inert gas is preferably 1 vol% or less.

The material for forming an organic film of the present invention can be sufficiently cured without generation of sublimates even when heated (subjected to the thermal treatment) under such an inert gas atmosphere, and can form the organic film having excellent adhesiveness to the substrate. In addition, heating under such an inert gas atmosphere can prevent corrosion of the substrate to be processed.

Specifically, the present invention provides a method for forming an organic film that functions as an organic film used in a semiconductor device manufacturing process, the method comprising steps of subjecting the substrate to be processed to the thermal treatment under the inert gas atmosphere to form an cured film, wherein the thermal treatment is performed under an atmosphere with an oxygen concentration of 1 vol% or less in order to prevent corrosion of the substrate to be processed.

In this method for forming an organic film, the aforementioned material for forming an organic film of the present invention is firstly applied on the substrate to be processed by spin-coating. After the spin-coating, the material is baked in air at 300°C or lower, and then subjected to the second baking under the atmosphere with an oxygen concentration of 1 vol% or less in a case of the two-step baking. In a case of the one-step baking, the first step baking in air is skipped. Examples of the atmosphere during the baking include an inert gas such as nitrogen, argon, and helium. The material of the present invention can form the sufficiently cured organic film without generation of sublimates even when baked under such an inert gas atmosphere.

In the method for forming an organic film of the present invention, a substrate to be processed having a structure or step with 30 nm or more in height may be used. As noted above, the material for forming an organic film of the present invention has excellent filling/planarization characteristics, and thereby the plane cured film can be formed even when the substrate to be processed has a structure or step (roughness) with 30 nm or more in height. That is, the method for forming an organic film of the present invention is particularly useful when a plane organic film is formed on such a substrate to be processed.

A thickness of the organic film to be formed is appropriately selected, but preferably 30 to 20,000 nm, and particularly preferably 50 to 15,000 nm.

The above method for forming an organic film can be applied for both cases of: forming the organic film for an organic film using the material for forming an organic film of the present invention; and forming the organic film for a planarizing film.

For example, one modification of the method for forming an organic film of the present invention is a method for forming an organic film that can planarize a surface of a stepped substrate used in a semiconductor apparatus manufacturing process, the method comprising steps of: applying the aforementioned material for forming an organic film of the present invention on a substrate to be processed by spin-coating; heat-treating the substrate to be processed coated with the material for forming an organic film in air at a temperature of 50°C or higher and 300°C or lower within a range of 5 seconds to 600 seconds to form an applied film; and subsequently heat-treating the applied film under an inert gas atmosphere at a temperature of 200°C or higher and 600°C or lower for 10 seconds to 7200 seconds to form a cured film.

In this method for forming an organic film, first, the aforementioned material for forming an organic film of the present invention is rotationally applied on the substrate to be processed (spin-coating). Using the spin-coating method can yield good filling characteristics. After the spin-coating, baking (thermal treatment) is performed in order to promote planarization with thermal flow and the crosslinking reaction. Since this baking can evaporate the solvent in the material, mixing can be inhibited even when the resist upper layer film or the silicon-containing resist intermediate film is formed on the organic film.

### <Patterning Process>

The material for forming an organic film of the present invention can be suitably used for various patterning processes such as: a three-layer resist process using the silicon-containing resist intermediate film or the inorganic hard mask; and a four-layer resist process additionally using the organic anti-reflection film. In a semiconductor device manufacturing process, forming the circuit pattern by the patterning process of the present invention as above can manufacture the semiconductor devices with a good yield. Hereinafter, examples of the patterning process using the material for forming an organic film of the present invention will be described.
(1) A patterning process, comprising steps of: forming an organic film on a body to be processed by using the material for forming an organic film of the present invention; forming a silicon-containing resist intermediate film on the organic film by using a silicon-containing resist intermediate film material; forming a resist upper layer film on the silicon-containing resist intermediate film by using a photoresist composition; forming a circuit pattern in the resist upper layer film; transferring the pattern to the silicon-containing resist intermediate film by etching while using the patterned resist upper layer film as a mask; transferring the pattern to the organic film by etching while using the patterned silicon-containing resist intermediate film as a mask; and transferring the pattern to the body to be processed by etching while using the patterned organic film as a mask.
(2) A patterning process, comprising steps of: forming an organic film on a body to be processed by using the composition for forming an organic film of the present invention; forming a silicon-containing resist intermediate film on the organic film by using a silicon-containing resist intermediate film material; forming an organic anti-reflection film on the silicon-containing resist intermediate film; forming a resist upper layer film on the organic anti-reflection film by using a photoresist composition to form a four-layer film structure; forming a circuit pattern in the resist upper layer film; transferring the pattern to the organic anti-reflection film and the silicon-containing resist intermediate film by etching while using the patterned resist upper layer film as a mask; transferring the pattern to the organic film by etching while using the patterned silicon-containing resist intermediate film as a mask; and transferring the pattern to the body to be processed by etching while using the patterned organic film as a mask.
(3) A patterning process, comprising steps of: forming an organic film on a body to be processed by using the composition for forming an organic film of the present invention; forming an inorganic hard mask selected from a silicon oxide film, a silicon nitride film, a silicon oxynitride film, a titanium oxide film, and a titanium nitride film on the organic film; forming a resist upper layer film on the inorganic hard mask by using a photoresist composition; forming a circuit pattern in the resist upper layer film; transferring the pattern to the inorganic hard mask by etching while using the patterned resist upper layer film as a mask; transferring the pattern to the organic film by etching while using the patterned inorganic hard mask as a mask; and transferring the pattern to the body to be processed by etching while using the patterned organic film as a mask.
(4) A patterning process, comprising steps of: forming an organic film on a body to be processed by using the composition for forming an organic film of the present invention; forming an inorganic hard mask selected from a silicon oxide film, a silicon nitride film, a silicon oxynitride film, a titanium oxide film, and a titanium nitride film on the organic film; forming an organic anti-reflection film on the inorganic hard mask; forming a resist upper layer film on the organic anti-reflection film by using a photoresist composition to form a four-layer film structure; forming a circuit pattern in the resist upper layer film; transferring the pattern to the organic anti-reflection film and the inorganic hard mask by etching while using the patterned resist upper layer film as a mask; transferring the pattern to the organic film by etching while using the patterned inorganic hard mask as a mask; and transferring the pattern to the body to be processed by etching while using the patterned organic film as a mask.

Hereinafter, the patterning process will be described with examples, but the patterning process is not limited thereto.

### Three-Layer Resist Method using Silicon-Containing Resist Intermediate Film

In addition, the present invention provides a patterning process comprising steps of: forming an organic film on a body to be processed by using the aforementioned material for forming an organic film of the present invention; forming a silicon-containing resist intermediate film on the organic film by using a silicon-containing resist intermediate film material; forming a resist upper layer film on the silicon-containing resist intermediate film by using a photoresist composition; forming a circuit pattern in the resist upper layer film; transferring the pattern to the silicon-containing resist intermediate film by etching the silicon-containing resist intermediate film while using the patterned resist upper layer film as a mask; transferring the pattern to the organic film by etching the organic film while using the patterned silicon-containing resist intermediate film as a mask; and transferring the pattern to the body to be processed by etching the body to be processed while using the patterned organic film as a mask.

As the body to be processed, a semiconductor device substrate, or a substrate in which any of a metal film, a metal carbide film, a metal oxide film, a metal nitride film, a metal oxycarbide film, and a metal oxynitride film is formed on the semiconductor device substrate is preferably used. More specifically, a substrate such as Si, α-Si, p-Si, SiO₂, SiN, SiON, W, TiN, and Al; a substrate in which the above metal film, etc. is formed on the substrate as a layer to be processed; etc. are used, but not particularly limited thereto.

As the layer to be processed, various low-k films such as Si, SiO₂, SiON, SiN, p-Si, α-Si, W, W-Si, Al, Cu, and Al-Si, and stopper films thereof are used. The layer to be processed can be formed to have a thickness of typically 50 to 10,000 nm, and particularly 100 to 5,000 nm. When the layer to be processed is formed, the substrate and the layer to be processed having different materials are used.

A metal to constitute the body to be processed preferably contains silicon, titanium, tungsten, hafnium, zirconium, chromium, germanium, copper, silver, gold, aluminum, indium, gallium, arsenic, palladium, iron, tantalum, iridium, cobalt, manganese, molybdenum, or an alloy thereof.

A substrate to be processed having a structure or step with 30 nm or more in height may be used as the substrate to be processed.

For forming the organic film on the body to be processed by using the material for forming an organic film of the present invention, the aforementioned method for forming an organic film of the present invention is applied.

Then, the resist intermediate film (silicon-containing resist intermediate film) is formed on the organic film by using the resist intermediate film material containing a silicon atom. As the silicon-containing resist intermediate film material, a polysiloxane-based intermediate film material is preferable. Imparting an anti-reflective effect to the silicon-containing resist intermediate film can inhibit reflection. In particular, using a material for 193-nm exposure containing a large amount of aromatic groups and having high etching selectivity to the substrate as the composition for forming an organic film increases the k-value to increase reflection on the substrate. However, the silicon-containing resist intermediate film having the absorption so as to have an appropriate k-value can inhibit the reflection, resulting in the substrate reflection of 0.5% or less. Preferably used for the silicon-containing resist intermediate film having the anti-reflective effect are anthracene for 248-nm or 157-nm exposure, and a polysiloxane crosslinkable with an acid or heat for 193-nm exposure. The polysiloxane has a light absorption group having a phenyl group or silicon-silicon bond at the pendant structure or in the polysiloxane structure.

Then, the resist upper layer film is formed on the silicon-containing resist intermediate film by using the resist upper layer film material composed of the photoresist composition. The resist upper layer film material may be any of positive-type or negative-type, and a material same as commonly used photoresist compositions may be used. After the resist upper layer film material is applied by spin-coating, the pre-baking is preferably performed within a range at 60 to 180°C for 10 to 300 seconds. Thereafter, the exposure, then post exposure baking (PEB), and development are performed in accordance with a common method to obtain the resist upper layer film pattern. A thickness of the resist upper layer film is not particularly limited, and preferably 30 to 500 nm, particularly preferably 50 to 400 nm.

Then, the circuit pattern (resist upper layer film pattern) is formed in the resist upper layer film. The circuit pattern is preferably formed by lithography using light having a wavelength of 10 nm or longer and 300 nm or shorter, direct writing with electron beam, nanoimprinting, or a combination thereof.

Examples of the exposure light include high-energy ray having a wavelength of 300 nm or shorter, and specifically, far ultraviolet ray, KrF excimer laser light (248 nm), ArF excimer laser light (193 nm), F₂ laser light (157 nm), Kr₂ laser light (146 nm), Ar₂ laser light (126 nm), soft X-ray with 3 to 20 nm (EUV), electron beam (EB), ion beam, and X-ray.

The circuit pattern is preferably developed with an alkali or an organic solvent.

Then, the pattern is transferred to the silicon-containing resist intermediate film by etching the silicon-containing resist intermediate film while using the circuit-patterned resist upper layer film as a mask. The etching of the silicon-containing resist intermediate film while using the resist upper layer film pattern as a mask is preferably performed by using a fluorocarbon-based gas. This etching forms the silicon-containing resist intermediate film pattern.

Then, the pattern is transferred to the organic film by etching the organic film while using the patterned silicon-containing resist intermediate film as a mask. Since the silicon-containing resist intermediate film has etching resistance higher than the organic film against oxygen gas or hydrogen gas, the etching of the organic film while using the silicon-containing resist intermediate film pattern as a mask is preferably performed by using an etching gas mainly composed of oxygen gas or hydrogen gas. This etching can form the organic film pattern.

Then, the pattern is transferred to the body to be processed by etching the body to be processed while using the patterned organic film as a mask. The subsequent etching of the body to be processed (layer to be processed) may be performed by a common method. For example, when the body to be processed is a SiO₂-based, SiN-based, or silica-based low dielectric-constant insulative film, the etching is performed mainly using a fluorocarbon-based gas. When the body to be processed is p-Si, Al, or W, the etching is performed mainly using a chlorine-based or bromine-based gas. When the substrate is processed by etching with the fluorocarbon-based gas, the silicon-containing resist intermediate film pattern is simultaneously removed with the substrate processing. Meanwhile, when the substrate is processed by etching with the chlorine-based or bromine-based gas, dry-etching removal with the fluorocarbon-based gas is required to be separately performed after the substrate processing in order to remove the silicon-containing resist intermediate film pattern.

The organic film obtained by using the composition for forming an organic film of the present invention has excellent etching resistance during the etching of the body to be processed as above.

### Four-Layer Resist Method using Silicon-Containing Resist Intermediate Film and Organic Anti-Reflection Film

In addition, the present invention provides a patterning process comprising steps of: forming an organic film on a body to be processed by using the aforementioned composition for forming an organic film of the present invention; forming a silicon-containing resist intermediate film on the organic film by using a silicon-containing resist intermediate film material; forming an organic anti-reflection film on the silicon-containing resist intermediate film; forming a resist upper layer film on the organic anti-reflection film by using a photoresist composition to form a four-layer film structure; forming a circuit pattern in the resist upper layer film; transferring the pattern to the organic anti-reflection film and the silicon-containing resist intermediate film by etching the organic anti-reflection film and the silicon-containing resist intermediate film while using the patterned resist upper layer film as a mask; transferring the pattern to the organic film by etching the organic film while using the patterned silicon-containing resist intermediate film as a mask; and transferring the pattern to the body to be processed by etching the body to be processed while using the patterned organic film as a mask.

This method can be performed in the same manner as the above three-layer resist method using the silicon-containing resist intermediate film except that the organic anti-reflective film (BARC) is formed between the silicon-containing resist intermediate film and the resist upper layer film.

The organic anti-reflective film may be formed with spin-coating by using a known organic anti-reflective film material.

### Three-Layer Resist Method using Inorganic Hard Mask

In addition, the present invention provides, as a patterning process with a three-resist method using the aforementioned composition for forming an organic film of the present invention, patterning process comprising steps of: forming an organic film on a body to be processed by using the composition for forming an organic film of the present invention; forming an inorganic hard mask selected from a silicon oxide film, a silicon nitride film, a silicon oxynitride film, a titanium oxide film, and a titanium nitride film on the organic film; forming a resist upper layer film on the inorganic hard mask by using a photoresist composition; forming a circuit pattern in the resist upper layer film; transferring the pattern to the inorganic hard mask by etching the inorganic hard mask while using the patterned resist upper layer film as a mask; transferring the pattern to the organic film by etching the organic film while using the patterned inorganic hard mask as a mask; and transferring the pattern to the body to be processed by etching the body to be processed while using the patterned organic film as a mask.

This method can be performed in the same manner as the above three-layer resist method using the silicon-containing resist intermediate film except that the inorganic hard mask is formed on the organic film instead of the silicon-containing resist intermediate film.

The inorganic hard mask selected from a silicon oxide film, a silicon nitride film, and a silicon oxynitride film (SiON film) can be formed by a CVD method, an ALD method, etc. The method for forming the silicon nitride film is described in, for example, JP 2002-334869 A, WO 2004/066377, etc. A film thickness of the inorganic hard mask is preferably 5 to 200 nm, and more preferably 10 to 100 nm. As the inorganic hard mask, the SiON film, which is highly effective as the anti-reflective film, is most preferably used. Since the substrate temperature during the SiON film formation reaches 300 to 500°C, the organic film is required to resist the temperature of 300 to 500°C. The organic film formed by using the material for forming an organic film of the present invention has high heat resistance and can resist the high temperature of 300°C to 500°C, and thereby the inorganic hard mask formed by the CVD method or the ALD method and the organic film formed by the spin-coating method can be combined.

The inorganic hard mask selected from the titanium oxide film and the titanium nitride film can also be formed by the CVD method, the ALD method, etc.

### Four-Layer Resist Method using Inorganic Hard Mask and Organic Anti-Reflection Film

In addition, the present invention provides, as a patterning process with a four-resist method using the aforementioned composition for forming an organic film of the present invention, a patterning process comprising steps of: forming an organic film on a body to be processed by using the composition for forming an organic film of the present invention; forming an inorganic hard mask selected from a silicon oxide film, a silicon nitride film, a silicon oxynitride film, a titanium oxide film, and a titanium nitride film on the organic film; forming an organic anti-reflection film on the inorganic hard mask; forming a resist upper layer film on the organic anti-reflection film by using a photoresist composition to form a four-layer film structure; forming a circuit pattern in the resist upper layer film; transferring the pattern to the organic anti-reflection film and the inorganic hard mask by etching the organic anti-reflection film and the inorganic hard mask while using the patterned resist upper layer film as a mask; transferring the pattern to the organic film by etching the organic film while using the patterned inorganic hard mask as a mask; and transferring the pattern to the body to be processed by etching the body to be processed while using the patterned organic film as a mask.

This method can be performed in the same manner as the above three-layer resist method using the inorganic hard mask except that the organic anti-reflective film (BARC) is formed between the inorganic hard mask and the resist upper layer film.

In particular, when the SiON film is used as the inorganic hard mask, the reflection can be inhibited by the two anti-reflective films of the SiON film and the BARC even with immersion exposure having high NA of more than 1.0. Another merit of forming the BARC is an effect of reducing bottom footing of the resist upper layer film pattern just on the SiON film.

Here, an example of the patterning process of the present invention with the three-layer resist method is described in FIGS. 2(A) to (F). In the three-layer resist method, as illustrated in FIG. 2(A), an organic film 3 is formed by using the composition for forming an organic film of the present invention on a layer 2 to be processed formed on a substrate 1, then a silicon-containing resist intermediate film 4 is formed, and a resist upper layer film 5 is formed thereon. Then, as illustrated in FIG. 2(B), an exposure portion 6 in the resist upper layer film 5 is exposed, and post exposure baking (PEB) is performed. Thereafter, as illustrated in FIG. 2(C), development is performed to form a resist upper layer film pattern 5a. Subsequently, as illustrated in FIG. 2(D), the silicon-containing resist intermediate film 4 is processed by dry etching using a fluorocarbon-based gas while using the resist upper layer film pattern 5a as a mask to form a silicon-containing resist intermediate film pattern 4a. Then, as illustrated in FIG. 2(E), the resist upper layer film pattern 5a is removed, and then the organic film 3 is etched with oxygen plasma while using the silicon-containing resist intermediate film pattern 4a as a mask to form an organic film pattern 3a. Further, as illustrated FIG. 2(F), the silicon-containing resist intermediate film pattern 4a is removed, and then the layer 2 to be processed is processed by etching while using the organic film pattern 3a as a mask to form a pattern 2a.

When the inorganic hard mask is formed, the silicon-containing resist intermediate film 4 is replaced with the inorganic hard mask. When the BARC is formed, the BARC is formed between the silicon-containing resist intermediate film 4 and the resist upper layer film 5. The BARC may be subsequently etched prior to the etching of the silicon-containing resist intermediate film 4. Alternatively, only the BARC may be etched, and then the etching apparatus may be changed, etc. to etch the silicon-containing resist intermediate film 4.

As above, the patterning process of the present invention can form a fine pattern in the body to be processed with high accuracy using the multilayer resist method.

### EXAMPLE

Hereinafter, the present invention will be described more specifically with showing Synthesis Examples, Comparative Synthesis Examples, Examples, and Comparative Examples, but the present invention is not limited by these examples. As for a molecular weight and a dispersion degree, determined were a weight-average molecular weight (Mw) and a number-average molecular weight (Mn) in terms of polystyrene by gel permeation chromatography (GPC) with tetrahydrofuran as an eluent, and a dispersion degree (Mw/Mn) was determined.

### Synthesis Example: Synthesis of Compound for Forming Organic Film

For synthesizing compounds for forming an organic film (A1) to (A13), fluorenols (B1) to (B7), and phenols and naphthols (C1) to (C4) shown below were used.

### Fluorenols:

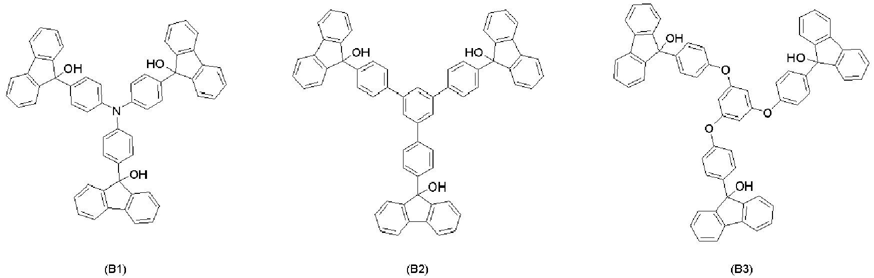

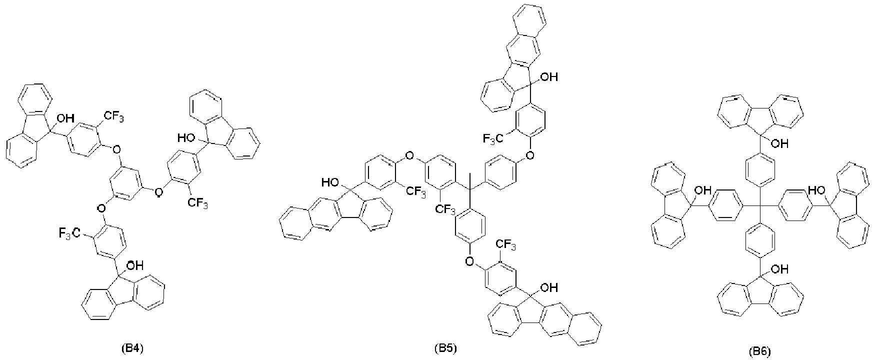

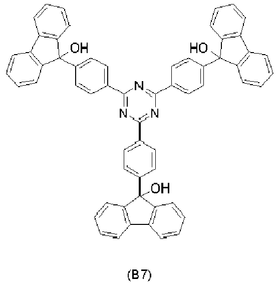

### Phenols or Naphthols:

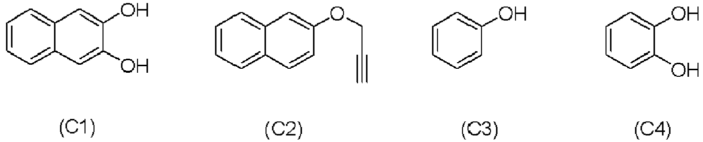

As a synthesis examples of the fluorenols, a synthesis example of (B1) will be shown below. Synthesis Example 1: Synthesis of Fluorenol (B1)

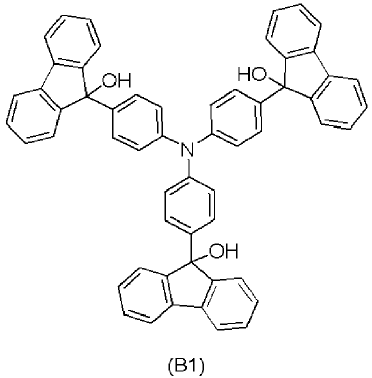

Into a mixed liquid of 189.9 g of tris(4-bromophenyl)amine (D1), 1000 mL of t-butyl methyl ether (MTBE), and 1000 ml of tetrahydrofuran (THF), which was cooled to -20°C under a N₂ atmosphere, 500 mL of a 2.60-M n-butyllithium hexane solution was added, and the mixture was stirred at -20°C for 20 minutes. A 25-wt% THF solution in which 191.7 g of 9-fluorenone was dissolved in THF in advance was slowly added dropwise, the temperature was gradually raised to room temperature, and the mixture was stirred at room temperature for 4 hours. Into the mixture, 1000 ml of a saturated ammonium chloride aqueous solution was added to terminate the reaction, then 2000 ml of MTBE was added, and the separated aqueous layer was removed. The organic layer was washed with 500 ml of pure water five times, and then the solvent was distilled off. After the distillation, 500 g of THF was added, and then 2000 g of methanol was further added to perform recrystallization. The precipitated crystal was separated by filtration, and washed with 500 g of methanol twice to recover the crystal. The recovered crystal was dried in vacuo at 70°C to obtain a compound (B1) .

The other fluorenols (B2) to (B7) were synthesized by using the following fluorenol sources. Specifically, (B2) to (B7) were each synthesized in the same manner as in Synthesis Example 1 by replacing the compound corresponding to the raw material (D1) used in Synthesis Example 1 with corresponding bromides (D2) to (D7) according to the target product structures. The mole ratio between the n-butyllithium solution and 9-fluorenone was same. (B5) was synthesized by using the ketone (D8) instead of 9-fluorenone.

### Fluorenol raw materials:

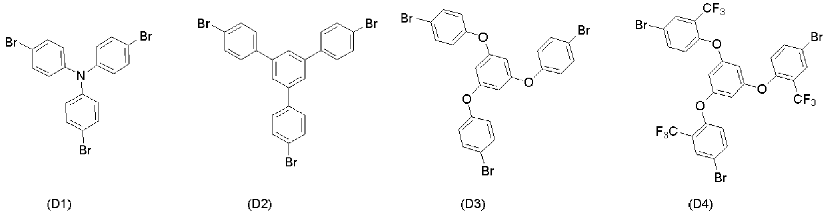

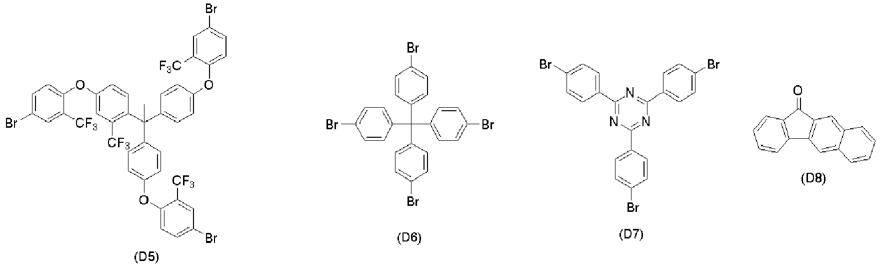

### Synthesis Example 2: Synthesis of Compound (A1)

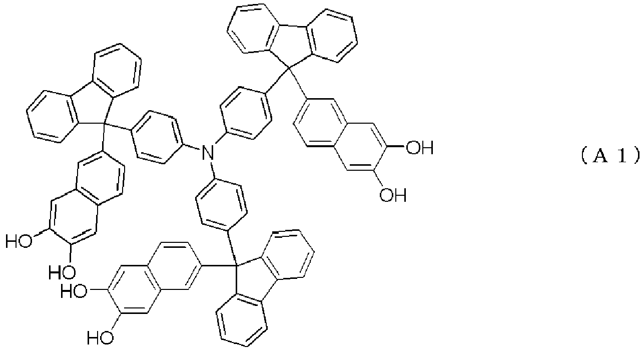

Under a nitrogen atmosphere, 30.0 g of the compound (B1), 55.0 g of the compound (C1), and 400 g of 1,2-dichloroethane were added to prepare a uniform dispersion liquid at an internal temperature of 60°C. Into the dispersion liquid, 22.0 g of methanesulfonic acid was slowly added, and a reaction was performed at an internal temperature of 70°C for 1 hour. After the reaction was finished, the mixture was cooled to room temperature, 1000 ml of methyl isobutyl ketone (MIBK) was added, washed with 200 ml of pure water six times, and the organic layer was dried and solidified under a reduced pressure. Into the residue, 200 g of THF was added to form a uniform solution, and then the product was crystallized into 2000 g of diisopropyl ether (IPE). The precipitated crystal was separated by filtration, and washed with 500 g of IPE twice to recover the crystal. The recovered crystal was dried in vacuo at 70°C to obtain a compound (A1).

A weight-average molecular weight (Mw) and a dispersion degree (Mw/Mn) were determined by GPC to demonstrate the following results.
(A1): Mw=1350, Mw/Mn=1.04

### Synthesis Example 3: Synthesis of Compound (A2)

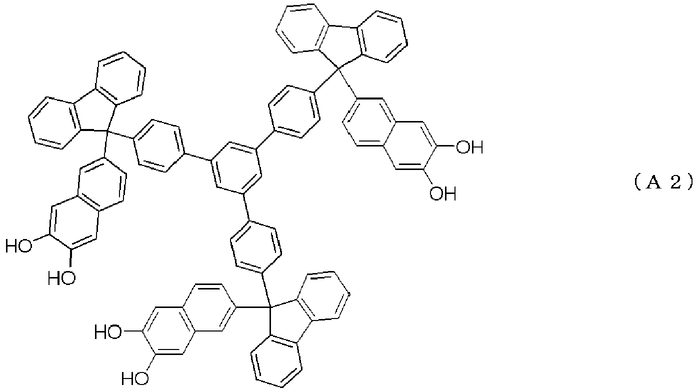

Under a nitrogen atmosphere, 30.0 g of the compound (B2), 19.9 g of the compound (C1), and 250 g of 1,2-dichloroethane were added to prepare a uniform dispersion liquid at an internal temperature of 60°C. Into the dispersion liquid, 20.4 g of methanesulfonic acid was slowly added, and a reaction was performed at an internal temperature of 70°C for 3 hours. After the reaction was finished, the mixture was cooled to room temperature, 1000 ml of MIBK was added, washed with 200 ml of pure water six times, and the organic layer was dried and solidified under a reduced pressure. Into the residue, 100 g of THF was added to form a uniform solution, and then the product was crystallized into 1000 g of diisopropyl ether (IPE). The precipitated crystal was separated by filtration, and washed with 300 g of IPE twice to recover the crystal. The recovered crystal was dried in vacuo at 70°C to obtain a compound (A2) .

A weight-average molecular weight (Mw) and a dispersion degree (Mw/Mn) were determined by GPC to demonstrate the following results.
(A2): Mw=1520, Mw/Mn=1.04

### Synthesis Example 4: Synthesis of Compound (A3)

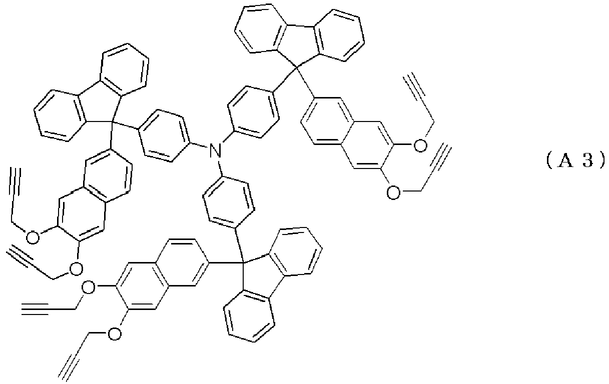

Under a nitrogen atmosphere, 10.0 g of the compound (A1), 10.3 g of potassium carbonate, and 60 g of N,N-dimethylformamide (DMF) were added to prepare a uniform dispersion liquid at an internal temperature of 50°C. Into the dispersion liquid, 8.8 g of propargyl bromide was slowly added, and a reaction was performed at an internal temperature of 50°C for 16 hours. After the reaction was finished, 150 ml of MIBK was added, washed with 50 ml of pure water six times, and the organic layer was dried and solidified under a reduced pressure. Into the residue, 40 g of MIBK was added to form a uniform solution, and then the product was crystallized into 200 g of methanol (MeOH). The precipitated crystal was separated by filtration, and washed with 100 g of MeOH twice to recover the crystal. The recovered crystal was dried in vacuo at 70°C to obtain a compound (A3).

A weight-average molecular weight (Mw) and a dispersion degree (Mw/Mn) were determined by GPC to demonstrate the following results.
(A3): Mw=1590, Mw/Mn=1.04

### Synthesis Example 5: Synthesis of Compound (A4)

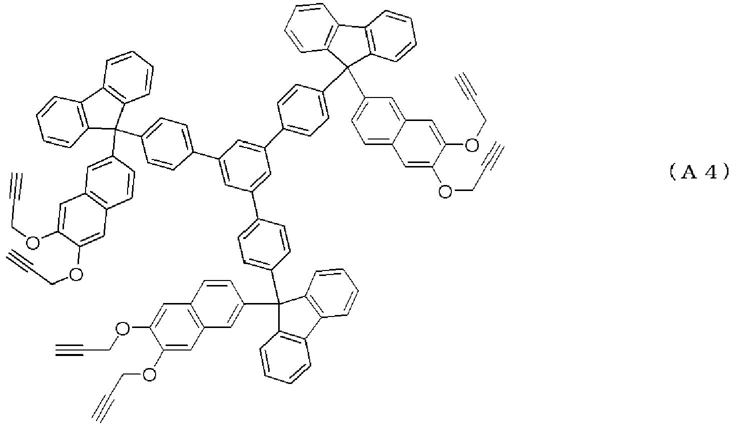

Under a nitrogen atmosphere, 10.0 g of the compound (A2), 9.8 g of potassium carbonate, and 60 g of DMF were added to prepare a uniform dispersion liquid at an internal temperature of 50°C. Into the dispersion liquid, 8.4 g of propargyl bromide was slowly added, and a reaction was performed at an internal temperature of 50°C for 16 hours. After the reaction was finished, 150 ml of MIBK was added, washed with 50 ml of pure water six times, and the organic layer was dried and solidified under a reduced pressure. Into the residue, 40 g of MIBK was added to form a uniform solution, and then the product was crystallized into 200 g of MeOH. The precipitated crystal was separated by filtration, and washed with 100 g of MeOH twice to recover the crystal. The recovered crystal was dried in vacuo at 70°C to obtain a compound (A4).

A weight-average molecular weight (Mw) and a dispersion degree (Mw/Mn) were determined by GPC to demonstrate the following results.
(A4): Mw=1830, Mw/Mn=1.04

### Synthesis Example 6: Synthesis of Compound (A5)

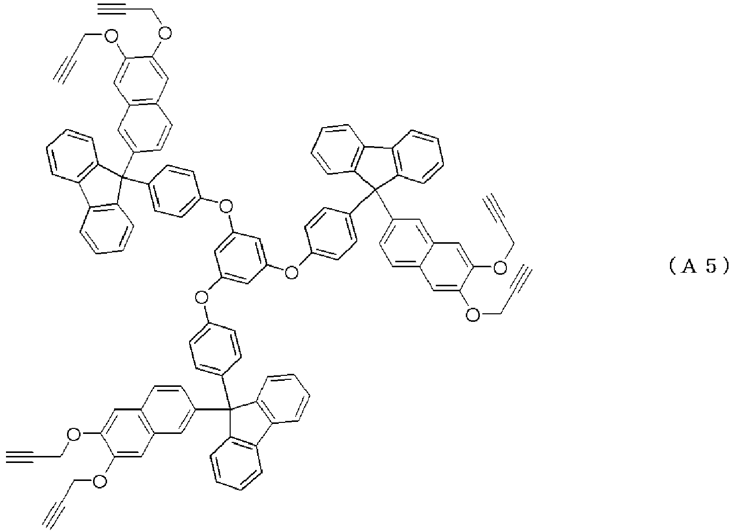

Under a nitrogen atmosphere, 20.0 g of the compound (B3), 21.5 g of the compound (C1), and 200 g of 1,2-dichloroethane were added to prepare a uniform dispersion liquid at an internal temperature of 60°C. Into the dispersion liquid, 12.9 g of methanesulfonic acid was slowly added, and a reaction was performed at an internal temperature of 70°C for 3 hours. After the reaction was finished, the mixture was cooled to room temperature, 1000 ml of MIBK was added, washed with 200 ml of pure water six times, and the organic layer was dried and solidified under a reduced pressure. Into the residue, 100 g of THF was added to form a uniform solution, and then 300 g of hexane was added dropwise while stirring the mixture to precipitate viscous sediment. The sediment was left to stand for a while, and then the clear supernatant was removed by decantation. Into the sediment, 100 g of THF was added again to form a uniform solution, 300 g of hexane was added to precipitate viscous sediment, then the sediment was left to stand, and the supernatant was removed by decantation. DMF was added to the sediment to form a uniform solution, then distilled off under a reduced pressure, and DMF was added to form a DMF solution adjusted at 30 wt% in terms of yield.

Into the obtained DMF solution, 23.9 g of potassium carbonate was added to prepare a uniform dispersion liquid under a nitrogen atmosphere at an internal temperature of 50°C. Into the dispersion liquid, 20.6 g of propargyl bromide was slowly added, and a reaction was performed at an internal temperature of 50°C for 16 hours. After the reaction was finished, 300 ml of MIBK was added, washed with 100 ml of pure water six times, and the organic layer was dried and solidified under a reduced pressure. Into the residue, 80 g of MIBK was added to form a uniform solution, and then the product was crystallized into 400 g of MeOH. The precipitated crystal was separated by filtration, and washed with 200 g of MeOH twice to recover the crystal. The recovered crystal was dried in vacuo at 70°C to obtain a compound (A5).

A weight-average molecular weight (Mw) and a dispersion degree (Mw/Mn) were determined by GPC to demonstrate the following results.
(A5): Mw=1750, Mw/Mn=1.05

### Synthesis Example 7: Synthesis of Compound (A6)

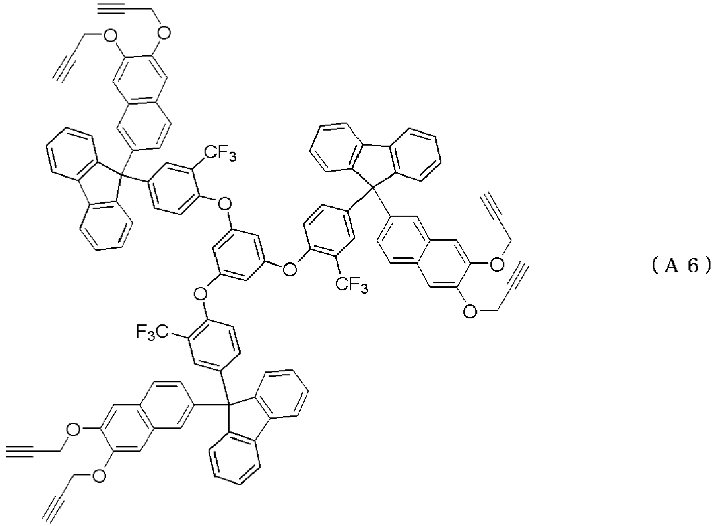

Under a nitrogen atmosphere, 20.0 g of the compound (B4), 17.5 g of the compound (C1), and 190 g of 1,2-dichloroethane were added to prepare a uniform solution at an internal temperature of 60°C. Into the solution, 10.5 g of methanesulfonic acid was slowly added, and a reaction was performed at an internal temperature of 70°C for 3 hours. After the reaction was finished, the mixture was cooled to room temperature, 1000 ml of MIBK was added, washed with 200 ml of pure water six times, and the organic layer was dried and solidified under a reduced pressure. Into the residue, 100 g of THF was added to form a uniform solution, and then 300 g of hexane was added dropwise while stirring the mixture to precipitate viscous sediment. The sediment was left to stand for a while, and then the clear supernatant was removed by decantation. Into the sediment, 100 g of THF was added again to form a uniform solution, 300 g of hexane was added to precipitate viscous sediment, then the sediment was left to stand, and the supernatant was removed by decantation. DMF was added to the sediment to form a uniform solution, then distilled off under a reduced pressure, and DMF was added to form a DMF solution adjusted at 30 wt% in terms of yield.

Into the obtained DMF solution, 22.6 g of potassium carbonate was added to prepare a uniform dispersion liquid under a nitrogen atmosphere at an internal temperature of 50°C. Into the dispersion liquid, 19.5 g of propargyl bromide was slowly added, and a reaction was performed at an internal temperature of 50°C for 16 hours. After the reaction was finished, 300 ml of MIBK was added, washed with 100 ml of pure water six times, and the organic layer was dried and solidified under a reduced pressure. Into the residue, 80 g of MIBK was added to form a uniform solution, and then the product was crystallized into 400 g of MeOH. The precipitated crystal was separated by filtration, and washed with 200 g of MeOH twice to recover the crystal. The recovered crystal was dried in vacuo at 70°C to obtain a compound (A6).

A weight-average molecular weight (Mw) and a dispersion degree (Mw/Mn) were determined by GPC to demonstrate the following results.
(A6): Mw=1870, Mw/Mn=1.03

### Synthesis Example 8: Synthesis of Compound (A7)

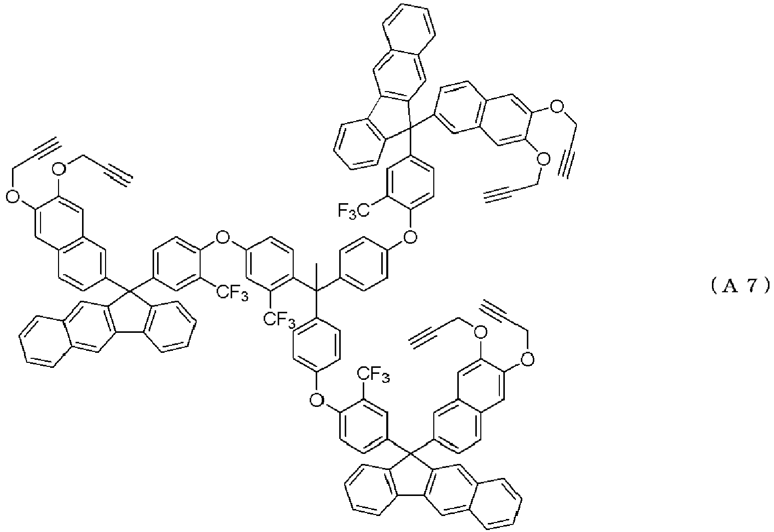

Under a nitrogen atmosphere, 20.0 g of the compound (B5), 12.8 g of the compound (C1), and 160 g of 1,2-dichloroethane were added to prepare a uniform solution at an internal temperature of 60°C. Into the solution, 7.7 g of methanesulfonic acid was slowly added, and a reaction was performed at an internal temperature of 70°C for 3 hours. After the reaction was finished, the mixture was cooled to room temperature, 1000 ml of MIBK was added, washed with 200 ml of pure water six times, and the organic layer was dried and solidified under a reduced pressure. Into the residue, 100 g of THF was added to form a uniform solution, and then 300 g of hexane was added dropwise while stirring the mixture to precipitate viscous sediment. The sediment was left to stand for a while, and then the clear supernatant was removed by decantation. Into the sediment, 100 g of THF was added again to form a uniform solution, 300 g of hexane was added to precipitate viscous sediment, then the sediment was left to stand, and the supernatant was removed by decantation. DMF was added to the sediment to form a uniform solution, then distilled off under a reduced pressure, and DMF was added to form a DMF solution adjusted at 30 wt% in terms of yield.

Into the obtained DMF solution, 16.6 g of potassium carbonate was added to prepare a uniform dispersion liquid under a nitrogen atmosphere at an internal temperature of 50°C. Into the dispersion liquid, 14.3 g of propargyl bromide was slowly added, and a reaction was performed at an internal temperature of 50°C for 16 hours. After the reaction was finished, 300 ml of MIBK was added, washed with 100 ml of pure water six times, and the organic layer was dried and solidified under a reduced pressure. Into the residue, 80 g of MIBK was added to form a uniform solution, and then the product was crystallized into 400 g of MeOH. The precipitated crystal was separated by filtration, and washed with 200 g of MeOH twice to recover the crystal. The recovered crystal was dried in vacuo at 70°C to obtain a compound (A7).

A weight-average molecular weight (Mw) and a dispersion degree (Mw/Mn) were determined by GPC to demonstrate the following results.
(A7): Mw=2320, Mw/Mn=1.05

### Synthesis Example 9: Synthesis of Compound (A8)

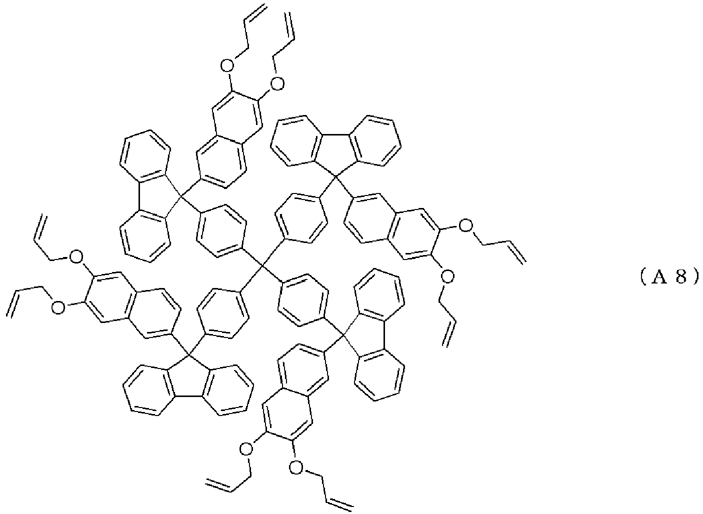

Under a nitrogen atmosphere, 20.0 g of the compound (B6), 18.5 g of the compound (C1), and 200 g of 1,2-dichloroethane were added to prepare a uniform dispersion liquid at an internal temperature of 60°C. Into the dispersion liquid, 11.1 g of methanesulfonic acid was slowly added, and a reaction was performed at an internal temperature of 70°C for 3 hours. After the reaction was finished, the mixture was cooled to room temperature, 1000 ml of MIBK was added, washed with 200 ml of pure water six times, and the organic layer was dried and solidified under a reduced pressure. Into the residue, 100 g of THF was added to form a uniform solution, and then 300 g of hexane was added dropwise while stirring the mixture to precipitate viscous sediment. The sediment was left to stand for a while, and then the clear supernatant was removed by decantation. Into the sediment, 100 g of THF was added again to form a uniform solution, 300 g of hexane was added to precipitate viscous sediment, then the sediment was left to stand, and the supernatant was removed by decantation. DMF was added to the sediment to form a uniform solution, then distilled off under a reduced pressure, and DMF was added to form a DMF solution adjusted at 30 wt% in terms of yield.

Into the obtained DMF solution, 26.5 g of potassium carbonate was added to prepare a uniform dispersion liquid under a nitrogen atmosphere at an internal temperature of 50°C. Into the dispersion liquid, 23.2 g of allyl bromide was slowly added, and a reaction was performed at an internal temperature of 50°C for 16 hours. After the reaction was finished, 300 ml of MIBK was added, washed with 100 ml of pure water six times, and the organic layer was dried and solidified under a reduced pressure. Into the residue, 80 g of MIBK was added to form a uniform solution, and then the product was crystallized into 400 g of MeOH. The precipitated crystal was separated by filtration, and washed with 200 g of MeOH twice to recover the crystal. The recovered crystal was dried in vacuo at 70°C to obtain a compound (A8).

A weight-average molecular weight (Mw) and a dispersion degree (Mw/Mn) were determined by GPC to demonstrate the following results.
(A8): Mw=2110, Mw/Mn=1.05

### Synthesis Example 10: Synthesis of Compound (A9)

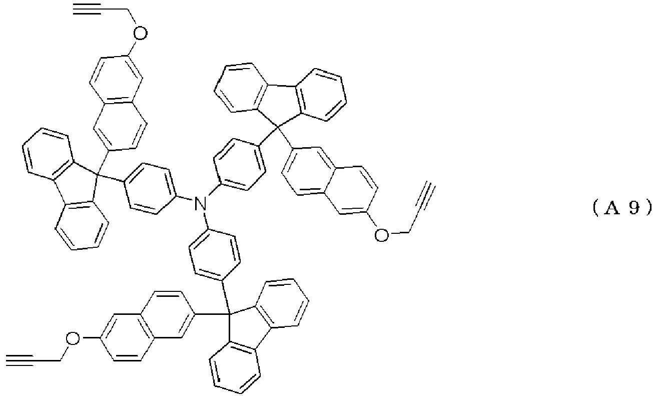

Under a nitrogen atmosphere, 20.0 g of the compound (B1), 41.7 g of the compound (C2), and 300 g of 1,2-dichloroethane were added to prepare a uniform dispersion liquid at an internal temperature of 60°C. Into the dispersion liquid, 22.0 g of methanesulfonic acid was slowly added, and a reaction was performed at an internal temperature of 70°C for 1 hour. After the reaction was finished, the mixture was cooled to room temperature, 1000 ml of methyl isobutyl ketone (MIBK) was added, washed with 200 ml of pure water six times, and the organic layer was dried and solidified under a reduced pressure. Into the residue, 200 g of THF was added to form a uniform solution, and then the product was crystallized into 2000 g of diisopropyl ether (IPE). The precipitated crystal was separated by filtration, and washed with 500 g of IPE twice to recover the crystal. The recovered crystal was dried in vacuo at 70°C to obtain a compound (A9).

A weight-average molecular weight (Mw) and a dispersion degree (Mw/Mn) were determined by GPC to demonstrate the following results.
(A9): Mw=1300, Mw/Mn=1.02

### Synthesis Example 11: Synthesis of Compound (A10)

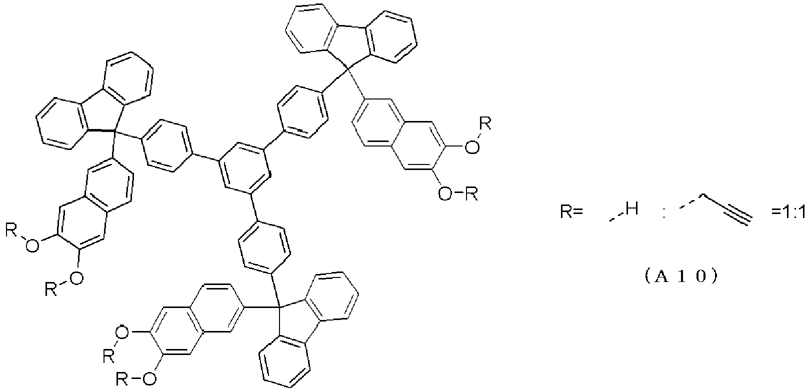

Under a nitrogen atmosphere, 10.0 g of the compound (A2), 6.5 g of potassium carbonate, and 60 g of DMF were added to prepare a uniform dispersion liquid at an internal temperature of 50°C. Into the dispersion liquid, 2.8 g of propargyl bromide was slowly added, and a reaction was performed at an internal temperature of 50°C for 16 hours. After the reaction was finished, 150 ml of MIBK was added, washed with 50 ml of pure water six times, and the organic layer was dried and solidified under a reduced pressure. Into the residue, 40 g of MIBK was added to form a uniform solution, and then the product was crystallized into 200 g of MeOH. The precipitated crystal was separated by filtration, and washed with 100 g of MeOH twice to recover the crystal. The recovered crystal was dried in vacuo at 70°C to obtain a compound (A10).

A weight-average molecular weight (Mw) and a dispersion degree (Mw/Mn) were determined by GPC to demonstrate the following results.
(A10): Mw=1690, Mw/Mn=1.07

### Synthesis Example 12: Synthesis of Compound (A11)

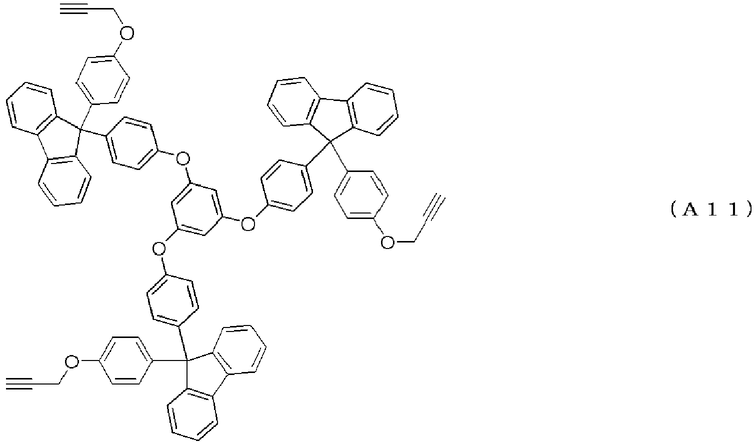

Under a nitrogen atmosphere, 20.0 g of the compound (B3), 25.2 g of the compound (C3), and 200 g of 1,2-dichloroethane were added to prepare a uniform dispersion liquid at an internal temperature of 60°C. Into the dispersion liquid, 12.9 g of methanesulfonic acid was slowly added, and a reaction was performed at an internal temperature of 70°C for 3 hours. After the reaction was finished, the mixture was cooled to room temperature, 1000 ml of MIBK was added, washed with 200 ml of pure water six times, and the organic layer was dried and solidified under a reduced pressure. Into the residue, 100 g of THF was added to form a uniform solution, and then 300 g of hexane was added dropwise while stirring the mixture to precipitate viscous sediment. The sediment was left to stand for a while, and then the clear supernatant was removed by decantation. Into the sediment, 100 g of THF was added again to form a uniform solution, 300 g of hexane was added to precipitate viscous sediment, then the sediment was left to stand, and the supernatant was removed by decantation. DMF was added to the sediment to form a uniform solution, then distilled off under a reduced pressure, and DMF was added to form a DMF solution adjusted at 30 wt% in terms of yield.

Into the obtained DMF solution, 15.4 g of potassium carbonate was added to prepare a uniform dispersion liquid under a nitrogen atmosphere at an internal temperature of 50°C. Into the dispersion liquid, 13.3 g of propargyl bromide was slowly added, and a reaction was performed at an internal temperature of 50°C for 16 hours. After the reaction was finished, 300 ml of MIBK was added, washed with 100 ml of pure water six times, and the organic layer was dried and solidified under a reduced pressure. Into the residue, 80 g of MIBK was added to form a uniform solution, and then the product was crystallized into 400 g of MeOH. The precipitated crystal was separated by filtration, and washed with 200 g of MeOH twice to recover the crystal. The recovered crystal was dried in vacuo at 70°C to obtain a compound (A11).

A weight-average molecular weight (Mw) and a dispersion degree (Mw/Mn) were determined by GPC to demonstrate the following results.
(A11): Mw=1330, Mw/Mn=1.06

### Synthesis Example 13: Synthesis of Compound (A12)

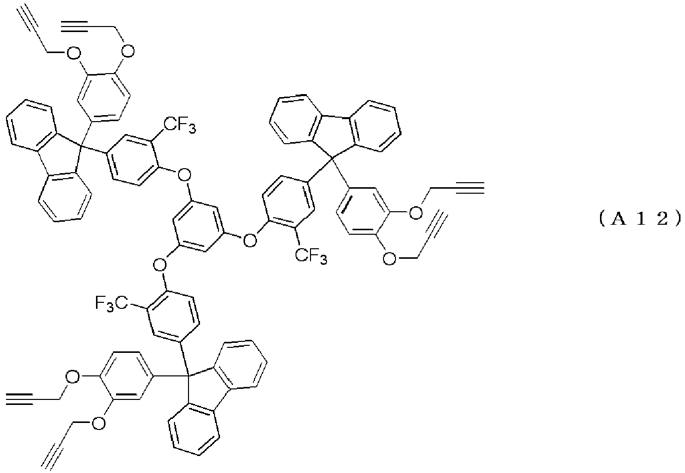

Under a nitrogen atmosphere, 20.0 g of the compound (B4), 12.0 g of the compound (C4), and 160 g of 1,2-dichloroethane were added to prepare a uniform solution at an internal temperature of 60°C. Into the solution, 10.5 g of methanesulfonic acid was slowly added, and a reaction was performed at an internal temperature of 70°C for 3 hours. After the reaction was finished, the mixture was cooled to room temperature, 1000 ml of MIBK was added, washed with 200 ml of pure water six times, and the organic layer was dried and solidified under a reduced pressure. Into the residue, 100 g of THF was added to form a uniform solution, and then 300 g of hexane was added dropwise while stirring the mixture to precipitate viscous sediment. The sediment was left to stand for a while, and then the clear supernatant was removed by decantation. Into the sediment, 100 g of THF was added again to form a uniform solution, 300 g of hexane was added to precipitate viscous sediment, then the sediment was left to stand, and the supernatant was removed by decantation. DMF was added to the sediment to form a uniform solution, then distilled off under a reduced pressure, and DMF was added to form a DMF solution adjusted at 30 wt% in terms of yield.

Into the obtained DMF solution, 22.6 g of potassium carbonate was added to prepare a uniform dispersion liquid under a nitrogen atmosphere at an internal temperature of 50°C. Into the dispersion liquid, 19.5 g of propargyl bromide was slowly added, and a reaction was performed at an internal temperature of 50°C for 16 hours. After the reaction was finished, 300 ml of MIBK was added, washed with 100 ml of pure water six times, and the organic layer was dried and solidified under a reduced pressure. Into the residue, 80 g of MIBK was added to form a uniform solution, and then the product was crystallized into 400 g of MeOH. The precipitated crystal was separated by filtration, and washed with 200 g of MeOH twice to recover the crystal. The recovered crystal was dried in vacuo at 70°C to obtain a compound (A12).

A weight-average molecular weight (Mw) and a dispersion degree (Mw/Mn) were determined by GPC to demonstrate the following results.
(A12): Mw=1540, Mw/Mn=1.03

### Synthesis Example 14: Synthesis of Compound (A13)

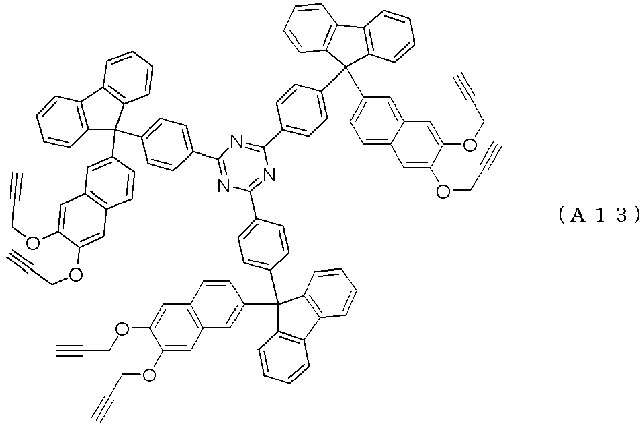

Under a nitrogen atmosphere, 20.0 g of the compound (B7), 15.1 g of the compound (C1), and 200 g of 1,2-dichloroethane were added to prepare a uniform solution at an internal temperature of 60°C. Into the solution, 9.1 g of methanesulfonic acid was slowly added, and a reaction was performed at an internal temperature of 70°C for 3 hours. After the reaction was finished, the mixture was cooled to room temperature, 1000 ml of MIBK was added, washed with 200 ml of pure water six times, and the organic layer was dried and solidified under a reduced pressure. Into the residue, 120 g of THF was added to form a uniform solution, and then 350 g of hexane was added dropwise while stirring the mixture to precipitate viscous sediment. The sediment was left to stand for a while, and then the clear supernatant was removed by decantation. Into the sediment, 120 g of THF was added again to form a uniform solution, 350 g of hexane was added to precipitate viscous sediment, then the sediment was left to stand, and the supernatant was removed by decantation. DMF was added to the sediment to form a uniform solution, then distilled off under a reduced pressure, and DMF was added to form a DMF solution adjusted at 30 wt% in terms of yield.

Into the obtained DMF solution, 29.3 g of potassium carbonate was added to prepare a uniform dispersion liquid under a nitrogen atmosphere at an internal temperature of 50°C. Into the dispersion liquid, 25.2 g of propargyl bromide was slowly added, and a reaction was performed at an internal temperature of 50°C for 16 hours. After the reaction was finished, 400 ml of MIBK was added, washed with 100 ml of pure water six times, and the organic layer was dried and solidified under a reduced pressure. Into the residue, 100 g of MIBK was added to form a uniform solution, and then the product was crystallized into 500 g of MeOH. The precipitated crystal was separated by filtration, and washed with 200 g of MeOH twice to recover the crystal. The recovered crystal was dried in vacuo at 70°C to obtain a compound (A13).

A weight-average molecular weight (Mw) and a dispersion degree (Mw/Mn) were determined by GPC to demonstrate the following results.
(A13): Mw=1810, Mw/Mn=1.04

Compounds (R1) to (R3) for Comparative Example were synthesized by using the following compounds (a1) to (a3).

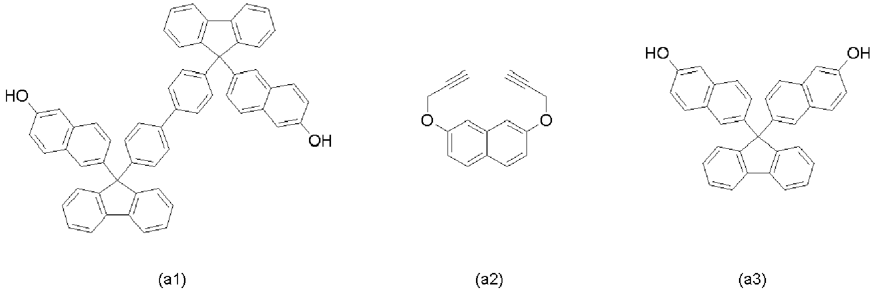

### Comparative Synthesis Example 1: Synthesis of Compound (R1) for Comparative Example

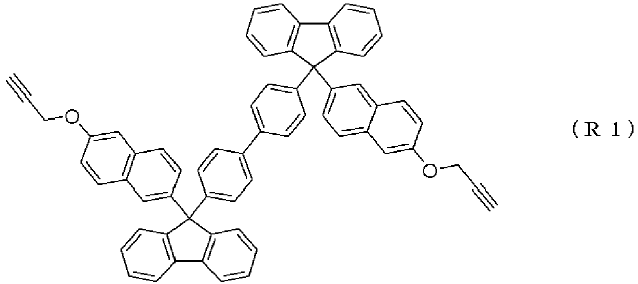

Under a nitrogen atmosphere, 10.00 g of the compound (a1), 4.76 g of potassium carbonate, and 50 g of DMF were added to prepare a uniform dispersion liquid at an internal temperature of 50°C. Into the dispersion liquid, 3.72 g of propargyl bromide was slowly added dropwise, and a reaction was performed at an internal temperature of 50°C for 16 hours. After the mixture was cooled to room temperature, 100 g of methyl isobutyl ketone and 50 g of pure water were added to form uniform solutions, and then the aqueous layer was removed. The organic layer was washed with 30 g of 3.0% nitric acid aqueous solution twice and 30 g of pure water five times, and the organic layer was dried and solidified under a reduced pressure. Into the residue, 30 g of THF was added, and a crystal was precipitated with 100 g of methanol. The precipitated crystal was separated by filtration, and washed with 60 g of methanol twice to recover the crystal. The recovered crystal was dried in vacuo at 70°C to obtain (R1).

A weight-average molecular weight (Mw) and a dispersion degree (Mw/Mn) were determined by GPC to demonstrate the following results.
(R1): Mw=960, Mw/Mn=1.07

### Comparative Synthesis Example 2: Synthesis of Compound (R2) for Comparative Example

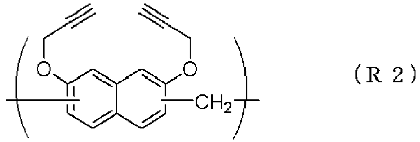

Under a nitrogen atmosphere, 78.8 g of the compound (a2), 21.6 g of 37% formalin solution, and 250 g of 1,2-dichloroethane were added to prepare a uniform solution at a liquid temperature of 70°C. Then, 5 g of methanesulfonic acid was slowly added, and the mixture was stirred at a liquid temperature of 80°C for 12 hours. The mixture was cooled to room temperature, then 500 g of MIBK was added, the organic layer was washed with 200 g of pure water five times, and the organic layer was dried and solidified under a reduced pressure. Into the residue, 300 mL of THF was added, and a polymer was reprecipitated with 2000 mL of hexane. The precipitated polymer was separated by filtration, and dried under a reduced pressure to obtain a compound (R2).

A weight-average molecular weight (Mw) and a dispersion degree (Mw/Mn) were determined by GPC to demonstrate the following results.
(R2): Mw=2720, Mw/Mn=1.55

### Comparative Synthesis Example 3: Synthesis of Compound (R3) for Comparative Example

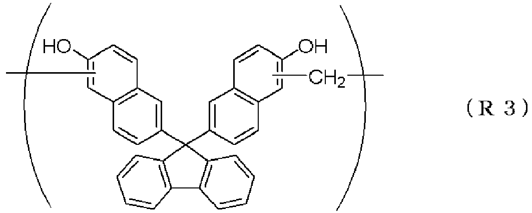

Under a nitrogen atmosphere, 90.1 g of the compound (a3), 10.5 g of 37% formalin solution, and 270 g of 2-methoxy-1-propanol were added to prepare a uniform solution at a liquid temperature of 80°C. Then, 18 g of a 20% solution of paratoluenesulfonic acid in 2-methoxy-1-propanol was slowly added, and the mixture was stirred at a liquid temperature of 110°C for 8 hours. The mixture was cooled to room temperature, then 600 g of MIBK was added, the organic layer was washed with 200 g of pure water five times, and then the organic layer was dried and solidified under a reduced pressure. Into the residue, 320 g of THF was added, and a polymer was reprecipitated with 1350 g of hexane. The precipitated polymer was separated by filtration, and dried under a reduced pressure to obtain a compound (R3).

A weight-average molecular weight (Mw) and a dispersion degree (Mw/Mn) were determined by GPC to demonstrate the following results.
(R3): Mw=3700, Mw/Mn=2.82

Table 1 and Table 2-1 list the structural formulae, the weight-average molecular weight (Mw), and the dispersion degree (Mw/Mn) of the compounds obtained above. Table 2-2 shows the structural formulae, Mw, and Mw/Mn of the compounds (R1) to (R3) used in Comparative Examples.

**[Table 1]**

| Synthesis Example | Compound | Mw | Mw/Mn |
|---|---|---|---|
| 2 | 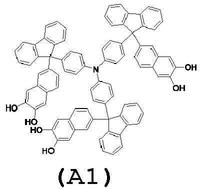 | 1350 | 1.04 |
| 3 | 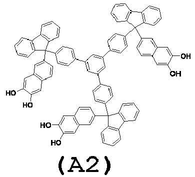 | 1520 | 1.04 |
| 4 | 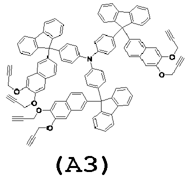 | 1590 | 1.04 |
| 5 | 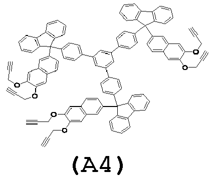 | 1830 | 1.04 |
| 6 | 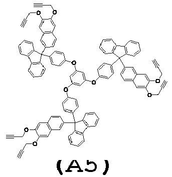 | 1750 | 1.05 |
| 7 | 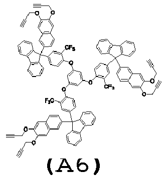 | 1870 | 1.03 |
| 8 | 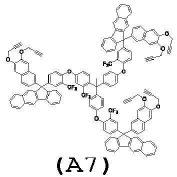 | 2320 | 1.05 |

**[Table 2-1]**

| Synthesis Example | Compound | Mw | Mw/Mn |
|---|---|---|---|
| 9 | 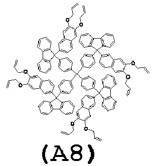 | 2110 | 1.05 |
| 10 | 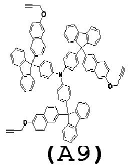 | 1300 | 1.02 |
| 11 | 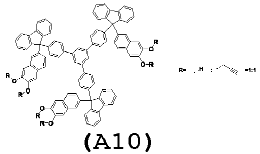 | 1690 | 1.07 |
| 12 | 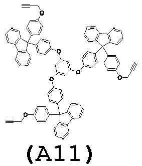 | 1330 | 1.06 |
| 13 | 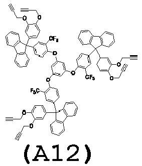 | 1540 | 1.03 |
| 14 | 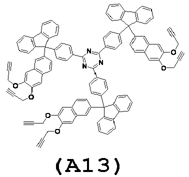 | 1810 | 1.04 |

**[Table 2-2]**

| Synthesis Example | Compound | Mw | Mw/Mn |
|---|---|---|---|
| Comparative Synthesis Example 1 | 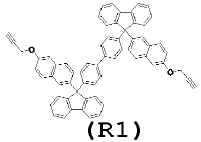 | 960 | 1.07 |
| Comparative Synthesis Example 2 | 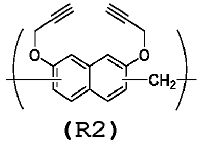 | 2720 | 1.55 |
| Comparative Synthesis Example 3 | 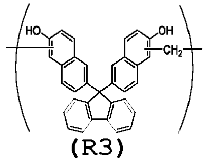 | 3700 | 2.82 |

### Preparation of Material for Forming Organic Film (UDL-1 to 19 and Comparative UDL-1 to 3)

The compounds (A1) to (A13) and (R1) to (R3) were each dissolved in a solvent containing 0.1 mass% of a surfactant FC-4430 (manufactured by Sumitomo 3M Limited.) at a proportion shown in Table 3, and the solution was filtered with a filter made of a fluororesin with 0.1 µm to prepare each of materials for forming an organic film (UDL-1 to 19 and Comparative UDL-1 to 3). For preparing UDL-1 to 15, propylene glycol monomethyl ether acetate (PGMEA) was used as the solvent. For preparing UDL-16 to 19, a mixture of: PGMEA; and any one high-boiling-point solvent of 1,6-diacetoxyhexane (S1) having a boiling of 260°C and tripropylene glycol monomethyl ether (S2) having a boiling point of 242°C was used as the solvent. For preparing Comparative UDL-1 to 3, PGMEA was used as the solvent.

**[Table 3]**

| Materia for forming organic film | Compound (parts by mass) | Crosslinker (parts by mass) | Thermal acid generator (parts by mass) | High-boiling-point solvent (parts by mass) | PGMEA (parts by mass) |
|---|---|---|---|---|---|
| UDL-1 | A3 (10) | - | - | - | 90 |
| UDL-2 | A4 (10) | - | - | - | 90 |
| UDL-3 | A5 (10) | - | - | - | 90 |
| UDL-4 | A6 (10) | - | - | - | 90 |
| UDL-5 | A7 (10) | - | - | - | 90 |
| UDL-6 | A8 (10) | - | - | - | 90 |
| UDL-7 | A9 (10) | - | - | - | 90 |
| UDL-8 | A10 (10) | - | - | - | 90 |
| UDL-9 | A11 (10) | - | - | - | 90 |
| UDL-10 | A12 (10) | - | - | - | 90 |
| UDL-11 | A13 (10) | - | - | - | 90 |
| UDL-12 | A1 (5) | - | - | - | 90 |
| | A3 (5) | | | | |
| UDL-13 | A2 (5) | - | - | - | 90 |
| | A4 (5) | | | | |
| UDL-14 | A1 (10) | XL (2.6) | TAG (0.25) | - | 90 |
| UDL-15 | A5 (10) | XL (2.6) | TAG (0.25) | - | 90 |
| UDL-16 | A3 (10) | - | - | S1 (10) | 80 |
| UDL-17 | A4 (10) | - | - | S2 (10) | 80 |
| UDL-18 | A6 (10) | - | - | S1 (10) | 80 |
| UDL-19 | A8 (10) | - | - | S2 (10) | 80 |
| Comparative UDL-1 | R1 (10) | - | - | - | 90 |
| Comparative UDL-2 | R2 (10) | - | - | - | 90 |
| Comparative UDL-3 | R3 (10) | - | - | - | 90 |

### Example 1: Measurement of Solvent Resistance (Examples 1-1 to 1-19 and Comparative Examples 1-1 to 1-3

The material for forming an organic film (UDL-1 to 19 and Comparative UDL-1 to 3) prepared above was applied on a silicon substrate, baked under a nitrogen flow with a managed oxygen concentration of 0.2 vol% or less at 400°C for 60 seconds, and then a film thickness ("a" [Å]) was measured. A PGMEA solvent was dispensed thereon, left for 30 seconds, spin-dried, baked at 100°C for 60 seconds to evaporate PGMEA, and a film thickness ("b" [Å]) was measured. A difference in the thickness before and after the PGMEA treatment (film residual rate: (b/a)×100) was determined. The following Table 4 shows the results.

**[Table 4]**

| | Material for forming organic film | After film formation | After PGMEA treatment | b/a×100 |
|---|---|---|---|---|
| | | Film thickness: a (Å) | Film thickness :b (Å) | (%) |
| Example 1-1 | UDL-1 | 2005 | 2004 | 100.0 |
| Example 1-2 | UDL-2 | 2006 | 2004 | 99.9 |
| Example 1-3 | UDL-3 | 1998 | 1994 | 99.8 |
| Example 1-4 | UDL-4 | 2002 | 2000 | 99.9 |
| Example 1-5 | UDL-5 | 2003 | 1999 | 99.8 |
| Example 1-6 | UDL-6 | 2003 | 2001 | 99.9 |
| Example 1-7 | UDL-7 | 1995 | 1992 | 99.8 |
| Example 1-8 | UDL-8 | 1992 | 1988 | 99.8 |
| Example 1-9 | UDL-9 | 1991 | 1989 | 99.9 |
| Example 1-10 | UDL-10 | 1996 | 1992 | 99.8 |
| Example 1-11 | UDL-11 | 1999 | 1996 | 99.8 |
| Example 1-12 | UDL-12 | 2001 | 1998 | 99.9 |
| Example 1-13 | UDL-13 | 1993 | 1991 | 99.9 |
| Example 1-14 | UDL-14 | 2004 | 2002 | 99.9 |
| Example 1-15 | UDL-15 | 2004 | 2000 | 99.8 |
| Example 1-16 | UDL-16 | 2010 | 2005 | 99.8 |
| Example 1-17 | UDL-17 | 2015 | 2014 | 100.0 |
| Example 1-18 | UDL-18 | 2012 | 2011 | 100.0 |
| Example 1-19 | UDL-19 | 1996 | 1994 | 99.9 |
| Comparative Example 1-1 | Comparative UDL-1 | 1998 | 1798 | 90.0 |
| Comparative Example 1-2 | Comparative UDL-2 | 2011 | 1816 | 90.3 |
| Comparative Example 1-3 | Comparative UDL-3 | 1999 | 770 | 38.5 |

As shown in Table 4, the materials for forming an organic film of the present invention (Examples 1-1 to 1-19) have a film residual rate after the PGMEA treatment of 99.5% or more, and it is found that the crosslinking reaction occurs even under the nitrogen atmosphere to exhibit sufficient solvent resistance. In contrast, Comparative Example 1-3 had no substituent to be the crosslinking group, and thereby the film residual rate after the PGMEA treatment was less than 50% to exhibit no solvent resistance because the curing reaction did not occur. It is found from these results that the substituent represented by OR₃, which is introduced as a substituent, effectively functions as the thermal crosslinking group.

### Example 2: Evaluation of Heat-Resistant Characteristics (Examples 2-1 to 2-19 and Comparative Examples 2-1 to 2-3)

The materials for forming an organic film (UDL-1 to 19 and Comparative UDL-1 to 3) were each applied on a silicon substrate, baked in air at 180°C to form an applied film with 200 nm, and a film thickness A [Å] was measured. This substrate was further baked under a nitrogen flow with a managed oxygen concentration of 0.2 vol% or less at 400°C for 10 minutes, and then a film thickness B [Å] was measured. The film thickness B was divided by the film thickness A to determine a film residual rate. Table 5 shows these results.

**[Table 5]**

| | Material for forming organic film | After baking | 400°C | Film residual rate % |
|---|---|---|---|---|
| | | Film thickness: A (Å) | Film thickness: B (Å) | (B/A) |
| Example 2-1 | UDL-1 | 2002 | 1984 | 99.1 |
| Example 2-2 | UDL-2 | 1997 | 1984 | 99.3 |
| Example 2-3 | UDL-3 | 2000 | 1991 | 99.6 |
| Example 2-4 | UDL-4 | 1992 | 1977 | 99.2 |
| Example 2-5 | UDL-5 | 2003 | 1997 | 99.7 |
| Example 2-6 | UDL-6 | 2000 | 1941 | 97.1 |
| Example 2-7 | UDL-7 | 1998 | 1945 | 97.3 |
| Example 2-8 | UDL-8 | 2006 | 1981 | 98.8 |
| Example 2-9 | UDL-9 | 1996 | 1947 | 97.5 |
| Example 2-10 | UDL-10 | 1994 | 1969 | 98.7 |
| Example 2-11 | UDL-11 | 2001 | 1988 | 99.4 |
| Example 2-12 | UDL-12 | 2001 | 1969 | 98.4 |
| Example 2-13 | UDL-13 | 2004 | 1976 | 98.6 |
| Example 2-14 | UDL-14 | 2004 | 1978 | 98.7 |
| Example 2-15 | UDL-15 | 2011 | 1982 | 98.6 |
| Example 2-16 | UDL-16 | 2010 | 2004 | 99.7 |
| Example 2-17 | UDL-17 | 2014 | 2007 | 99.7 |
| Example 2-18 | UDL-18 | 2005 | 1986 | 99.1 |
| Example 2-19 | UDL-19 | 2001 | 1942 | 97.1 |
| Comparative | Comparative | 1996 | 1795 | 89.9 |
| Example 2-1 | UDL-1 | | | |
| Comparative Example 2-2 | Comparative UDL-2 | 1996 | 1718 | 86.1 |
| Comparative Example 2-3 | Comparative UDL-3 | 2014 | 1811 | 89.9 |

As shown in Table 5, the materials for forming an organic film of the present invention (Examples 2-1 to 2-19) exhibit reduction in the film thickness of less than 3% even after the bake at 400°C for 10 minutes, and it is found that the material for forming an organic film of the present invention keeps the film thickness even after the bake at 400°C, and has high heat resistance. Among these, Examples 2-1 to 2-5, 2-11, and 2-16 to 2-18, which contain the compound having the structure with n2=1 and n3=2, result in further reduced reduction in the film thickness. In contrast, Comparative Example 2-1, which used the divalent compound, and Comparative Example 2-2, which used the compound without fluorene structure, exhibited the large reduction in the film thickness of more than 10%. Comparative Example 2-3, which used no crosslinking group, also resulted in the reduction in the film thickness of more than 10%. It is found from the above results that the compound having the polyvalent skeleton and the benzene or naphthalene structure forms the dense film having high heat resistance.

### Example 3: Evaluation of Filling Characteristics (Examples 3-1 to 3-19 and Comparative Examples 3-1 to 3-3)

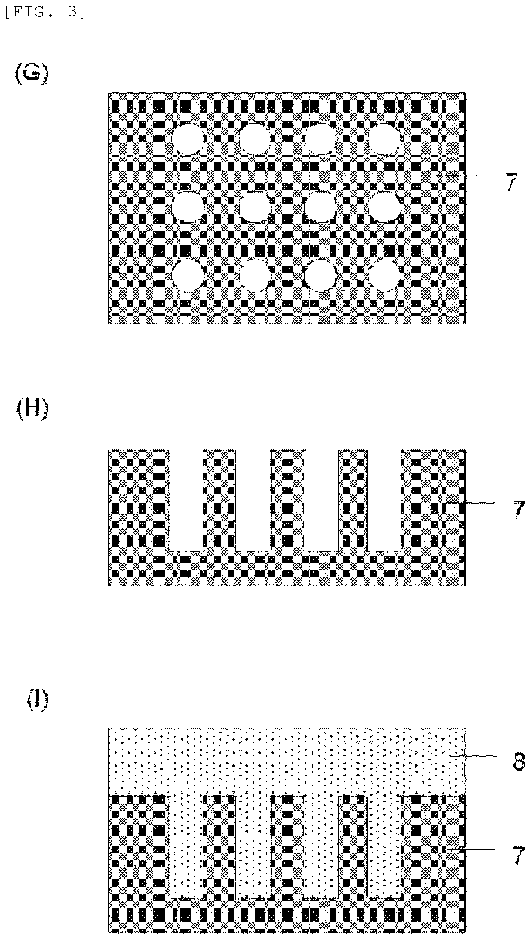

As in FIG. 3, the materials for forming an organic film (UDL-1 to 19 and Comparative UDL-1 to 3) were each applied on a SiO₂ wafer substrate having a dense hole pattern (hole diameter: 0.16 um, hole depth: 0.50 um, and distance between centers of adjacent two holes: 0.32 µm), and the material was baked by using a hot plate under a nitrogen flow with a managed oxygen concentration of 0.2 vol% or less at 400°C for 60 seconds to form an organic film 8. The used substrate was a base substrate (SiO₂ wafer substrate) 7 having the dense hole pattern as illustrated in FIG. 3(G) (overhead view) and FIG. 3(H) (sectional view). A sectional shape of the obtained each wafer substrate was observed by using a scanning electron microscope (SEM) to check whether voids (gaps) were absent inside the holes and were filled with the organic film. Table 6 shows the result. If a material for forming an organic film having poor filling characteristics is used, voids are generated inside the holes in this evaluation. When a material for forming an organic film having good filling characteristics is used, voids are absent inside the holes as illustrated in FIG. 3(I) and filled with the organic film in this evaluation.

**[Table 6]**

| | Material for forming organic film | Presence/absence of void |
|---|---|---|
| Example 3-1 | UDL-1 | absence |
| Example 3-2 | UDL-2 | absence |
| Example 3-3 | UDL-3 | absence |
| Example 3-4 | UDL-4 | absence |
| Example 3-5 | UDL-5 | absence |
| Example 3-6 | UDL-6 | absence |
| Example 3-7 | UDL-7 | absence |
| Example 3-8 | UDL-8 | absence |
| Example 3-9 | UDL-9 | absence |
| Example 3-10 | UDL-10 | absence |
| Example 3-11 | UDL-11 | absence |
| Example 3-12 | UDL-12 | absence |
| Example 3-13 | UDL-13 | absence |
| Example 3-14 | UDL-14 | absence |
| Example 3-15 | UDL-15 | absence |
| Example 3-16 | UDL-16 | absence |
| Example 3-17 | UDL-17 | absence |
| Example 3-18 | UDL-18 | absence |
| Example 3-19 | UDL-19 | absence |
| Comparative Example 3-1 | Comparative UDL-1 | absence |
| Comparative Example 3-2 | Comparative UDL-2 | absence |
| Comparative Example 3-3 | Comparative UDL-3 | presence |

As shown in Table 6, it was confirmed that the materials for forming an organic film of the present invention (Examples 3-1 to 3-19) filled the hole pattern without generation of voids, and had good filling characteristics. Although, Comparative Examples 3-1 and 3-2 exhibited insufficient heat resistance as the results in the evaluation of heat resistance, generation of voids was not observed. Meanwhile, it was confirmed that Comparative Example 3-3 generated voids due to not only the insufficient heat resistance but also the insufficient crosslinking property to exhibit poor filling characteristics. It was confirmed from this result that the material for forming an organic film of the present invention had good filling characteristics.

### Example 4: Evaluation of Planarization Characteristics (Examples 4-1 to 4-19 and Comparative Examples 4-1 to 4-3)

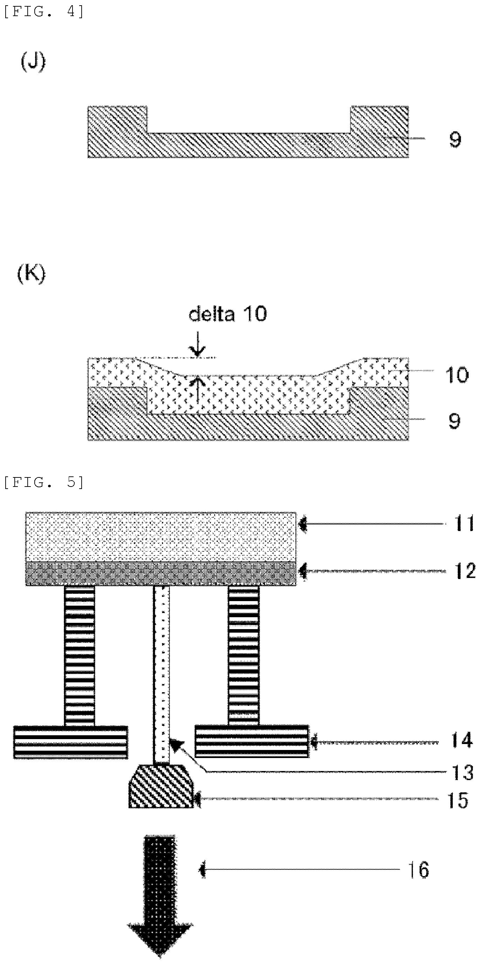

The materials for forming an organic film (UDL-1 to 19 and Comparative UDL-1 to 3) were each applied on a base substrate 9 (SiO₂ wafer substrate) having a giant isolated trench pattern (FIG. 4(J), trench width: 10 µm, trench depth: 0.10 µm). The material was baked under a nitrogen flow with a managed oxygen concentration of 0.2 vol% or less at 400°C for 60 seconds. Then, a step on an organic film 10 between the trenched portion and the non-trenched portion (delta 10 in FIG. 4(K)) was observed by using NX10 atomic force microscope (AFM), manufactured by Park Systems Corporation. Table 7 shows the results. In this evaluation, the smaller the step, the better the planarization characteristics. In this evaluation, the trench pattern with 0.10 µm in depth is planarized by using the material for forming an organic film with a typical film thickness of approximately 0.2 µm, which is a strict evaluation condition to evaluate relative planarization characteristics.

**[Table 7]**

| | Material for forming organic film | Step (nm) |
|---|---|---|
| Example 4-1 | UDL-1 | 25 |
| Example 4-2 | UDL-2 | 30 |
| Example 4-3 | UDL-3 | 25 |
| Example 4-4 | UDL-4 | 30 |
| Example 4-5 | UDL-5 | 25 |
| Example 4-6 | UDL-6 | 40 |
| Example 4-7 | UDL-7 | 40 |
| Example 4-8 | UDL-8 | 35 |
| Example 4-9 | UDL-9 | 40 |
| Example 4-10 | UDL-10 | 35 |
| Example 4-11 | UDL-11 | 30 |
| Example 4-12 | UDL-12 | 30 |
| Example 4-13 | UDL-13 | 30 |
| Example 4-14 | UDL-14 | 35 |
| Example 4-15 | UDL-15 | 35 |
| Example 4-16 | UDL-16 | 15 |
| Example 4-17 | UDL-17 | 20 |
| Example 4-18 | UDL-18 | 20 |
| Example 4-19 | UDL-19 | 30 |
| Comparative Example 4-1 | Comparative UDL-1 | 70 |
| Comparative Example 4-2 | Comparative UDL-2 | 95 |
| Comparative Example 4-3 | Comparative UDL-3 | 100 |

As shown in Table 7, the material for forming an organic film of the present invention (Examples 4-1 to 4-19) caused a small step of the organic film between the trenched portion and non-trenched portion compared with Comparative Examples 4-1 to 4-3, and it was confirmed that the material for forming an organic film of the present invention had excellent planarization characteristics. Although Comparative Examples 4-1 to 4-2, which exhibited insufficient heat resistance as the results of the evaluation of heat resistance, achieved the filling characteristics as the evaluation of filling characteristics, the film was shrunk during the baking at the high temperature, resulting in poor planarization characteristics. In Comparative Example 4-3, the crosslinking property was insufficient, and the planarization characteristics were not good. With each comparing Examples 4-15 to 4-19, in which the high-boiling-point solvent is added, and Examples 4-1, 4-2, 4-4, and 4-6, in which the high-boiling-point solvent is not added, it is found that the addition of the high-boiling-point solvent more improves the planarization characteristics. It is found from this result that the material for forming an organic film of the present invention has excellent heat resistance, and thereby film shrinkage during the baking at high temperature is inhibited to exhibit the excellent planarization characteristics.

### Example 5: Adhesion Test (Examples 5-1 to 5-15 and Comparative Examples 5-1 to 5-2

The material for forming an organic film (UDL-1 to 15 and Comparative UDL 1 to 2) was applied on a SiO₂ wafer substrate, and baked under a nitrogen flow with a managed oxygen concentration of 0.2 vol% or less at 400°C for 60 seconds to form an organic film with 200 nm in film thickness. This wafer with the organic film was cut in square of 1 cm ×1 cm, and an aluminum pin with an epoxy adhesive was adhered to the wafer cut out by using a special tool. Then, the wafer was heated by using an oven at 150°C for 1 hour to adhere the aluminum pin to the substrate. After the cooling to room temperature, a thin-film adhesion strength measurement apparatus (Sebastian Five-A) was used to evaluate initial adhesiveness with resistance force. Note that Comparative UDL-3, which failed to achieve solvent resistance in Example 1, failed to perform the adhesion test.

FIG. 5 shows an explanatory diagram illustrating the method for measuring adhesiveness. In FIG. 5, 11 represents the silicon wafer (substrate), 12 represents the cured film, 13 represents the aluminum pin with the adhesive, 14 represents a support table, 15 represents a grip, and 16 represents a tensile direction. The adhesive force is an average value of values at twelve measured points. The higher value, the higher adhesiveness of the adhesion film to the substrate. The obtained values were compared to evaluate the adhesiveness. Table 8 shows the results.

**[Table 8]**

| | Material for forming organic film | Adhesive force (mN) |
|---|---|---|
| Example 5-1 | UDL-1 | 600 |
| Example 5-2 | UDL-2 | 590 |
| Example 5-3 | UDL-3 | 550 |
| Example 5-4 | UDL-4 | 550 |
| Example 5-5 | UDL-5 | 590 |
| Example 5-6 | UDL-6 | 560 |
| Example 5-7 | UDL-7 | 500 |
| Example 5-8 | UDL-8 | 630 |
| Example 5-9 | UDL-9 | 510 |
| Example 5-10 | UDL-10 | 580 |
| Example 5-11 | UDL-11 | 600 |
| Example 5-12 | UDL-12 | 650 |
| Example 5-13 | UDL-13 | 650 |
| Example 5-14 | UDL-14 | 590 |
| Example 5-15 | UDL-15 | 580 |
| Comparative Example 5-1 | Comparative UDL-1 | 350 |
| Comparative Example 5-2 | Comparative UDL-2 | 250 |

As shown in Examples 5-1 to 5-15 in Table 8, it is found that the organic film material using the compound of the present invention exhibits higher adhesive force than Comparative Examples 5-1 and 5-2. Among these, Examples 5-1 to 5-6, 5-8, and 5-10 to 5-15, which used the structure with n3=2, exhibited high adhesive force, and Examples 5-8, 5-12, and 5-13, which used the structure with R₃=H, exhibited particularly high adhesive force. Meanwhile, Comparative Examples 5-1 and 5-2 exhibited low adhesive force due to insufficient heat resistance. It is found also from this result that the compound of the present invention exhibits high heat resistance and adhesiveness to the substrate.

### Example 6: Patterning Test 1 (Examples 6-1 to 6-19 and Comparative Example 6-1)

The materials for forming an organic film (UDL-1 to 19 and Comparative UDL-1) were each applied on a silicon wafer substrate on which a SiO₂ film with 300 nm was formed, and baked under a nitrogen flow with a managed oxygen concentration of 0.2 vol% or less at 400°C for 60 seconds to form an organic film (resist underlayer film). A CVD-SiON hard mask was formed thereon, an organic anti-reflection film material (ARC-29A: manufactured by Nissan Chemical Corporation) was further applied and baked at 210°C for 60 seconds to form an organic anti-reflection film with 80 nm in film thickness, a single layer resist for ArF being a resist upper layer film material was applied thereon and baked at 105°C for 60 seconds to form a photoresist film (resist upper layer film) with 100 nm in film thickness. On the photoresist film, an immersion protective film material (TC-1) was applied and baked at 90°C for 60 seconds to form a protective film with 50 nm in film thickness. Note that Comparative UDL-2 and UDL-3 had poor heat resistance and crosslinking ability to fail to form the CVD-SiON hard mask, and thereby failed to perform the subsequent patterning test.

The resist upper layer film material (single layer resist for ArF) was prepared by: dissolving a polymer (RP 1), an acid generator (PAG 1), and a basic compound (Amine 1) in a solvent containing 0.1 mass% of FC-430 (manufactured by Sumitomo 3M Limited.) at a proportion shown in Table 9; and filtering the solution with a filter made of a fluororesin with 0.1 µm.

**[Table 9]**

| | Polymer (parts by mass) | Acid generator (parts by mass) | Basic compound (parts by mass) | Solvent (parts by mass) |
|---|---|---|---|---|
| Single layer resist for ArF | RP 1 (100) | PAG 1 (6.6) | Amine 1 (0.8) | PGMEA (2500) |

The used polymer (RP 1), acid generator (PAG 1), and basic compound (Amine 1) are shown below.

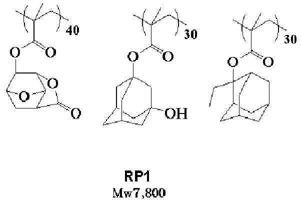

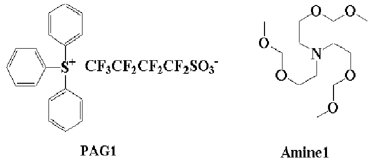

The immersion protective film material (TC-1) was prepared by: dissolving a protective film polymer (PP 1) in an organic solvent at a proportion shown in Table 10; and filtering the solution with a filter made of a fluororesin with 0.1 µm.

**[Table 10]**

| | Polymer (parts by mass) | Organic solvent (parts by mass) |
|---|---|---|
| TC-1 | PP 1 (100) | Diisoamyl ether (2700) |
| | | 2-Methyl-1-butanol (270) |

The used polymer (PP 1) is shown as follows.

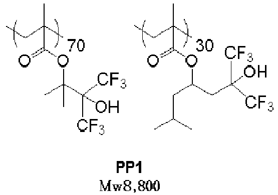

Then, the film was exposed with an ArF immersion exposure device (manufactured by NIKON CORPORATION; NSR-S610C, NA 1.30, σ 0.98/0.65, 35°-dipole s-polarized illumination, 6% halftone phase-shifting mask), baked at 100°C for 60 seconds (PEB), and developed with a 2.38 mass% aqueous solution of tetramethylammonium hydroxide (TMAH) for 30 seconds to obtain a positive line-and-space pattern of 1:1 with 55 nm.

Then, while using the resist pattern as a mask, the organic anti-reflection film and the CVD-SiON hard mask were processed by dry etching using an etching apparatus "Telius", manufactured by Tokyo Electron Ltd., to form a hard mask pattern. While using the obtained hard mask pattern as a mask, the organic film was etched to form an organic film pattern. While using the obtained organic film pattern as a mask, the SiO₂ film was processed by etching. The etching conditions were as follows.

### Transferring conditions of resist pattern to SiON hard mask

Chamber pressure: 10.0 Pa
RF power: 1,500 W
CF₄ gas flow rate: 75 sccm
O₂ gas flow rate: 15 sccm
Time: 15 sec.

### Transferring conditions of hard mask pattern to organic film

Chamber pressure: 2.0 Pa
RF power: 500 W
Ar gas flow rate: 75 sccm
O₂ gas flow rate: 45 sccm
Time: 120 sec.

### Transferring conditions of organic film pattern to SiO₂ film

Chamber pressure: 2.0 Pa
RF power: 2,200 W
C₅F₁₂ gas flow rate: 20 sccm
C₂F₆ gas flow rate: 10 sccm
Ar gas flow rate: 300 sccm
O₂ gas flow rate: 60 sccm
Time: 90 sec.

Table 11 shows the result of observing a cross section of the pattern with an electron microscope (S-4700) manufactured by Hitachi, Ltd.

**[Table 11]**

| | Material for forming organic film | Pattern shape after substrate transferring etching |
|---|---|---|
| Example 6-1 | UDL-1 | vertical |
| Example 6-2 | UDL-2 | vertical |
| Example 6-3 | UDL-3 | vertical |
| Example 6-4 | UDL-4 | vertical |
| Example 6-5 | UDL-5 | vertical |
| Example 6-6 | UDL-6 | vertical |
| Example 6-7 | UDL-7 | vertical |
| Example 6-8 | UDL-8 | vertical |
| Example 6-9 | UDL-9 | vertical |
| Example 6-10 | UDL-10 | vertical |
| Example 6-11 | UDL-11 | vertical |
| Example 6-12 | UDL-12 | vertical |
| Example 6-13 | UDL-13 | vertical |
| Example 6-14 | UDL-14 | vertical |
| Example 6-15 | UDL-15 | vertical |
| Example 6-16 | UDL-16 | vertical |
| Example 6-17 | UDL-17 | vertical |
| Example 6-18 | UDL-18 | vertical |
| Example 6-19 | UDL-19 | vertical |
| Comparative Example 6-1 | Comparative UDL-1 | vertical |

As shown in Table 11, it was confirmed from the results of the materials for forming an organic film of the present invention (Examples 6-1 to 6-19) that the resist upper layer film pattern was favorably and finally transferred to the substrate in all the cases, and that the material for forming an organic film of the present invention was suitably used for fine processing with the multilayer resist method. In Comparative Example 6-1, heat resistance was insufficient but the pattern was formed.

The materials for forming an organic film of the present invention (UDL 1 to 19) formed the organic film exhibiting excellent adhesiveness to the substrate, and consequently inhibited peeling of the CVD-SiON hard mask formed directly on the organic film and the resist upper layer film formed thereon. As a result, in Examples 6-1 to 6-19, the resist upper layer film pattern was favorably and finally transferred to the substrate, as described above.

### Example 7: Patterning Test 2 (Examples 7-1 to 7-19 and Comparative Example 7-1)

An applied film was formed in the same manner as in Patterning Test 1 except that the materials for forming an organic film (UDL-1 to 19 and Comparative UDL-1) were each applied on a SiO₂ wafer substrate having a trench pattern (trench width: 10 um, trench depth: 0.10 µm), and baked under a nitrogen flow with a managed oxygen concentration of 0.2 vol% or less at 400°C for 60 seconds. The patterning and dry-etching were performed, and the shape of the formed pattern was observed. Table 12 shows the results.

**[Table 12]**

| | Material for forming organic film | Pattern shape after substrate transferring etching |
|---|---|---|
| Example 7-1 | UDL-1 | vertical |
| Example 7-2 | UDL-2 | vertical |
| Example 7-3 | UDL-3 | vertical |
| Example 7-4 | UDL-4 | vertical |
| Example 7-5 | UDL-5 | vertical |
| Example 7-6 | UDL-6 | vertical |
| Example 7-7 | UDL-7 | vertical |
| Example 7-8 | UDL-8 | vertical |
| Example 7-9 | UDL-9 | vertical |
| Example 7-10 | UDL-10 | vertical |
| Example 7-11 | UDL-11 | vertical |
| Example 7-12 | UDL-12 | vertical |
| Example 7-13 | UDL-13 | vertical |
| Example 7-14 | UDL-14 | vertical |
| Example 7-15 | UDL-15 | vertical |
| Example 7-16 | UDL-16 | vertical |
| Example 7-17 | UDL-17 | vertical |
| Example 7-18 | UDL-18 | vertical |
| Example 7-19 | UDL-19 | vertical |
| Comparative Example 7-1 | Comparative UDL-1 | presence of pattern collapse |

As shown in Table 12, it was confirmed from the results of the material for forming an organic film of the present invention (Examples 7-1 to 7-19) that the resist upper layer film pattern was favorably and finally transferred to the substrate in all the cases, and that the material for forming an organic film of the present invention was suitably used for fine processing with the multilayer resist method. Meanwhile, in Comparative Example 7-1, the planarization characteristics were poor as the result in the planarization characteristics evaluation, and thereby the pattern was collapsed during the patterning process, leading to failure to finally obtain a good pattern.

The materials for forming an organic film of the present invention (Examples 7-1 to 7-19) formed the organic film exhibiting excellent adhesiveness to the substrate, and consequently inhibited peeling of the CVD-SiON hard mask formed directly on the organic film and the resist upper layer film formed thereon. As a result, in Examples 7-1 to 7-19, the resist upper layer film pattern was favorably and finally transferred to the substrate, as described above.

From the above, it has been revealed that the material for forming an organic film of the present invention containing the compound for forming an organic film of the present invention has heat resistance of 400°C or higher even in an inert gas containing no oxygen, excellent adhesiveness to the substrate, and advanced filling/planarization characteristics, and thereby is extremely useful as the material for forming an organic film used in the multilayer resist method, and the patterning process of the present invention using this material can accurately form a fine pattern even when the body to be processed is a stepped substrate.

It should be noted that the present invention is not limited to the above-described embodiments. The embodiments are just examples, and any examples that substantially have the same feature and demonstrate the same functions and effects as those in the technical concept disclosed in claims of the present invention are included in the technical scope of the present invention.

The present description includes the following embodiments.
[1]: A material for forming an organic film, comprising:
   a compound for forming an organic film (A) represented by the following general formula (1A); and
   an organic solvent (B), 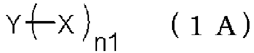
   wherein Y represents an n1-valent organic group, a trivalent nitrogen atom, or a tetravalent carbon atom; n1 represents an integer of 3 to 8; and X represents a partial structure represented by the following general formula (1B), 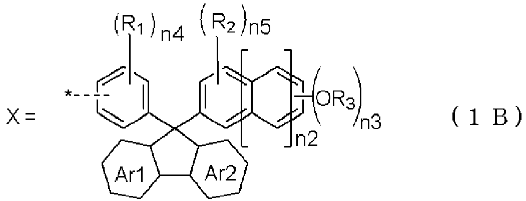
   wherein a broken line represents an attachment point; R₁ and R₂ represent a halogen atom, an alkyl group having 1 to 4 carbon atoms, an alkyloxy group having 1 to 4 carbon atoms, an alkynyl group having 2 to 4 carbon atoms, or an alkenyl group having 2 to 4 carbon atoms, a hydrogen atom on a carbon atom to constitute the alkyl group, the alkyloxy group, the alkynyl group, and the alkenyl group may be substituted with a fluorine atom, and aromatic rings optionally substituted with R₁ may form a cross-linked structure with a single bond or via a divalent group obtained by removing one hydrogen atom from the R₁; n4 and n5 represent an integer of 0 to 2; Ar1 and Ar2 each represent a benzene ring or a naphthalene ring; n2 represents 0 or 1; n3 represents 1 or 2; and R₃ represents a hydrogen atom, an alkyl group having 1 to 4 carbon atoms, an alkynyl group having 2 to 4 carbon atoms, or an alkenyl group having 2 to 4 carbon atoms.
[2]: The material for forming an organic film of the above [1], wherein R₃ in the general formula (1B) represents any one of groups represented by the following formula (1C), 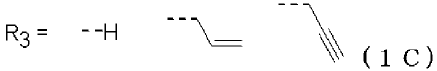 wherein a broken line represents an attachment point.
[3]: The material for forming an organic film of the above [1] or [2], wherein n1 in the general formula (1A) represents 3 or 4.
[4]: The material for forming an organic film of any one of the above [1] to [3], wherein n2 in the general formula (1B) represents 1.
[5]: The material for forming an organic film of any one of the above [1] to [4], wherein Y in the general formula (1A) represents any one of partial structures represented by the following formula (1D), 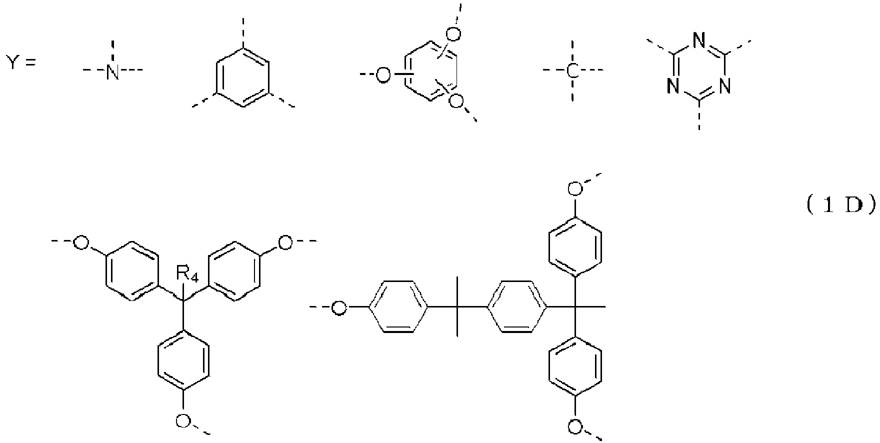 wherein a broken line represents an attachment point; and R₄ represents a hydrogen atom or a methyl group.
[6]: The material for forming an organic film of any one of the above [1] to [5], wherein a ratio Mw/Mn of a weight-average molecular weight Mw to a number-average molecular weight Mn of the component (A) in terms of polystyrene by a gel permeation chromatography method is 1.00 ≤ Mw/Mn ≤ 1.10.
[7]: The material for forming an organic film of any one of the above [1] to [6], wherein the organic solvent (B) is a mixture of one or more kinds of organic solvents having a boiling point of lower than 180°C and one or more kinds of organic solvents having a boiling point of 180°C or higher.
[8]: The material for forming an organic film of any one of the above [1] to [7], further comprising one or more of an acid generator (C), a surfactant (D), a crosslinker (E), and a plasticizer (F).
[9]: A substrate for manufacturing a semiconductor device, comprising a cured organic film of the material for forming an organic film of any one of the above [1] to [8] on a substrate.
[10] A method for forming an organic film applied in a semiconductor device manufacturing process, the method comprising steps of:
   applying the material for forming an organic film of any one of the above [1] to [8] on a substrate to be processed by spin-coating; and
   heat-treating the substrate to be processed coated with the material for forming an organic film under an inert gas atmosphere at a temperature of 50°C or higher and 600°C or lower within a range of 5 seconds to 7200 seconds to obtain a cured film.
[11]: A method for forming an organic film applied in a semiconductor device manufacturing process, the method comprising steps of:
   applying the material for forming an organic film of any one of the above [1] to [8] on a substrate to be processed by spin-coating;
   heat-treating the substrate to be processed coated with the material for forming an organic film in air at a temperature of 50°C or higher and 300°C or lower within a range of 5 seconds to 600 seconds to form an applied film; and
   subsequently heat-treating the applied film under an inert gas atmosphere at a temperature of 200°C or higher and 600°C or lower within a range of 10 seconds to 7200 seconds to obtain a cured film.
[12]: The method for forming an organic film of the above [10] or [11], wherein an oxygen concentration in the inert gas is 1 vol% or less.
[13]: The method for forming an organic film of any one of the above [10] to [12], wherein a substrate to be processed having a structure or step with 30 nm or more in height is used as the substrate to be processed.
[14]: A patterning process, comprising steps of:
   forming an organic film on a body to be processed by using the material for forming an organic film of any one of the above [1] to [8];
   forming a silicon-containing resist intermediate film on the organic film by using a silicon-containing resist intermediate film material;
   forming a resist upper layer film on the silicon-containing resist intermediate film by using a photoresist composition;
   forming a circuit pattern in the resist upper layer film;
   transferring the pattern to the silicon-containing resist intermediate film by etching while using the patterned resist upper layer film as a mask;
   transferring the pattern to the organic film by etching while using the patterned silicon-containing resist intermediate film as a mask; and
   transferring the pattern to the body to be processed by etching while using the patterned organic film as a mask.
[15]: A patterning process, comprising steps of:
   forming an organic film on a body to be processed by using the composition for forming an organic film of any one of the above [1] to [8];
   forming a silicon-containing resist intermediate film on the organic film by using a silicon-containing resist intermediate film material;
   forming an organic anti-reflection film on the silicon-containing resist intermediate film;
   forming a resist upper layer film on the organic anti-reflection film by using a photoresist composition to form a four-layer film structure;
   forming a circuit pattern in the resist upper layer film;
   transferring the pattern to the organic anti-reflection film and the silicon-containing resist intermediate film by etching while using the patterned resist upper layer film as a mask;
   transferring the pattern to the organic film by etching while using the patterned silicon-containing resist intermediate film as a mask; and
   transferring the pattern to the body to be processed by etching while using the patterned organic film as a mask.
[16]: A patterning process, comprising steps of:
   forming an organic film on a body to be processed by using the composition for forming an organic film of any one of the above [1] to [8];
   forming an inorganic hard mask selected from a silicon oxide film, a silicon nitride film, a silicon oxynitride film, a titanium oxide film, and a titanium nitride film on the organic film;
   forming a resist upper layer film on the inorganic hard mask by using a photoresist composition;
   forming a circuit pattern in the resist upper layer film;
   transferring the pattern to the inorganic hard mask by etching while using the patterned resist upper layer film as a mask;
   transferring the pattern to the organic film by etching while using the patterned inorganic hard mask as a mask; and
   transferring the pattern to the body to be processed by etching while using the patterned organic film as a mask.
[17]: A patterning process, comprising steps of:
   forming an organic film on a body to be processed by using the composition for forming an organic film of any one of the above [1] to [8];
   forming an inorganic hard mask selected from a silicon oxide film, a silicon nitride film, a silicon oxynitride film, a titanium oxide film, and a titanium nitride film on the organic film;
   forming an organic anti-reflection film on the inorganic hard mask;
   forming a resist upper layer film on the organic anti-reflection film by using a photoresist composition to form a four-layer film structure;
   forming a circuit pattern in the resist upper layer film;
   transferring the pattern to the organic anti-reflection film and the inorganic hard mask by etching while using the patterned resist upper layer film as a mask;
   transferring the pattern to the organic film by etching while using the patterned inorganic hard mask as a mask; and
   transferring the pattern to the body to be processed by etching while using the patterned organic film as a mask.
[18]: The patterning process of the above [16] or [17], wherein the inorganic hard mask is formed by a CVD method or an ALD method.
[19]: The patterning process of any one of the above [14] to [18], wherein the circuit pattern is formed by a lithography using light having a wavelength of 10 nm or longer and 300 nm or shorter, a direct writing with electron beam, nanoimprinting, or a combination thereof.
[20]: The patterning process of any one of the above [14] to [19], wherein the circuit pattern is developed with an alkaline development or an organic solvent.
[21]: The patterning process of any one of the above [14] to [20], wherein a semiconductor device substrate, or a substrate in which any of a metal film, a metal carbide film, a metal oxide film, a metal nitride film, a metal oxycarbide film, and a metal oxynitride film is formed on the semiconductor device substrate is used as the body to be processed.
[22]: The method for forming a pattern of the above [21], wherein a material containing silicon, titanium, tungsten, hafnium, zirconium, chromium, germanium, copper, silver, gold, aluminum, indium, gallium, arsenic, palladium, iron, tantalum, iridium, cobalt, manganese, molybdenum, or an alloy thereof is used as the body to be processed.
[23]: A compound represented by the following general formula (1A), 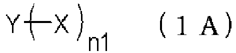
   wherein Y represents an n1-valent organic group, a trivalent nitrogen atom, or a tetravalent carbon atom; n1 represents an integer of 3 to 8; and X represents a partial structure represented by the following general formula (1B), 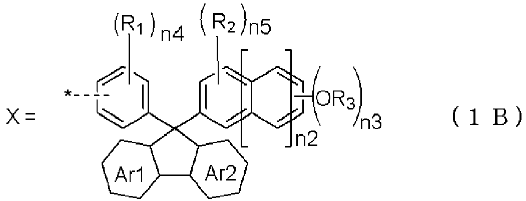
   wherein a broken line represents an attachment point; R₁ and R₂ represent a halogen atom, an alkyl group having 1 to 4 carbon atoms, an alkyloxy group having 1 to 4 carbon atoms, an alkynyl group having 2 to 4 carbon atoms, or an alkenyl group having 2 to 4 carbon atoms, a hydrogen atom on a carbon atom to constitute the alkyl group, the alkyloxy group, the alkynyl group, and the alkenyl group may be substituted with a fluorine atom, and aromatic rings optionally substituted with R₁ may form a cross-linked structure with a single bond or via a divalent group obtained by removing one hydrogen atom from the R₁; n4 and n5 represent an integer of 0 to 2; Ar1 and Ar2 each represent a benzene ring or a naphthalene ring; n2 represents 0 or 1; n3 represents 1 or 2; and R₃ represents a hydrogen atom, an alkyl group having 1 to 4 carbon atoms, an alkynyl group having 2 to 4 carbon atoms, or an alkenyl group having 2 to 4 carbon atoms.
[24]: The compound of the above [23], wherein R₃ in the general formula (1B) represents any one of groups represented by the following formula (1C), 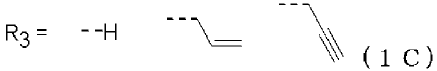 wherein a broken line represents an attachment point.
[25]: The compound of the above [23] or [24], wherein n1 in the general formula (1A) represents 3 or 4.
[26]: The compound of any one of the above [23] to [25], wherein n2 in the general formula (1B) represents 1.
[27] The compound of any one of the above [23] to [26], wherein Y in the general formula (1A) represents any one of partial structures represented by the following formula (1D), 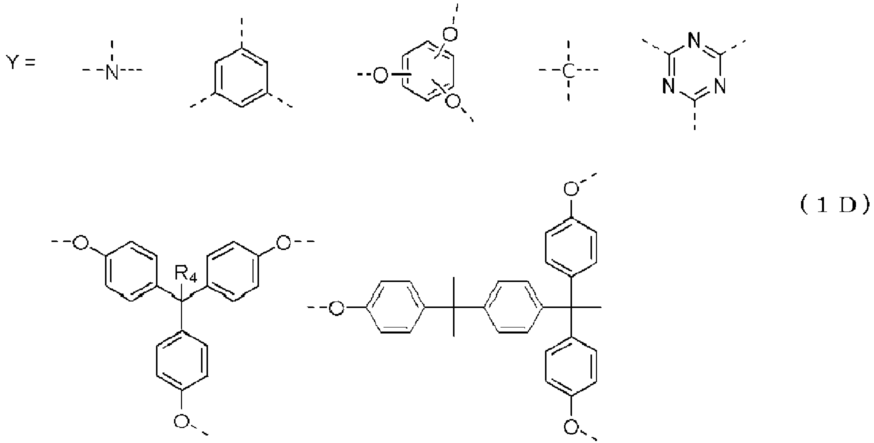 wherein a broken line represents an attachment point; and R₄ represents a hydrogen atom or a methyl group.

The present invention also refers to the following numbered embodiments, wherein the term "claim" means "embodiment":
1. A material for forming an organic film, comprising:
   a compound for forming an organic film (A) represented by the following general formula (1A); and
   an organic solvent (B), 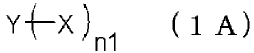
   wherein Y represents an n1-valent organic group, a trivalent nitrogen atom, or a tetravalent carbon atom; n1 represents an integer of 3 to 8; and X represents a partial structure represented by the following general formula (1B), 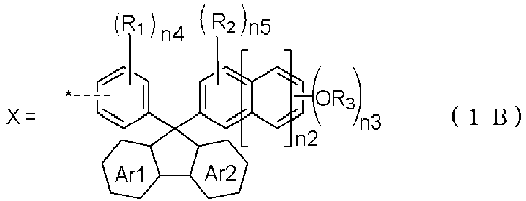
   wherein a broken line represents an attachment point; R₁ and R₂ represent a halogen atom, an alkyl group having 1 to 4 carbon atoms, an alkyloxy group having 1 to 4 carbon atoms, an alkynyl group having 2 to 4 carbon atoms, or an alkenyl group having 2 to 4 carbon atoms, a hydrogen atom on a carbon atom to constitute the alkyl group, the alkyloxy group, the alkynyl group, and the alkenyl group may be substituted with a fluorine atom, and aromatic rings optionally substituted with R₁ may form a cross-linked structure with a single bond or via a divalent group obtained by removing one hydrogen atom from the R₁; n4 and n5 represent an integer of 0 to 2; Ar1 and Ar2 each represent a benzene ring or a naphthalene ring; n2 represents 0 or 1; n3 represents 1 or 2; and R₃ represents a hydrogen atom, an alkyl group having 1 to 4 carbon atoms, an alkynyl group having 2 to 4 carbon atoms, or an alkenyl group having 2 to 4 carbon atoms.
2. The material for forming an organic film according to claim 1, wherein R₃ in the general formula (1B) represents any one of groups represented by the following formula (1C), 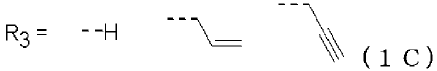 wherein a broken line represents an attachment point.
3. The material for forming an organic film according to claim 1 or 2, wherein n1 in the general formula (1A) represents 3 or 4.
4. The material for forming an organic film according to any one of claim 1 to 3, wherein n2 in the general formula (1B) represents 1.
5. The material for forming an organic film according to any one of claim 1 to 4, wherein Y in the general formula (1A) represents any one of partial structures represented by the following formula (1D), 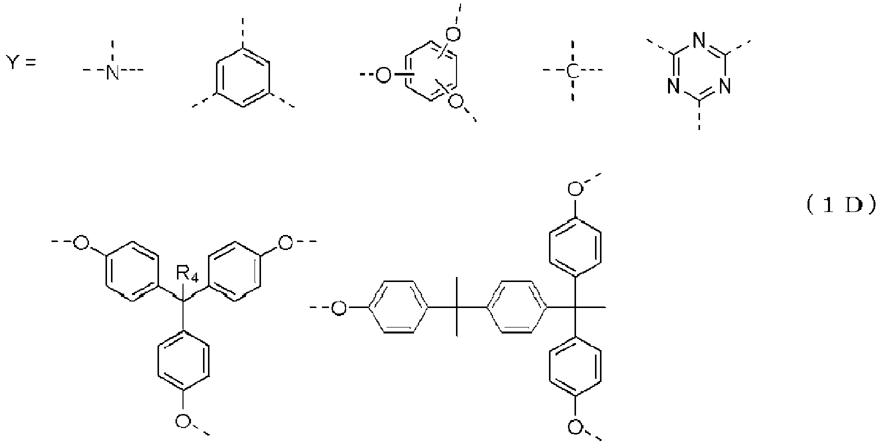 wherein a broken line represents an attachment point; and R₄ represents a hydrogen atom or a methyl group.
6. The material for forming an organic film according to any one of claim 1 to 5, wherein a ratio Mw/Mn of a weight-average molecular weight Mw to a number-average molecular weight Mn of the component (A) in terms of polystyrene by a gel permeation chromatography method is 1.00 ≤ Mw/Mn ≤ 1.10.
7. The material for forming an organic film according to any one of claim 1 to 6, wherein the organic solvent (B) is a mixture of one or more kinds of organic solvents having a boiling point of lower than 180°C and one or more kinds of organic solvents having a boiling point of 180°C or higher.
8. The material for forming an organic film according to any one of claim 1 to 7, further comprising one or more of an acid generator (C), a surfactant (D), a crosslinker (E), and a plasticizer (F).
9. A substrate for manufacturing a semiconductor device, comprising a cured organic film of the material for forming an organic film according to any one of claims 1 to 8 on a substrate.
10. A method for forming an organic film applied in a semiconductor device manufacturing process, the method comprising steps of:
   applying the material for forming an organic film according to any one of claims 1 to 8 on a substrate to be processed by spin-coating; and
   heat-treating the substrate to be processed coated with the material for forming an organic film under an inert gas atmosphere at a temperature of 50°C or higher and 600°C or lower within a range of 5 seconds to 7200 seconds to obtain a cured film.
11. A method for forming an organic film applied in a semiconductor device manufacturing process, the method comprising steps of:
   applying the material for forming an organic film according to any one of claims 1 to 8 on a substrate to be processed by spin-coating;
   heat-treating the substrate to be processed coated with the material for forming an organic film in air at a temperature of 50°C or higher and 300°C or lower within a range of 5 seconds to 600 seconds to form an applied film; and
   subsequently heat-treating the applied film under an inert gas atmosphere at a temperature of 200°C or higher and 600°C or lower within a range of 10 seconds to 7200 seconds to obtain a cured film.
12. The method for forming an organic film according to claim 10 or 11, wherein an oxygen concentration in the inert gas is 1 vol% or less.
13. The method for forming an organic film according to any one of claim 10 to 12, wherein a substrate to be processed having a structure or step with 30 nm or more in height is used as the substrate to be processed.
14. A patterning process, comprising steps of:
   forming an organic film on a body to be processed by using the material for forming an organic film according to any one of claims 1 to 8;
   forming a silicon-containing resist intermediate film on the organic film by using a silicon-containing resist intermediate film material;
   forming a resist upper layer film on the silicon-containing resist intermediate film by using a photoresist composition;
   forming a circuit pattern in the resist upper layer film;
   transferring the pattern to the silicon-containing resist intermediate film by etching while using the patterned resist upper layer film as a mask;
   transferring the pattern to the organic film by etching while using the patterned silicon-containing resist intermediate film as a mask; and
   transferring the pattern to the body to be processed by etching while using the patterned organic film as a mask.
15. A patterning process, comprising steps of:
   forming an organic film on a body to be processed by using the composition for forming an organic film according to any one of claims 1 to 8;
   forming a silicon-containing resist intermediate film on the organic film by using a silicon-containing resist intermediate film material;
   forming an organic anti-reflection film on the silicon-containing resist intermediate film;
   forming a resist upper layer film on the organic anti-reflection film by using a photoresist composition to form a four-layer film structure;
   forming a circuit pattern in the resist upper layer film;
   transferring the pattern to the organic anti-reflection film and the silicon-containing resist intermediate film by etching while using the patterned resist upper layer film as a mask;
   transferring the pattern to the organic film by etching while using the patterned silicon-containing resist intermediate film as a mask; and
   transferring the pattern to the body to be processed by etching while using the patterned organic film as a mask.
16. A patterning process, comprising steps of:
   forming an organic film on a body to be processed by using the composition for forming an organic film according to any one of claims 1 to 8;
   forming an inorganic hard mask selected from a silicon oxide film, a silicon nitride film, a silicon oxynitride film, a titanium oxide film, and a titanium nitride film on the organic film;
   forming a resist upper layer film on the inorganic hard mask by using a photoresist composition;
   forming a circuit pattern in the resist upper layer film;
   transferring the pattern to the inorganic hard mask by etching while using the patterned resist upper layer film as a mask;
   transferring the pattern to the organic film by etching while using the patterned inorganic hard mask as a mask; and
   transferring the pattern to the body to be processed by etching while using the patterned organic film as a mask.
17. A patterning process, comprising steps of:
   forming an organic film on a body to be processed by using the composition for forming an organic film according to any one of claims 1 to 8;
   forming an inorganic hard mask selected from a silicon oxide film, a silicon nitride film, a silicon oxynitride film, a titanium oxide film, and a titanium nitride film on the organic film;
   forming an organic anti-reflection film on the inorganic hard mask;
   forming a resist upper layer film on the organic anti-reflection film by using a photoresist composition to form a four-layer film structure;
   forming a circuit pattern in the resist upper layer film;
   transferring the pattern to the organic anti-reflection film and the inorganic hard mask by etching while using the patterned resist upper layer film as a mask;
   transferring the pattern to the organic film by etching while using the patterned inorganic hard mask as a mask; and
   transferring the pattern to the body to be processed by etching while using the patterned organic film as a mask.
18. The patterning process according to claim 16 or 17, wherein the inorganic hard mask is formed by a CVD method or an ALD method.
19. The patterning process according to any one of claim 14 to 18, wherein the circuit pattern is formed by a lithography using light having a wavelength of 10 nm or longer and 300 nm or shorter, a direct writing with electron beam, nanoimprinting, or a combination thereof.
20. The patterning process according to any one of claim 14 to 19, wherein the circuit pattern is developed with an alkaline development or an organic solvent.
21. The patterning process according to any one of claim 14 to 20, wherein a semiconductor device substrate, or a substrate in which any of a metal film, a metal carbide film, a metal oxide film, a metal nitride film, a metal oxycarbide film, and a metal oxynitride film is formed on the semiconductor device substrate is used as the body to be processed.
22. The method for forming a pattern according to claim 21, wherein a material containing silicon, titanium, tungsten, hafnium, zirconium, chromium, germanium, copper, silver, gold, aluminum, indium, gallium, arsenic, palladium, iron, tantalum, iridium, cobalt, manganese, molybdenum, or an alloy thereof is used as the body to be processed.
23. A compound represented by the following general formula (1A), 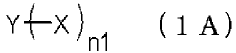
   wherein Y represents an n1-valent organic group, a trivalent nitrogen atom, or a tetravalent carbon atom; n1 represents an integer of 3 to 8; and X represents a partial structure represented by the following general formula (1B), 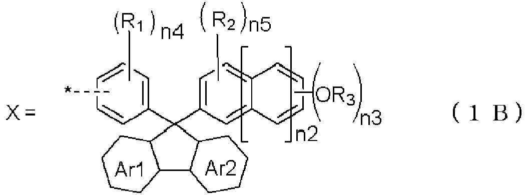
   wherein a broken line represents an attachment point; R₁ and R₂ represent a halogen atom, an alkyl group having 1 to 4 carbon atoms, an alkyloxy group having 1 to 4 carbon atoms, an alkynyl group having 2 to 4 carbon atoms, or an alkenyl group having 2 to 4 carbon atoms, a hydrogen atom on a carbon atom to constitute the alkyl group, the alkyloxy group, the alkynyl group, and the alkenyl group may be substituted with a fluorine atom, and aromatic rings optionally substituted with R₁ may form a cross-linked structure with a single bond or via a divalent group obtained by removing one hydrogen atom from the R₁; n4 and n5 represent an integer of 0 to 2; Ar1 and Ar2 each represent a benzene ring or a naphthalene ring; n2 represents 0 or 1; n3 represents 1 or 2; and R₃ represents a hydrogen atom, an alkyl group having 1 to 4 carbon atoms, an alkynyl group having 2 to 4 carbon atoms, or an alkenyl group having 2 to 4 carbon atoms.
24. The compound according to claim 23, wherein R₃ in the general formula (1B) represents any one of groups represented by the following formula (1C), 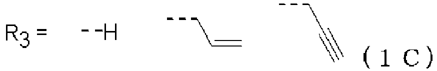 wherein a broken line represents an attachment point.
25. The compound according to claim 23 or 24, wherein n1 in the general formula (1A) represents 3 or 4.
26. The compound according to any one of claim 23 to 25, wherein n2 in the general formula (1B) represents 1.
27. The compound according to any one of claim 23 to 26, wherein Y in the general formula (1A) represents any one of partial structures represented by the following formula (1D), 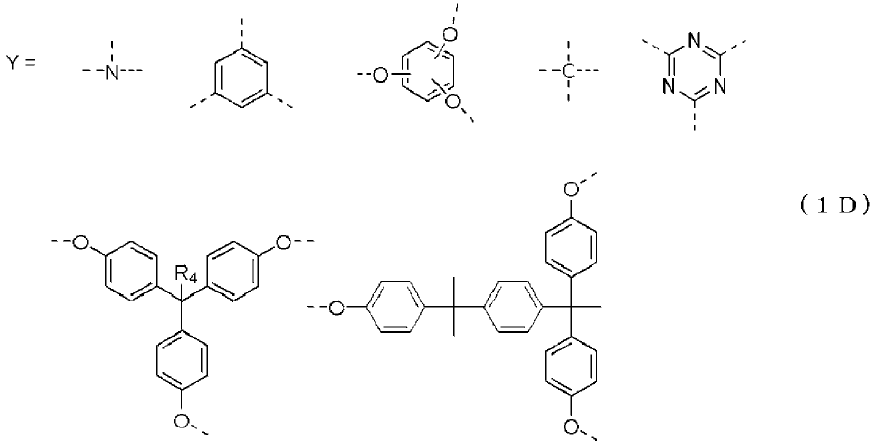 wherein a broken line represents an attachment point; and R₄ represents a hydrogen atom or a methyl group.

## Claims

1. A material for forming an organic film, comprising:
a compound for forming an organic film (A) represented by the following general formula (1A); and
an organic solvent (B), 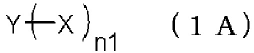
wherein Y represents an n1-valent organic group, a trivalent nitrogen atom, or a tetravalent carbon atom; n1 represents an integer of 3 to 8; and X represents a partial structure represented by the following general formula (1B), 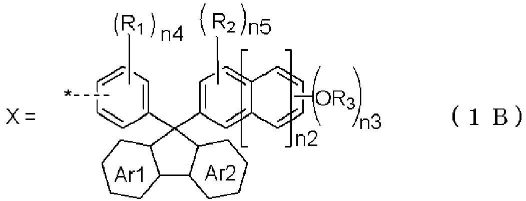
wherein a broken line represents an attachment point; R₁ and R₂ represent a halogen atom, an alkyl group having 1 to 4 carbon atoms, an alkyloxy group having 1 to 4 carbon atoms, an alkynyl group having 2 to 4 carbon atoms, or an alkenyl group having 2 to 4 carbon atoms, a hydrogen atom on a carbon atom to constitute the alkyl group, the alkyloxy group, the alkynyl group, and the alkenyl group may be substituted with a fluorine atom, and aromatic rings optionally substituted with R₁ may form a cross-linked structure with a single bond or via a divalent group obtained by removing one hydrogen atom from the R₁; n4 and n5 represent an integer of 0 to 2; Ar1 and Ar2 each represent a benzene ring or a naphthalene ring; n2 represents 0 or 1; n3 represents 1 or 2; and R₃ represents a hydrogen atom, an alkyl group having 1 to 4 carbon atoms, an alkynyl group having 2 to 4 carbon atoms, or an alkenyl group having 2 to 4 carbon atoms.

2. The material for forming an organic film according to claim 1, wherein
i) R₃ in the general formula (1B) represents any one of groups represented by the following formula (1C), 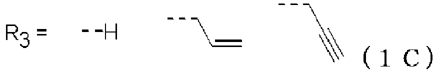 wherein a broken line represents an attachment point; and/or
ii) wherein n1 in the general formula (1A) represents 3 or 4; and/or
iii) wherein n2 in the general formula (1B) represents 1; and/or
iv) wherein Y in the general formula (1A) represents any one of partial structures represented by the following formula (1D), 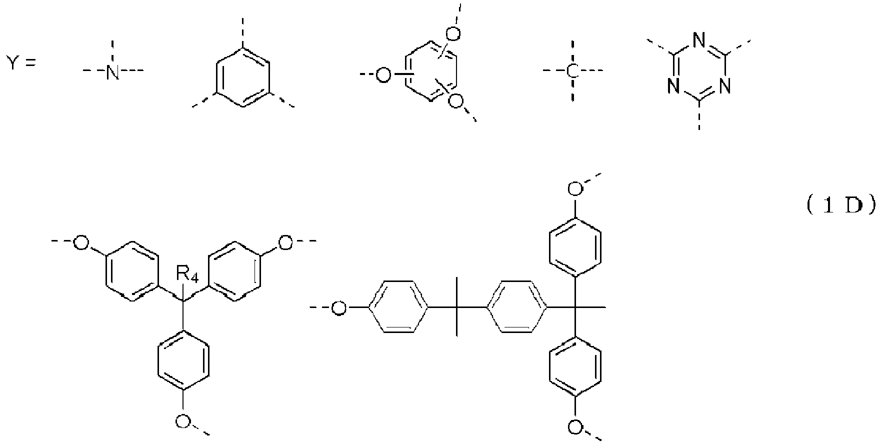 wherein a broken line represents an attachment point; and R₄ represents a hydrogen atom or a methyl group; and/or
v) wherein a ratio Mw/Mn of a weight-average molecular weight Mw to a number-average molecular weight Mn of the component (A) in terms of polystyrene by a gel permeation chromatography method is 1.00 ≤ Mw/Mn ≤ 1.10; and/or
vi) wherein the organic solvent (B) is a mixture of one or more kinds of organic solvents having a boiling point of lower than 180°C and one or more kinds of organic solvents having a boiling point of 180°C or higher.

3. The material for forming an organic film according to claim 1 or 2, further comprising one or more of an acid generator (C), a surfactant (D), a crosslinker (E), and a plasticizer (F).

4. A substrate for manufacturing a semiconductor device, comprising a cured organic film of the material for forming an organic film according to any one of claims 1 to 3 on a substrate.

5. A method for forming an organic film applied in a semiconductor device manufacturing process, the method comprising steps of:
A)
applying the material for forming an organic film according to any one of claims 1 to 3 on a substrate to be processed by spin-coating; and
heat-treating the substrate to be processed coated with the material for forming an organic film under an inert gas atmosphere at a temperature of 50°C or higher and 600°C or lower within a range of 5 seconds to 7200 seconds to obtain a cured film; or
B)
applying the material for forming an organic film according to any one of claims 1 to 3 on a substrate to be processed by spin-coating;
heat-treating the substrate to be processed coated with the material for forming an organic film in air at a temperature of 50°C or higher and 300°C or lower within a range of 5 seconds to 600 seconds to form an applied film; and
subsequently heat-treating the applied film under an inert gas atmosphere at a temperature of 200°C or higher and 600°C or lower within a range of 10 seconds to 7200 seconds to obtain a cured film.

6. The method for forming an organic film according to claim 5, wherein an oxygen concentration in the inert gas is 1 vol% or less.

7. The method for forming an organic film according to claim 5 or 6, wherein a substrate to be processed having a structure or step with 30 nm or more in height is used as the substrate to be processed.

8. A patterning process, comprising steps of:
A)
forming an organic film on a body to be processed by using the material for forming an organic film according to any one of claims 1 to 3;
forming a silicon-containing resist intermediate film on the organic film by using a silicon-containing resist intermediate film material;
forming a resist upper layer film on the silicon-containing resist intermediate film by using a photoresist composition;
forming a circuit pattern in the resist upper layer film;
transferring the pattern to the silicon-containing resist intermediate film by etching while using the patterned resist upper layer film as a mask;
transferring the pattern to the organic film by etching while using the patterned silicon-containing resist intermediate film as a mask; and
transferring the pattern to the body to be processed by etching while using the patterned organic film as a mask; or
B)
forming an organic film on a body to be processed by using the composition for forming an organic film according to any one of claims 1 to 3;
forming a silicon-containing resist intermediate film on the organic film by using a silicon-containing resist intermediate film material;
forming an organic anti-reflection film on the silicon-containing resist intermediate film;
forming a resist upper layer film on the organic anti-reflection film by using a photoresist composition to form a four-layer film structure;
forming a circuit pattern in the resist upper layer film;
transferring the pattern to the organic anti-reflection film and the silicon-containing resist intermediate film by etching while using the patterned resist upper layer film as a mask;
transferring the pattern to the organic film by etching while using the patterned silicon-containing resist intermediate film as a mask; and
transferring the pattern to the body to be processed by etching while using the patterned organic film as a mask; or
C)
forming an organic film on a body to be processed by using the composition for forming an organic film according to any one of claims 1 to 3;
forming an inorganic hard mask selected from a silicon oxide film, a silicon nitride film, a silicon oxynitride film, a titanium oxide film, and a titanium nitride film on the organic film;
forming a resist upper layer film on the inorganic hard mask by using a photoresist composition;
forming a circuit pattern in the resist upper layer film;
transferring the pattern to the inorganic hard mask by etching while using the patterned resist upper layer film as a mask;
transferring the pattern to the organic film by etching while using the patterned inorganic hard mask as a mask; and
transferring the pattern to the body to be processed by etching while using the patterned organic film as a mask; or
D)
forming an organic film on a body to be processed by using the composition for forming an organic film according to any one of claims 1 to 3;
forming an inorganic hard mask selected from a silicon oxide film, a silicon nitride film, a silicon oxynitride film, a titanium oxide film, and a titanium nitride film on the organic film;
forming an organic anti-reflection film on the inorganic hard mask;
forming a resist upper layer film on the organic anti-reflection film by using a photoresist composition to form a four-layer film structure;
forming a circuit pattern in the resist upper layer film;
transferring the pattern to the organic anti-reflection film and the inorganic hard mask by etching while using the patterned resist upper layer film as a mask;
transferring the pattern to the organic film by etching while using the patterned inorganic hard mask as a mask; and
transferring the pattern to the body to be processed by etching while using the patterned organic film as a mask.

9. The patterning process according to embodiment C) or D) of claim 8, wherein the inorganic hard mask is formed by a CVD method or an ALD method.

10. The patterning process according claim 8 or 9, wherein the circuit pattern is formed by a lithography using light having a wavelength of 10 nm or longer and 300 nm or shorter, a direct writing with electron beam, nanoimprinting, or a combination thereof; and/or, wherein the circuit pattern is developed with an alkaline development or an organic solvent.

11. The patterning process according to any one of claim 8 to 10, wherein a semiconductor device substrate, or a substrate in which any of a metal film, a metal carbide film, a metal oxide film, a metal nitride film, a metal oxycarbide film, and a metal oxynitride film is formed on the semiconductor device substrate is used as the body to be processed.

12. The patterning process according to claim 11, wherein a material containing silicon, titanium, tungsten, hafnium, zirconium, chromium, germanium, copper, silver, gold, aluminum, indium, gallium, arsenic, palladium, iron, tantalum, iridium, cobalt, manganese, molybdenum, or an alloy thereof is used as the body to be processed.

13. A compound represented by the following general formula (1A), 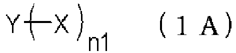 wherein Y represents an n1-valent organic group, a trivalent nitrogen atom, or a tetravalent carbon atom; n1 represents an integer of 3 to 8; and X represents a partial structure represented by the following general formula (1B), 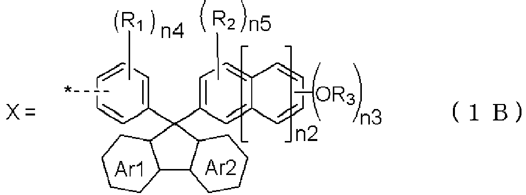 wherein a broken line represents an attachment point; R₁ and R₂ represent a halogen atom, an alkyl group having 1 to 4 carbon atoms, an alkyloxy group having 1 to 4 carbon atoms, an alkynyl group having 2 to 4 carbon atoms, or an alkenyl group having 2 to 4 carbon atoms, a hydrogen atom on a carbon atom to constitute the alkyl group, the alkyloxy group, the alkynyl group, and the alkenyl group may be substituted with a fluorine atom, and aromatic rings optionally substituted with R₁ may form a cross-linked structure with a single bond or via a divalent group obtained by removing one hydrogen atom from the R₁; n4 and n5 represent an integer of 0 to 2; Ar1 and Ar2 each represent a benzene ring or a naphthalene ring; n2 represents 0 or 1; n3 represents 1 or 2; and R₃ represents a hydrogen atom, an alkyl group having 1 to 4 carbon atoms, an alkynyl group having 2 to 4 carbon atoms, or an alkenyl group having 2 to 4 carbon atoms.

14. The compound according to claim 13, wherein
i) R₃ in the general formula (1B) represents any one of groups represented by the following formula (1C), 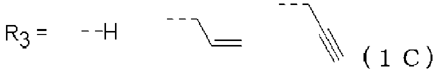 wherein a broken line represents an attachment point; and/or
ii) n1 in the general formula (1A) represents 3 or 4.

15. The compound according to claim 13 or 14, wherein n2 in the general formula (1B) represents 1; and/or wherein Y in the general formula (1A) represents any one of partial structures represented by the following formula (1D), 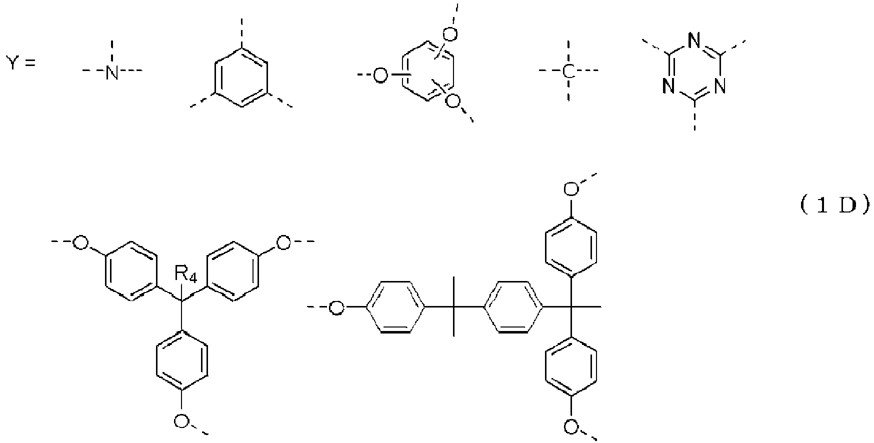 wherein a broken line represents an attachment point; and R₄ represents a hydrogen atom or a methyl group.
